(19) Europäisches Patentamt
European Patent Office
Office européen des brevets

(11) EP 0 770 064 B1

(12) **EUROPEAN PATENT SPECIFICATION**

(45) Date of publication and mention of the grant of the patent:
**05.11.2003 Bulletin 2003/45**

(21) Application number: **95924755.2**

(22) Date of filing: **27.06.1995**

(51) Int Cl.[7]: **C07D 307/20**, A61K 31/34, C07D 307/14, C07D 307/12 // C07D333/04, C07D207/02

(86) International application number:
**PCT/US95/08213**

(87) International publication number:
**WO 96/000212 (04.01.1996 Gazette 1996/02)**

(54) **COMPOUNDS AND METHODS FOR THE TREATMENT OF CARDIOVASCULAR, INFLAMMATORY AND IMMUNE DISORDERS**

VERBINDUNGEN UND VERFAHREN ZUR BEHANDLUNG VON CARDIOVASKULÄREN, INFLAMMATORISCHEN UND DAS IMMUNSYSTEM BETREFFENDE ERKRANKUNGEN

COMPOSES ET PROCEDES POUR LE TRAITEMENT DES TROUBLES CARDIO-VASCULAIRES, INFLAMMATOIRES ET IMMUNITAIRES

(84) Designated Contracting States:
**AT BE CH DE DK ES FR GB GR IE IT LI LU MC NL PT SE**
Designated Extension States:
**LT LV SI**

(30) Priority: **27.06.1994 US 265656**
**17.02.1995 US 390641**
**31.05.1995 US 454600**
**31.05.1995 US 454748**

(43) Date of publication of application:
**02.05.1997 Bulletin 1997/18**

(73) Proprietor: **MILLENIUM PHARMACEUTICALS, INC.**
**Cambridge, MA 02139 (US)**

(72) Inventors:
- **CAI, Xiong**
  **Belmont, MA02178 (US)**
- **GREWAL, Grewal**
  **Natick, MA 01760 (US)**
- **HUSSOIN, Sajjat**
  **Lexington, MA 02173 (US)**
- **FURA, Aberra**
  **Cambridge, MA 02139 (US)**
- **SCANNELL, Ralph**
  **Hopkinton, MA 01748 (US)**
- **BIFTU, Tesfaye**
  **Westfield, NJ 07090 (US)**
- **QIAN, Changgeng**
  **Wayland, MA 01778 (US)**

(74) Representative: **McNeeney, Stephen Phillip et al**
**Eric Potter Clarkson,**
**Park View House,**
**58 The Ropewalk**
**Nottingham NG1 5DD (GB)**

(56) References cited:

| | |
|---|---|
| **EP-A- 0 154 887** | **EP-A- 0 217 204** |
| **EP-A- 0 257 921** | **EP-A- 0 365 089** |
| **EP-A- 0 402 150** | **EP-A- 0 402 151** |
| **EP-A- 0 465 122** | **WO-A-91/17157** |
| **WO-A-94/01430** | **WO-A-95/05360** |
| **WO-A-95/18610** | **US-A- 4 604 407** |
| **US-A- 4 873 259** | **US-A- 5 037 853** |
| **US-A- 5 110 831** | **US-A- 5 169 854** |
| **US-A- 5 175 183** | **US-A- 5 183 818** |
| **US-A- 5 187 192** | **US-A- 5 244 896** |
| **US-A- 5 288 751** | **US-A- 5 326 787** |
| **US-A- 5 420 164** | |

- **CHEMICAL ABSTRACTS, Volume 118, issued 1993, IKEDA et al., "Preparation of Hydroxamic Acid and N-Hydroxyurea Derivatives and Their Use as Lipoxygenase Inhibitors", Abstract No. 59526.**

EP 0 770 064 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Remarks:

The file contains technical information submitted after the application was filed and not included in this specification

## Description

## FIELD OF THE INVENTION

This invention is in the area of 2,5-disubstituted tetrahydrothiophenes, tetrahydrofurans, pyrrolidines and 1,3-disubstituted cyclopentanes. The compounds exhibit biological activity by inhibiting the enzyme 5-lipoxygenase, acting as PAF receptor antagonists, or by exhibiting dual activity, i. e., by acting as both a PAF receptor antagonist and inhibitor of 5-lipoxygenase.

## BACKGROUND OF THE INVENTION

Leukotrienes are potent local mediators, playing a major role in inflammatory and allergic responses, including arthritis, asthma, psoriasis, and thrombotic disease. Leukotrienes are straight chain eicosanoids produced by the oxidation of arachidonic acid by lipoxygenases. Arachidonic acid is oxidized by 5-lipoxygenase to the hydroperoxide 5-hydroperoxy-eicosatetraenoic acid (5-HPETE), that is converted to leukotriene $A_4$, that in turn can be converted to leukotriene $B_4$, $C_4$, or $D_4$. The slow-reacting substance of anaphylaxis is now known to be a mixture of leukotrienes $C_4$, $D_4$, and $E_4$, all of which are potent bronchoconstrictors. There has been a research effort to develop specific receptor antagonists or inhibitors of leukotriene biosynthesis, to prevent or minimize pathogenic inflammatory responses mediated by these compounds.

European Patent Application Nos. 90117171.0 and 901170171.0 disclose indole, benzofuran, and benzothiophene lipoxygenase inhibiting compounds.

Recently, it was reported that the tetrahydrothiophene derivative of L-652,731, *trans*-2,5-bis-(3,4,5-trimethoxyphenyl)tetrahydrothiophene (L-653,150), is a potent PAF antagonist and a moderate inhibitor of 5-lipoxygenase. It has been disclosed that certain 2,5-diaryl tetrahydrothiophenes are PAF antagonists and leukotriene synthesis inhibitors. (Biftu, *et al.*, Abstr. of $6^{th}$ Int. Conf. on Prostaglandins and Related Compounds, June 3-6, 1986, Florence, Italy; U.S. Patent No. 4,757,084 to Biftu); WO 92/15294; WO 94/01430; WO 94/04537; and WO 94/06790.

WO 92/13848 discloses a class of racemic lipoxygenase-inhibiting hydroxamic acid and N-hydroxyurea derivatives of the structure

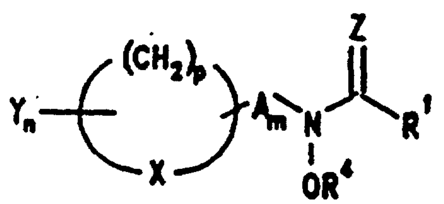

wherein $R^1$ is hydrogen, alkyl, alkenyl, amino or substituted amino, $R^4$ is hydrogen, a pharmaceutically acceptable cation, aroyl or alkoyl, A is alkylene or alkenylene, X is oxygen or sulfur, each Y is hydrogen, halo, cyano, hydroxy, alkyl, alkoxy, alkylthio, alkenyl, alkoxyalkyl, cycloalkyl, aryl, aryloxy, arylalkyl, arylalkenyl, arylalkoxy or substituted aryl, Z is oxygen or sulfur, m is 0 or 1, n is 1 to 5 and p is 2 to 6, inhibit the enzyme lipoxygenase.

Given the significant number of pathological immune and inflammatory responses that are mediated by 5-lipoxygenase, there remains a need to identify new compounds and compositions that inhibit this enzyme.

Platelet activating factor (PAF, 1-O-alkyl-2-acetyl-*sn*-glycerol-3-phosphorylcholine) is a potent inflammatory phospholipid mediator with a wide variety of biological activities. PAF was initially identified as a water soluble compound released by immunoglobulin E (IgE)-sensitized rabbit basophils. It is now known that PAF is also generated and released by monocytes, macrophages, polymorphonuclear leukocytes (PMNs), eosinophils, neutrophils, natural killer lymphocytes, platelets and endothelial cells, as well as by renal and cardiac tissues under appropriate immunological and non-immunological stimulation. (Hwang, "Specific receptors of platelet-activating factor, receptor heterogeneity, and signal transduction mechanisms", Journal of Lipid Mediators 2, 123 (1990)). PAF causes the aggregation and degranulation of platelets at very low concentrations. The potency (active at $10^{-12}$ to $10^{-9}$M), tissue level (picomoles) and short plasma half life (2-4 minutes) of PAF are similar to those of other lipid mediators such as thromboxane $A_2$, prostaglandins, and leukotrienes.

PAF mediates biological responses by binding to specific PAF receptors found in a wide variety of cells and tissues. Structure-activity studies on PAF and its analogs indicate that the ability of PAF to bind to these receptors is structure specific and stereospecific. (Shen, *et al.*, "The Chemical and Biological Properties of PAF Agonists, Antagonists, and Biosynthetic Inhibitors", Platelet-Activating Factor and Related Lipid Mediators, F. Snyder, Ed. Plenum Press, New York, NY 153 (1987)).

While PAF mediates essential biological responses, it also appears to play a role in pathological immune and inflammatory responses. Many published studies have provided evidence for the involvement of PAF in human diseases, including arthritis, acute inflammation, asthma, endotoxic shock, pain, psoriasis, ophthalmic inflammation, ischemia, gastrointestinal ulceration, myocardial infarction, inflammatory bowel diseases, and acute respiratory distress syndrome. Animal models also demonstrate that PAF is produced or increased in certain pathological states.

The involvement of PAF in pathological inflammatory and immune states has stimulated a substantial research effort to identify PAF receptor antagonists. In 1983, a phospholipid analog referred to as CV-3988 (*rac*-3-(N-*n*-octadecylcarbamoyloxy-ω-methoxypropyl-2-thiazolioethyl phosphate) was reported to have PAF receptor antagonist properties. (Terashita, et *al*., Life Sciences 32, 1975 (1983).) In other early work in this area, Shen, *et al*., (in Proc, Natl. Acad. Sci. (U.S.A.) 82, 672 (1985)), reported that kadsurenone, a neolignan derivative isolated from Piper futokadsura Sieb et Zucc (a Chinese herbal plant) was a potent, specific and competitive inhibitor of PAF activity at the receptor level.

Hwang, *et al*., disclosed in 1985 that *trans*-2,5-*bis*-(3,4,5-trimethoxyphenyl) tetrahydrofuran (L-652,731) inhibits the binding of tritiated PAF to PAF receptor sites. (Hwang, *et al*., "*Trans*-2,5-bis-(3,4,5-trimethoxyphenyl)tetrahydrofuran", Journal of Biological Chemistry 260, 15639 (1985).) L-652,731 was found to be orally active, and to inhibit PAF-induced rat cutaneous vascular permeability at a dosage of 30 mg/kg body weight. The compound was found to have no effect on the enzyme 5-lipoxygenase. Hwang, *et al.* also reported that *trans*-L-652,731 (wherein the aryl groups at the 2 and 5 positions are on opposite sides of the plane of the tetrahydrofuran ring) is approximately 1000 times more potent than *cis*-L-652,731 (wherein the 2 and 5 aryl substituents are on the same side of the plane of the tetrahydrofuran ring).

In 1988, Hwang, *et al*., reported that L-659,989 (*trans*-2-(3-methoxy-4-propoxyphenyl-5-methylsulfonyl)-5-(3,4,5-trimethoxyphenyl)tetrahydrofuran) is an orally active, potent, competitive PAF receptor antagonist, with an equilibrium inhibition constant 10 times greater than that of *trans*-L-652,731. (Hwang, *et al*., J. Pharmacol. Ther. 246, 534 (1988).)

U.S. Patent Nos. 4,996,203, 5,001,123 and 4,539,332 to Biftu, *et al*. and European Patent Application Nos. 89202593.3, 90306235.4, and 90306234.7 disclose that specific classes of 2,5-diaryl tetrahydrofurans are PAF receptor antagonists.

Bowles et al., Synlett, 1993, pp 111 disclose a limited number of substituted tetrahydrofurans which may possess PAF receptor antagonism.

Danyoshi et al., Chem. Pharm. Bull., 1989, pp 1969, disclose 2-substituted-N-alkoxycarbonyl pyrrolidines which inhibit PAF induced rabbit platelet aggregation.

Therefore, it is an object of the present invention to provide compounds that reduce the chemotaxis and respiratory burst leading to the formation of damaging oxygen radicals during an inflammatory or immune response.

It is another object of the present invention to provide pharmaceutical compositions for the treatment of pathological immune or inflammatory disorders mediated by products of 5-lipoxygenase.

It is another object of the present invention to provide a method for the treatment of pathological immune or inflammatory disorders mediated by products of 5-lipoxygenase.

It is still another object of the present invention to provide pharmaceutical compositions for the treatment of pathological immune or inflammatory disorders mediated by PAF.

It is another object of the present invention to provide the use of these compositions in a method for the treatment of pathological immune or inflammatory disorders mediated by PAF.

## SUMMARY OF THE INVENTION

Compounds of Formula I are provided

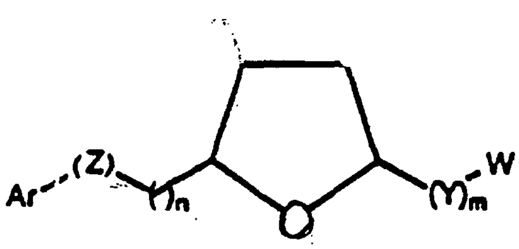

(I)

wherein:

Ar is an aryl or heteroaryl group that is optionally substituted, preferably with halo (including but not limited to fluoro), lower alkoxy (including methoxy), lower aryloxy (including phenoxy), W, cyano, or $R^3$;

m is 0 or 1;

n is 1-6;

W is independently -AN(OM)C(O)N($R^3$)$R^4$, -N(OM)C(O)N($R^3$)$R^4$, -AN($R^3$)C(O)N(OM)$R^4$, -N($R^3$)C(O)N(OM)$R^4$, -AN(OM)C(O)$R^4$, -N(OM)C(O)$R^4$-, AC(O)N(OM)$R^4$, -C(O)N(OM)$R^4$, -C(O)NHA;

A is lower alkyl, lower alkenyl, lower. alkynyl, alkylaryl or arylalkyl groups, wherein one or more carbons optionally can be replaced by O, N, or S (with valence completed with hydrogen or oxygen as necessary), however, -Y-A-, -A-, or -AW-should not include two adjacent heteroatoms (i.e., -O-O-, -S-S-, -O-S-, etc.) (In one embodiment, lower alkyl is a branched alkyl group such as $-(CH_2)_nC$(lower alkyl)H-, wherein n is 1-5, and specifically $-(CH_2)_2C(CH_3)H-$, or lower alkynyl of the formula -C≡C-CH(lower alkyl)-, including $-C{\equiv}C-CH(CH_3)-$) ;

M is hydrogen, a pharmaceutically acceptable cation, or a metabolically cleavable leaving group;

Y is O, S, S(O), $S(O)_2$, $NR^3$, or $CHR^5$;

Z is O, S, S(O), $S(O)_2$, $NR^3$;

$R^3$ and $R^4$ are independently hydrogen or alkyl, alkenyl, alkynyl, aryl, arylalkyl, alkylaryl, $C_{1-6}$ alkoxy-$C_{1-10}$ alkyl, $C_{1-6}$ alkylthio-$C_{1-10}$ alkyl, heteroaryl, or heteroarylalkyl-;

$R^5$ is hydrogen, lower alkyl, lower alkenyl, lower alkynyl, arylalkyl, alkyaryl, -AN(OM)C(O)N($R^3$)$R^4$, -AN($R^3$)C(O)N(OM)$R^4$, -AN(OM)C(O)$R^4$, -AC(O)N(OM)$R^4$, $-AS(O)_xR^3$, $-AS(O)_nCH_2C(O)R^3$, $-AS(O)_nCH_2CH(OH)R^3$, or -AC(O)NH$R^3$, and wherein x is 0-2.

The Ar group, in one embodiment, is selected from the group consisting of phenyl, trimethoxyphenyl, dimethoxyphenyl, fluorophenyl (specifically 4-fluorophenyl), difluorophenyl, pyridyl, dimethoxypyridyl, quinolinyl, furyl, imidazolyl, and thienyl groups.

Examples of preferred compounds are:

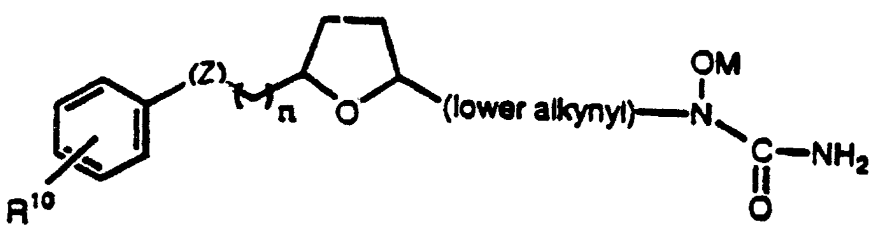

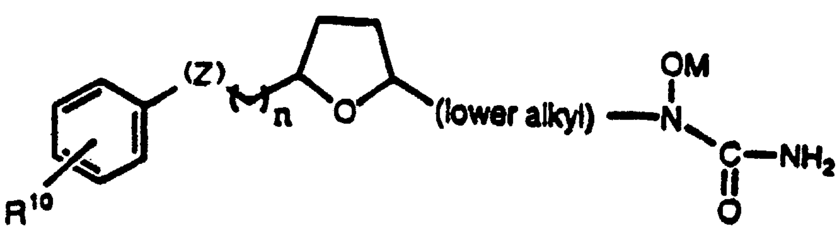

wherein $R^{10}$ is halogen, -CN, hydrogen, lower alkyl, lower alkenyl, lower alkynyl, lower alkoxy, or lower aryloxy.

These compounds in general reduce the chemotaxis and respiratory burst leading to the formation of damaging oxygen radicals of polymorphonuclear leukocytes during an inflammatory or immune response. The compounds exhibit this biological activity by inhibiting the enzyme 5-lipoxygenase, acting as PAF receptor antagonists, or by exhibiting dual activity, i.e., by acting as both a PAF receptor antagonist and inhibitor of 5-lipoxygenase.

Another embodiment of the present invention is a pharmaceutical composition that includes an effective amount of a compound of Formula I or its pharmaceutically acceptable salt or derivative in combination with a pharmaceutically acceptable carrier for any of the disorders described herein.

In a preferred embodiment, the compounds are used to treat disorders mediated by 5-lipoxygenase, by administering an effective amount of one or more of the above-identified compounds or a pharmaceutically acceptable salt or derivative thereof, optionally in a pharmaceutically acceptable carrier.

It has been surprisingly determined that the activity of the compound, for example, the 5-lipoxygenase activity, oral availability, and stability in vivo (for example, glucuronidation rate) can vary significantly among the optical isomers of the disclosed compounds. Therefore, in one embodiment of the invention, the compound is administered in an enantiomerically enriched form.

Examples of immune, allergic and cardiovascular disorders include general inflammation, cardiovascular disorders including hypertension, skeletal-muscular disorders, osteoarthritis, gout, asthma, lung edema, adult respiratory distress syndrome, pain, aggregation of platelets, shock, rheumatoid arthritis, juvenile rheumatoid arthritis, psoriatic arthritis, psoriasis, autoimmune uveitis, allergic encephalomyelitis, systemic lupus erythematosis, acute necrotizing hemorrhagic encephalopathy, idiopathic thrombocytopenia, polychondritis, chronic active hepatitis, idiopathic sprue, Crohn's disease, Graves ophthalmopathy, primary biliary cirrhosis, uveitis posterior, interstitial lung fibrosis; allergic asthma; and inappropriate allergic responses to environmental stimuli such as poison ivy, pollen, insect stings and certain foods, including atopic dermatitis and contact dermatitis.

The compounds disclosed herein can also be used as research tools to study biological pathways involving leukotrienes or PAF, as appropriate. The following are nonlimiting examples of compounds that fall within Formula I. These examples are merely exemplary.

trans-2-(3,4,5-trimethoxyphenoxymethyl)-5-[4-N'-methyl-N'-hydroxyureidyl)butyl]tetrahydrofuran
trans-2-(3,4,5-trimethoxyphenoxymethyl)-5-[4-N'-methyl-N'-hydroxyureidyl)but-1-ynyl]tetrahydrofuran
trans-2-(4-fluorophenoxymethyl)-5-[4-N'-methyl-N'-hydroxyureidyl)butyl]tetrahydrofuran
trans-2-(4-fluorophenoxymethyl)-5-[4-N'methyl-N'-hydroxyureidyl)but-1-ynyl]tetrahydrofuran
trans-2-(4-fluorophenoxymethyl)-5-[4-N'-butyl-N'-hydroxyureidyl)butyl] tetrahydrofuran
trans-2-(4-fluorophenoxymethyl)-5-[4-N'-butyl-N'-hydroxyureidyl)but-1-ynyl]tetrahydrofuran
trans-2-(3,4,5-trimethoxyphenoxymethyl)-5-[4-N'methyl-N-hydroxyureidyl)butyl]tetrahydrofuran
trans-2-(3,4,5-trimethoxyphenoxymethyl)-5-[4-N'-methyl-N-hydroxyureidyl)but-1-ynyl]tetrahydrofuran
trans-2-(4-fluorophenoxymethyl)-5-[4-N'-methyl-N-hydroxyureidyl)butyl]tetrahydrofuran
trans-2-(4-fluorophenoxymethyl)-5-[4-N'-methyl-N-hydroxyureidyl)but-1-ynyl]tetrahydrofuran
trans-2-(4-fluorophenoxymethyl)-5-[4-N'-butyl-N-hydroxyureidyl)butyl]tetrahydrofuran
trans-2-(4-fluorophenoxymethyl)-5-[4-N'-butyl-N-hydroxyureidyl)but-1-ynyl]tetrahydrofuran
trans-2-(3,4,5-trimethoxyphenoxymethyl)-5-[4-N'-methyl-N'-hydroxyureidyl)butyl]-tetrahydrothiophene
trans-2-(3,4,5-trimethoxyphenoxymethyl)-5-[4-N'-methyl-N'-hydroxyureidyl)but-1-ynyl]tetrahydrothiophene
trans-2-(4-fluorophenoxymethyl)-5-[4-N'methyl-N'-hydroxyureidyl)butyl]tetrahydrothiophene
trans-2-(4-fluorophenoxymethyl)-5-[4-N'methyl-N'-hydroxyureidyl)but-1-ynyl]tetrahydrothiophene
trans-2-(4-fluorophenoxymethyl)-5-[4-N'-butyl-N'-hydroxyureidyl)butyl]tetrahydrothiophene
trans-2-(4-fluorophenoxymethyl)-5-[4-N'-butyl-N'-hydroxyureidyl)but-1-ynyl]tetrahydrothiophene
trans-2-(3,4,5-trimethoxyphenoxymethyl)-5-[4-N'-methyl-N-hydroxyureidyl)butyl]tetrahydrothiophene
trans-2-(3,4,5-trimethoxyphenoxymethyl)-5-[4-N'-methyl-N-hydroxyureidyl)but-1-ynyl]tetrahydrothiophene
trans-2-(4-fluorophenoxymethyl)-5-[4-N'-methyl-N-hydroxyureidyl)butyl]tetrahydrothiophene
trans-2-(4-fluorophenoxymethyl)-5-[4-N'methyl-N-hydroxyureidyl)but-1-ynyl]tetrahydrofuran
trans-2-(4-fluorophenoxymethyl)-5-[4-N'-butyl-N-hydroxyureidyl)butyl]tetrahydrothiophene
trans-2-(4-fluorophenoxymethyl)-5-[4-N'-butyl-N-hydroxyureidyl)but-1-ynyl]tetrahydrothiophene
2-(3,4,5-trimethoxyphenyl)-5-[3-(N'-methyl-N'-hydroxyureidyl)propoxy] tetrahydrofuran;
2-(4-fluorophenyl)-5-[3-(N'-methyl-N'-hydroxyureidyl) propoxy] tetrahydrofuran;
2-(3,4,5-trimethoxyphenyl)-5-[3-(N'-n-butyl-N'-hydroxyureidyl)-propoxy]tetrahydrofuran;
2-(4-fluorophenyl)-5-[3-(N'-n-butyl-N'-hydroxyureidyl)propoxy] tetrahydrofuran;
2-(3',4'-dimethoxyphenyl)-5-[3-(N-butyl-N-hydroxyureidyl)]-propoxytetrahydrofuran;
2-[3',4'-dimethoxyphenyl)-5-[3-(N-methyl-N-hydroxyureidyl)]-propoxytetrahydrofuran;
2-(2,4,5-trimethoxyphenyl)-5-(3-hydroxyureidylpropoxy)-tetrahydrofuran;
2-(4-fluorophenyl)-5-(3-hydroxyureidylpropoxy)tetrahydrofuran;

2-(4-fluorophenyl)-5-[3-(N'-methyl-N'-hydroxyureidyl) propoxy] tetrahydrothiophene; and
2-(4-fluorophenyl)-5-(3-hydroxyureidylpropoxy)tetrahydrothiophene.

Further examples of other compounds that fall within Formula I are set forth below in Tables 1, 2 and 3, and Figures 1a and 1b.

TABLE 1

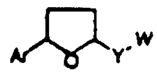

| Ar | W |
|---|---|
| 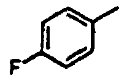 | $CH_2CH_2CH_2NHC(O)N(OH)CH_3$ |
| SAME | $CH_2CH_2CH_2N(OH)C(O)NH_2$ |
| SAME | $CH_2CH_2CH_2N(OH)C(O)NHCH_3$ |
| SAME | $CH_2$-CH=CH-$CH_2N(OH)CONH_2$ |
| 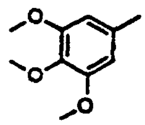 | SAME AS ABOVE |
| 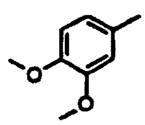 | SAME AS ABOVE |
| 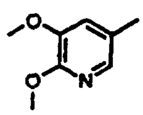 | SAME AS ABOVE |
| 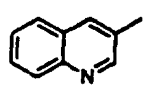 | |
| 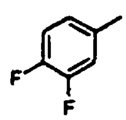 | SAME AS ABOVE |

*C refers to $CHR^5$. Y is O, $CHR^5$, S, or NH.

TABLE 2

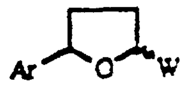

| Ar | W |
|---|---|
| 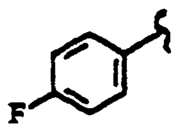 | 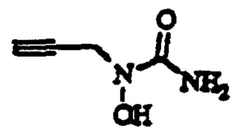 |
| same as above | 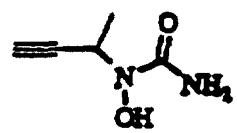 |
| same as above | 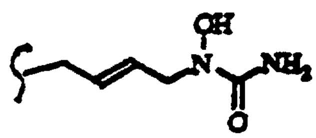 |
| same as above | 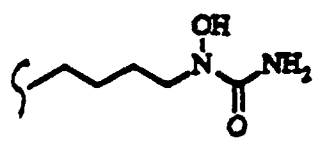 |
| 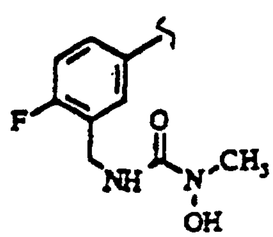 | 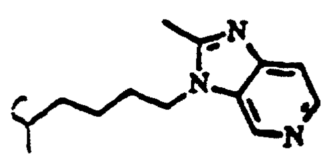 |

TABLE 3

| $-(Y)_m-W$ | $-(Y)_m-W$ |
|---|---|
| 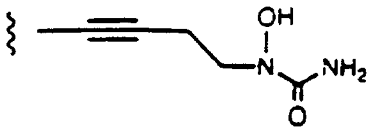 | 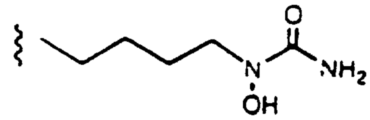 |
| 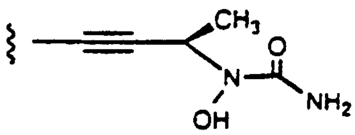 | 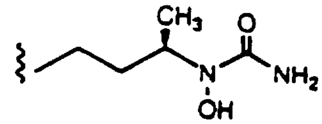 |
| 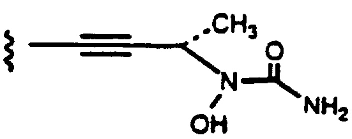 | 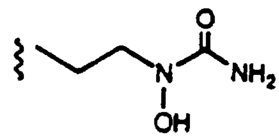 |
| | 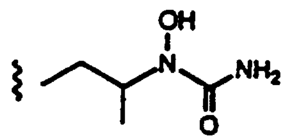 |
| | 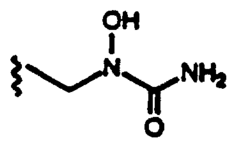 |
| 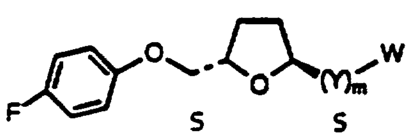 | 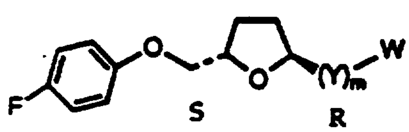 |
| 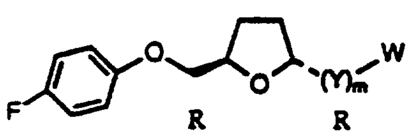 | 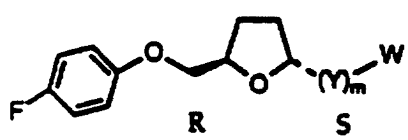 |
| 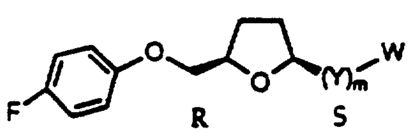 | 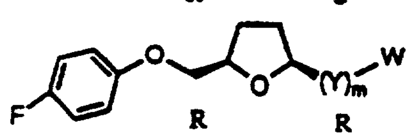 |
| 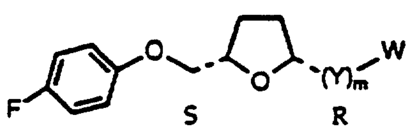 | 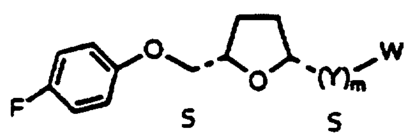 |

## BRIEF DESCRIPTION OF THE FIGURES

Figures 1a and 1b are illustrations of the chemical structures with indicated stereochemistry of selected active compounds.

Figure 2 illustrates the rate of glucuronidation of racemic compound 202, as well as its enantiomers, compounds 216, 217, 234, and 236.

Figure 3 illustrates the rate of glucuronidation for the following illustrated enantiomers.

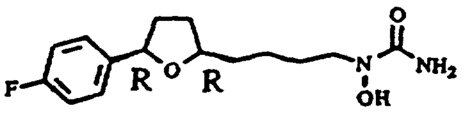

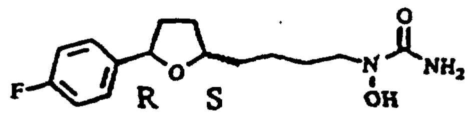

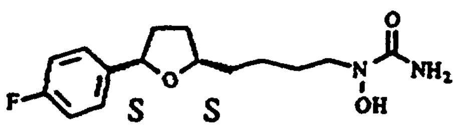

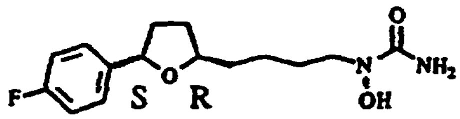

Figure 4 is an illustration of one process for the synthesis of 2S,5S-trans-2-(4-fluorophenoxymethyl)-5-(4-N-hydroxyureidyl-1-butynyl)tetrahydrofuran (compound 401) and 2S,5R-trans-2-(4-fluorophenoxymethyl)-5-(4-N-hydroxyureidylbutyl) tetrahydrofuran (compound 402).

Figure 5 is a graph of the rate of glucuronidation of compounds 401, 403, 404, and 406 (as illustrated in Table 4) as measured in percent metabolite versus time (hours).

## DETAILED DESCRIPTION OF THE INVENTION

### I. Description and Synthesis of the Compounds

### A. Compounds

As used herein, the term "enantiomerically enriched" refers to a compound in the form of at least approximately 95%, and preferably approximately 97%, 98%, 99%, or 100% of a single enantiomer of that compound.

The term alkyl, as used herein, unless otherwise specified, refers to a saturated straight, branched, or cyclic hydrocarbon of $C_1$ to $C_{10}$, and specifically includes methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl. The alkyl group can be optionally substituted with any appropriate group, including but not limited to $R^3$ or one or more moieties selected from the group consisting of halo, hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., "Protective Groups in Organic Synthesis," John Wiley and Sons, Second Edition, 1991.

The term halo, as used herein, refers to chloro, fluoro, iodo, or bromo.

The term lower alkyl, as used herein, and unless otherwise specified, refers to a $C_1$ to $C_6$ saturated straight, branched, or cyclic (in the case of $C_{5-6}$) hydrocarbon, and specifically includes methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl, optionally substituted as described above for the alkyl groups.

The term alkenyl, as referred to herein, and unless otherwise specified, refers to a straight, branched, or cyclic (in the case of $C_{5-6}$) hydrocarbon of $C_2$ to $C_{10}$ with at least one double bond, optionally substituted as described above.

The term lower alkenyl, as referred to herein, and unless otherwise specified, refers to an alkenyl group of $C_2$ to $C_6$, and specifically includes vinyl and allyl.

The term lower alkylamino refers to an amino group that has one or two lower alkyl substituents.

The term alkynyl, as referred to herein, and unless otherwise specified, refers to a $C_2$ to $C_{10}$ straight or branched hydrocarbon with at least one triple bond, optionally substituted as described above. The term lower alkynyl,

as referred to herein, and unless otherwise specified, refers to a $C_2$ to $C_6$ alkynyl group, specifically including acetylenyl, propynyl, and -C≡C-CH(alkyl)-, including -C≡C-CH($CH_3$)-.

The term aryl, as used herein, and unless otherwise specified, refers to phenyl, biphenyl, or napthyl, and preferably phenyl. The aryl group can be optionally substituted with any suitable group, including but not limited to one or more moieties selected from the group consisting of halo, hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., "Protective Groups in Organic Synthesis," John Wiley and Sons, Second Edition, 1991, and preferably with halo (including but not limited to fluoro), lower alkoxy (including methoxy), lower aryloxy (including phenoxy), W, cyano, or $R^3$.

The term haloalkyl, haloalkenyl, or haloalkynyl refers to a alkyl, alkenyl, or alkynyl group in which at least one of the hydrogens in the group has been replaced with a halogen atom.

The term heteroaryl, heterocycle or heteroaromatic, as used herein, refers to an aromatic moiety that includes at least one sulfur, oxygen, or nitrogen in the aromatic ring, which can optionally be substituted as described above for the aryl groups. Non-limiting examples are pyrryl, furyl, pyridyl, 1,2,4-thiadiazolyl, pyrimidyl, thienyl, isothiazolyl, imidazolyl, tetrazolyl, pyrazinyl, pyrimidyl, quinolyl, isoquinolyl, benzothienyl, isobenzofuryl, pyrazolyl, indolyl, purinyl, carbazolyl, benzimidazolyl, and isoxazolyl.

The term aralkyl refers to an aryl group with an alkyl substituent.

The term alkaryl refers to an alkyl group that has an aryl substituent.

The term organic or inorganic anion refers to an organic or inorganic moiety that carries a negative charge and can be used as the negative portion of a salt.

The term "pharmaceutically acceptable cation" refers to an organic or inorganic moiety that carries a positive charge and that can be administered in association with a pharmaceutical agent, for example, as a countercation in a salt. Pharmaceutically acceptable cations are known to those of skill in the art, and include but are not limited to sodium, potassium, and quaternary amine.

The term "metabolically cleavable leaving group" refers to a moiety that can be cleaved in vivo from the molecule to which it is attached, and includes but is not limited to an organic or inorganic anion, a pharmaceutically acceptable cation, acyl (for example (alkyl)C(O), including acetyl, propionyl, and butyryl), alkyl, phosphate, sulfate and sulfonate.

The term PAF receptor antagonist refers to a compound that binds to a PAF receptor with a binding constant of 30 μM or lower.

The term 5-lipoxygenase inhibitor refers to a compound that inhibits the enzyme at 30 μM or lower in a broken cell system.

The term pharmaceutically active derivative refers to any compound that upon administration to the recipient, is capable of providing directly or indirectly, the compounds disclosed herein.

The 2,5-disubstituted tetrahydrothiophenes, tetrahydrofurans and pyrrolidines, as well as the 1,3-disubstituted cyclopentanes described herein exhibit PAF receptor antagonist activity or inhibit the enzyme 5-lipoxygenase, or have dual activity, and are thus useful in the treatment of humans who have immune allergic or cardiovascular disorders that are mediated by PAF or products of 5-lipoxygenase.

## B. Stereochemistry

It has been surprisingly determined that the activity and properties of the active compounds, including 5-lipoxygenase activity, oral availability, and stability in vivo (for example, glucuronidation rate) can vary significantly among the optical isomers of the disclosed compounds. Therefore, in a preferred embodiment, the active compound or its precursor is administered in an enantiomerically enriched form, i.e., substantially in the form of one isomer. The preferred enantiomer is easily determined by evaluating the various possible enantiomers in selected biological assays, for example, those described in detail herein.

The 2,5-disubstituted tetrahydrofurans, tetrahydrothiophenes, and pyrrolidines exhibit a number of stereochemical configurations. Carbon atoms 2 and 5 in the center ring are chiral, and thus the center ring exists at a minimum as a diastereomeric pair. Each diastereomer exists as a set of enantiomers. Therefore, based on the chiral $C_2$ and $C_5$ atoms alone, the compound is a mixture of four enantiomers.

If non-hydrogen substituents are located on carbon atoms 3 and 4 in the center ring, then the $C_3$ and $C_4$ atoms are also chiral, and can also exist as a diastereomeric pair, that is also a mixture of four enantiomers.

The 1,3-cyclopentanes disclosed herein also exhibit a number of stereochemical configurations. Carbon atoms 1 and 3 in the center ring are chiral, and thus the center ring exists at a minimum as a diastereomeric pair. Each diastereomer exists as a set of enantiomers. Therefore, based on the chiral $C_1$ and $C_3$ atoms alone, the compound is a mixture of four enantiomers.

If non-hydrogen substituents are located on carbon atoms 4 and 5 in the center ring, then the $C_4$ and $C_5$

atoms are also chiral, and can also exist as a diastereomeric pair, that is also a mixture of four enantiomers.

One of ordinary skill in the art can easily synthesize and separate the enantiomers of the disclosed compounds using chiral reagents and known procedures, and can evaluate the biological activity of the isolated enantiomer using methods disclosed herein or otherwise known. Through the use of chiral NMR shift reagents, polarimetry, or chiral HPLC, the optical enrichment of the compound can be determined.

Classical methods of resolution include a variety of physical and chemical techniques. Often the simplest and most efficient technique is repeated recrystallization. Recrystallization can be performed at any stage in the preparation of the compound, or the final enantiomeric product. If successful, this simple approach represents a method of choice.

When recrystallization fails to provide material of acceptable optical purity, other methods can be evaluated. If the compound is basic, one can use chiral acids that form diastereomeric derivatives that may possess significantly different solubility properties. Non-limiting examples of chiral acids include malic acid, mandelic acid, dibenzoyl tartaric acid, 3-bromocamphor-8-sulfonic acid, 10-camphorsulfonic acid, and di-*p*-toluoyltartaric acid. Similarly, acylation of a free hydroxyl group with a chiral acid also results in the formation of diastereomeric derivatives whose physical properties may differ sufficiently to permit separation.

Enantiomerically pure or enriched compounds can be obtained by passing the racemic mixture through a chromatographic column that has been designed for chiral separations, or by enzymatic resolution of appropriately modified substrates.

**C. Syntheses of Active Compounds**

The 2,5-disubstituted tetrahydrofurans, tetrahydrothiophenes, and pyrrolidines disclosed herein can be prepared in a variety of ways known to those skilled in the art, including by methods disclosed by Whittaker et al, Synlett, 1993 pp 111, Biorg. Med. Lett., 1993 pp 1499; Achiwa et al., Chem. Pharm. Bull., 1989, pp. 1969.

For example, one method for the synthesis of enantiomerically enriched materials is set forth below in Scheme I. In this method, the enantiomeric synthesis begins with the chiral reduction of a ketone. After ring closure and reaction of the -OH group, the cis and trans isomers can be separated by standard means known to those skilled in the art, affecting a diastereomeric resolution. Additional chiral centers can be resolved using techniques known to those skilled in the art, including those set forth in the examples below.

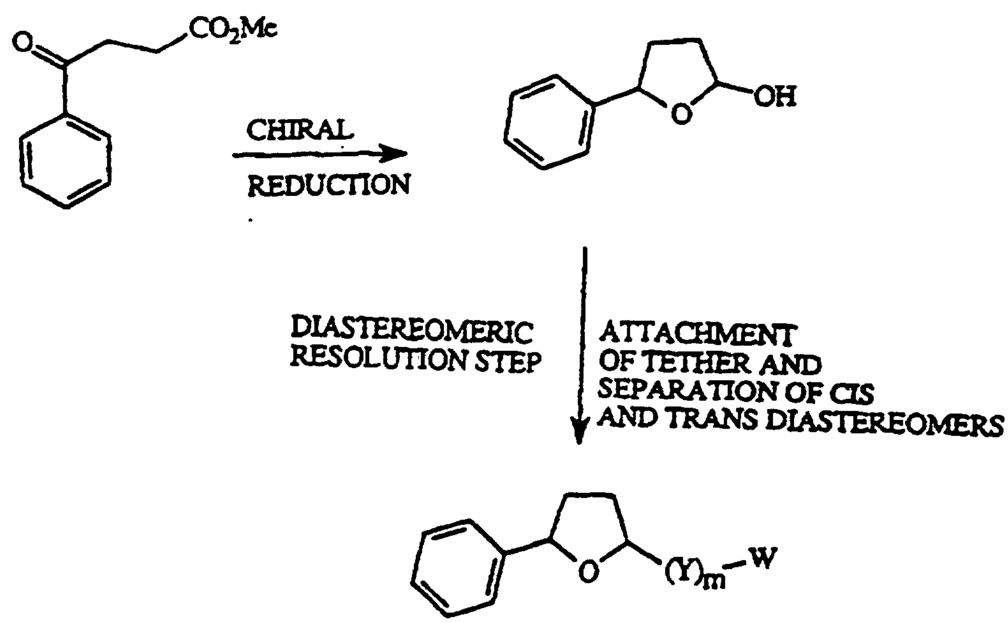


1,3-Disubstituted cyclopentanes can be prepared using the procedure of Graham, et al. (1.3-Diaryl Cyclopentanes: A New Class of Potent PAF Receptor Antagonists. 197th ACS National Meeting, Dallas, Texas, April 9-14, 1989, Division of Medicinal Chemistry, poster no. 25 (abstract)), or by other known methods.

A general procedure for preparing a hydroxyurea is shown below in Scheme 1:

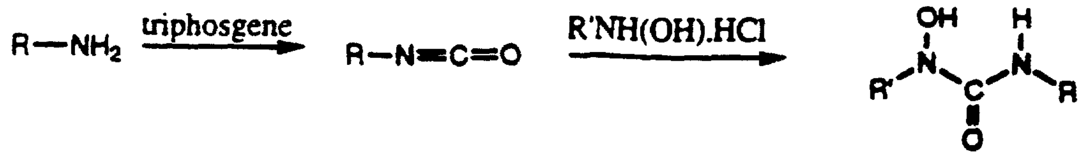


**Scheme 1 Preparation of Hydroxyureas**

General procedures for preparing reverse hydroxyureas are shown in Scheme 2:

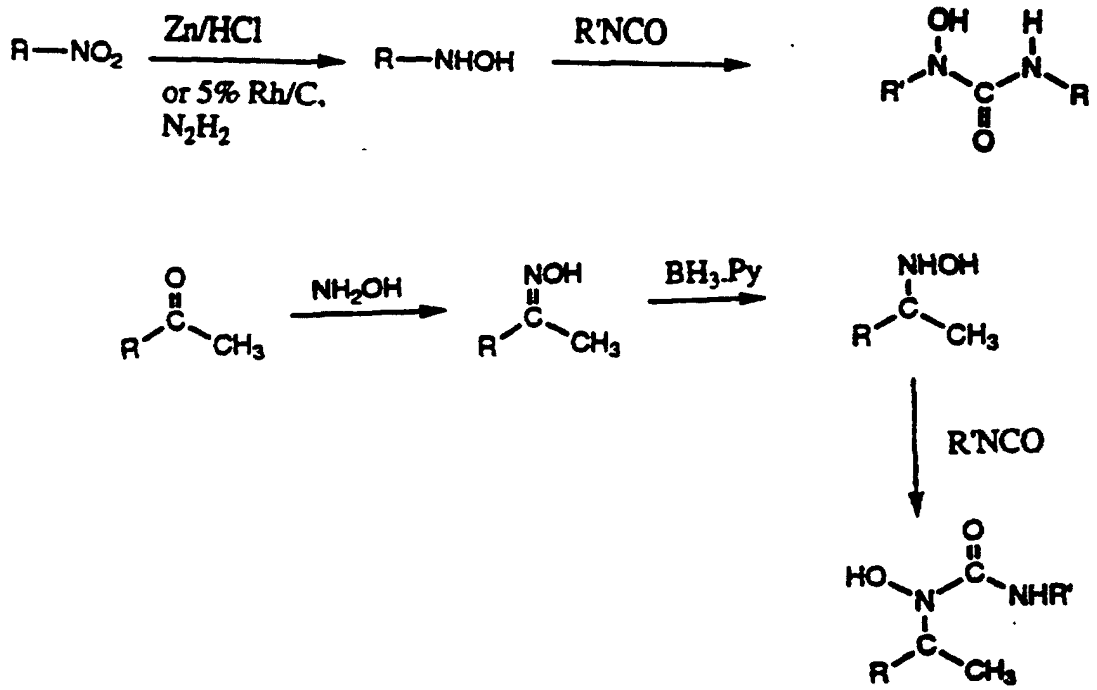


**Scheme 2 Preparation of Reverse Hydroxyureas**

A general procedure for preparing a hydroxamic acid is shown in Scheme 3:

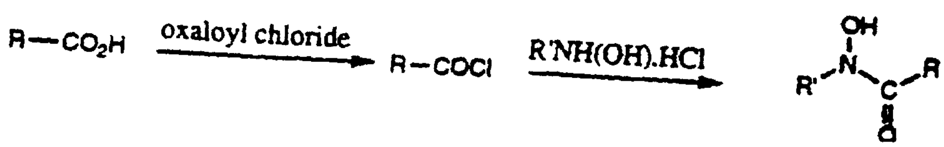


**Scheme 3 Preparation of Hydroxamic Acids**

A general procedure for preparing a reverse hydroxamic acid is shown in Scheme 4:

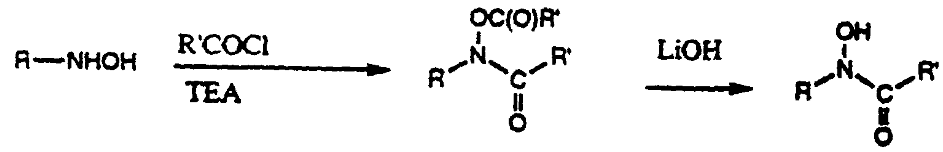


**Scheme 4 Preparation of Reverse Hydroxamic Acids**

Scheme 5 shows the synthesis of 2-(3,4,5-trimethoxyphenyl)-5-[3-(N'-substituted-N'-hydroxyureidyl)propoxy] tetrahydrofuran (**1-4**) and 2-(4-fluorophenyl)-5-[3-(N'-substituted-N'-hydroxyureidyl) propoxy] tetrahydrofuran (**9-12**):

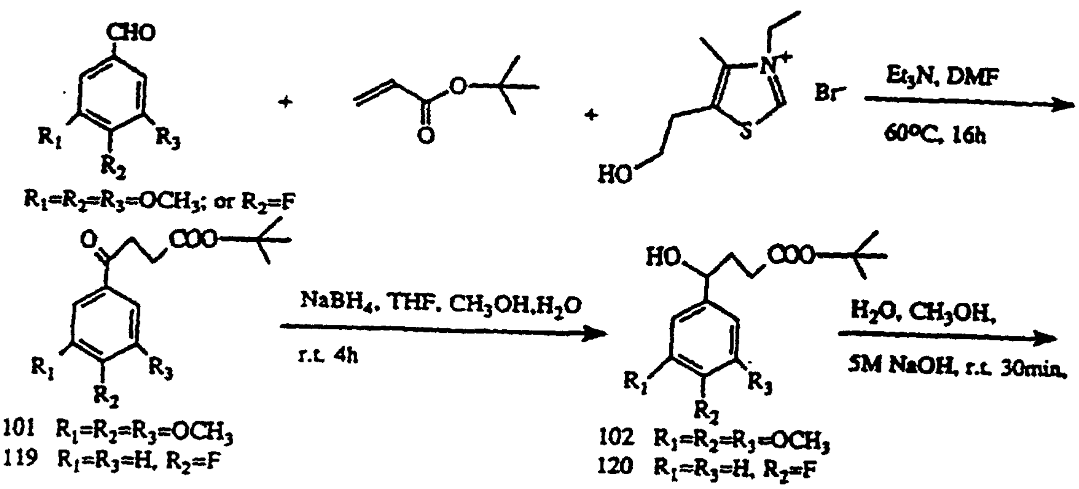


$R_1=R_2=R_3=OCH_3$; or $R_2=F$

101 $R_1=R_2=R_3=OCH_3$
119 $R_1=R_3=H$, $R_2=F$

102 $R_1=R_2=R_3=OCH_3$
120 $R_1=R_3=H$, $R_2=F$

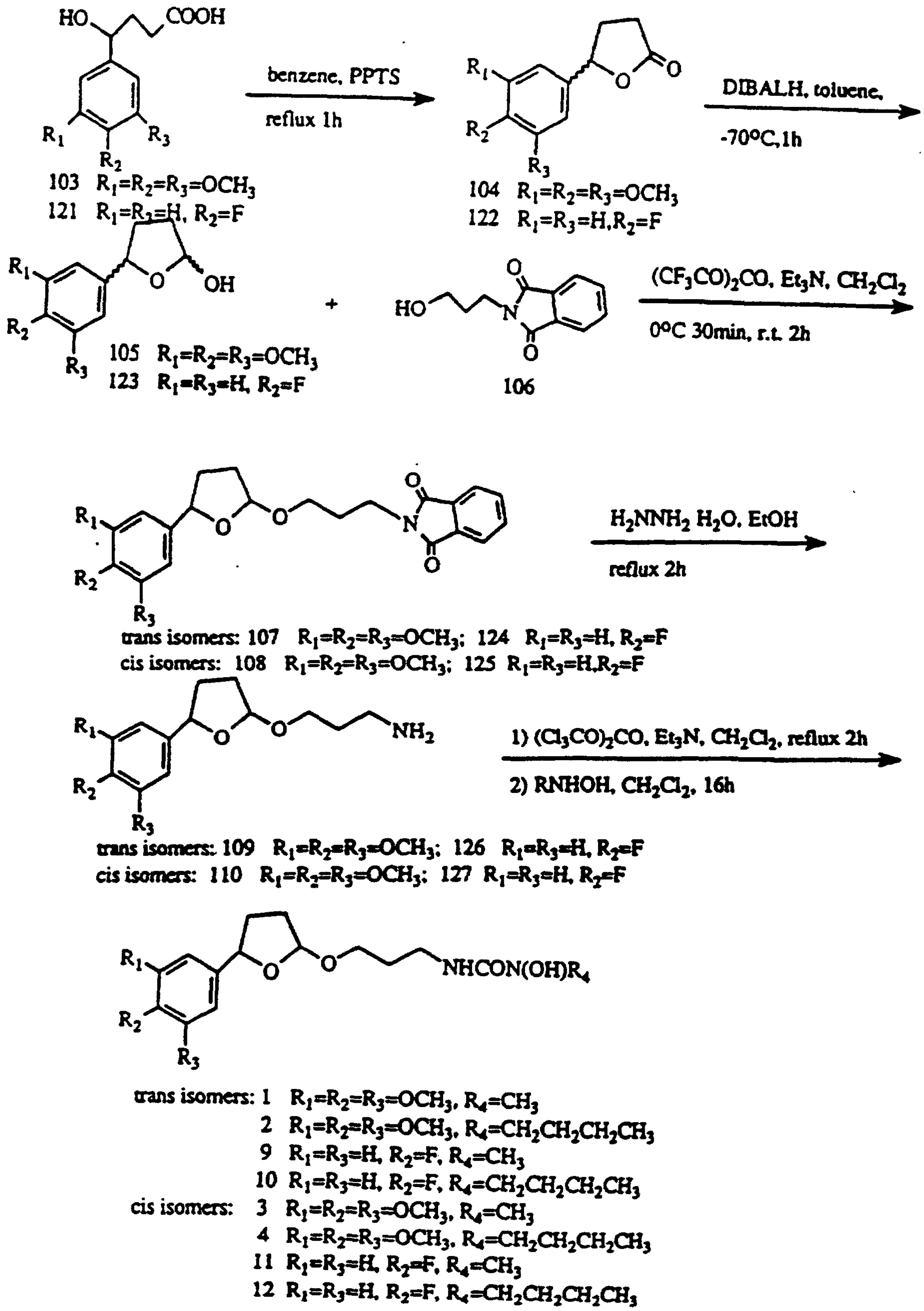


103 $R_1=R_2=R_3=OCH_3$
121 $R_1=R_3=H$, $R_2=F$

104 $R_1=R_2=R_3=OCH_3$
122 $R_1=R_3=H$, $R_2=F$

105 $R_1=R_2=R_3=OCH_3$
123 $R_1=R_3=H$, $R_2=F$

106

trans isomers: 107 $R_1=R_2=R_3=OCH_3$; 124 $R_1=R_3=H$, $R_2=F$
cis isomers: 108 $R_1=R_2=R_3=OCH_3$; 125 $R_1=R_3=H$, $R_2=F$

trans isomers: 109 $R_1=R_2=R_3=OCH_3$; 126 $R_1=R_3=H$, $R_2=F$
cis isomers: 110 $R_1=R_2=R_3=OCH_3$; 127 $R_1=R_3=H$, $R_2=F$

trans isomers: 1 $R_1=R_2=R_3=OCH_3$, $R_4=CH_3$
2 $R_1=R_2=R_3=OCH_3$, $R_4=CH_2CH_2CH_2CH_3$
9 $R_1=R_3=H$, $R_2=F$, $R_4=CH_3$
10 $R_1=R_3=H$, $R_2=F$, $R_4=CH_2CH_2CH_2CH_3$
cis isomers: 3 $R_1=R_2=R_3=OCH_3$, $R_4=CH_3$
4 $R_1=R_2=R_3=OCH_3$, $R_4=CH_2CH_2CH_2CH_3$
11 $R_1=R_3=H$, $R_2=F$, $R_4=CH_3$
12 $R_1=R_3=H$, $R_2=F$, $R_4=CH_2CH_2CH_2CH_3$

Scheme 5

Scheme 6 shows the synthesis of 2-(2,4,5-trimethoxyphenyl)-5-(3-hydroxyureidyl propoxy)tetrahydrofuran (**13**) and 2-(**4**-fluorophenyl)5-(3-hydroxyureidylpropoxy)tetrahydrofuran (**14, 15**)

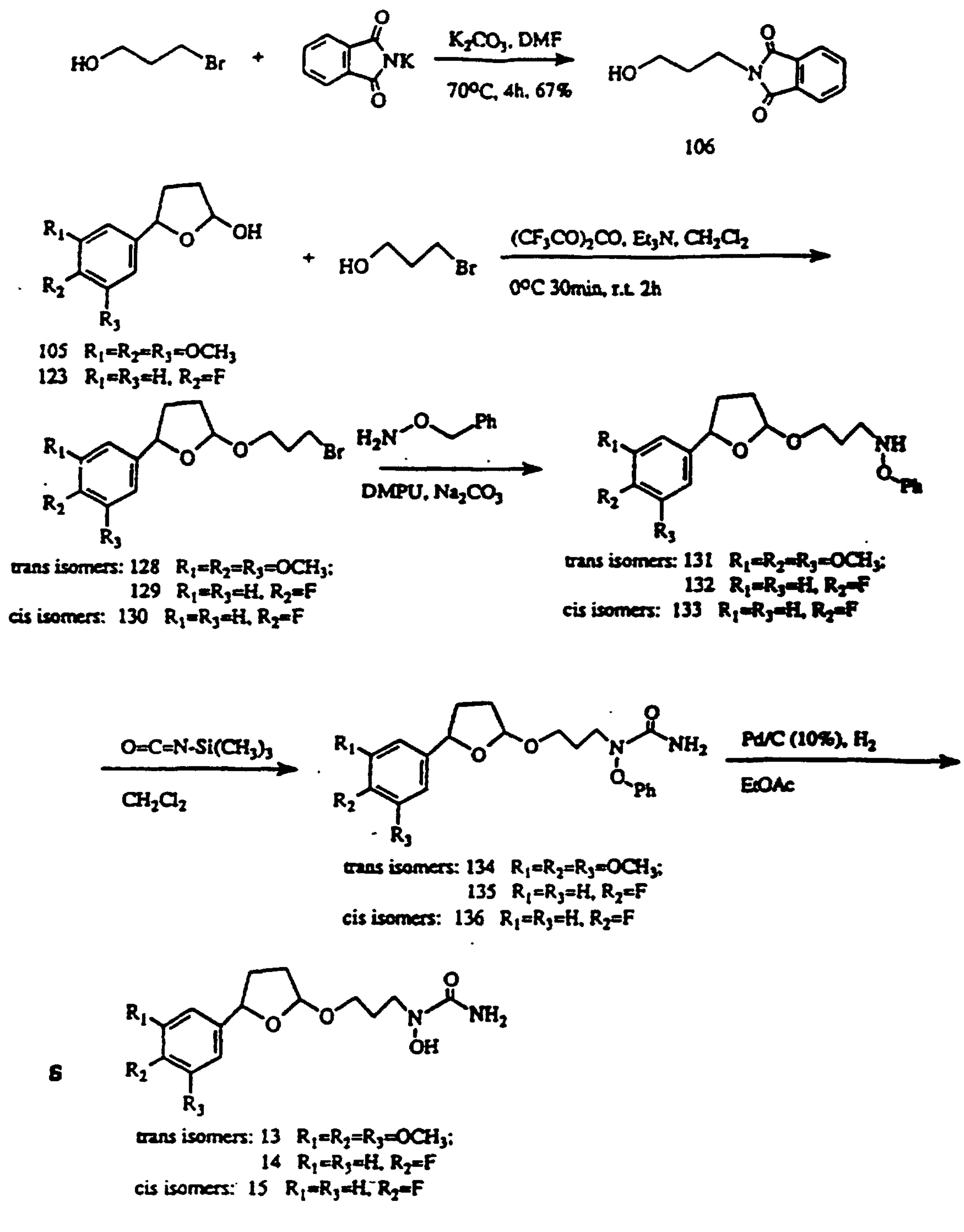


106

105 $R_1$=$R_2$=$R_3$=$OCH_3$
123 $R_1$=$R_3$=H, $R_2$=F

trans isomers: 128 $R_1$=$R_2$=$R_3$=$OCH_3$;
129 $R_1$=$R_3$=H, $R_2$=F
cis isomers: 130 $R_1$=$R_3$=H, $R_2$=F

trans isomers: 131 $R_1$=$R_2$=$R_3$=$OCH_3$;
132 $R_1$=$R_3$=H, $R_2$=F
cis isomers: 133 $R_1$=$R_3$=H, $R_2$=F

trans isomers: 134 $R_1$=$R_2$=$R_3$=$OCH_3$;
135 $R_1$=$R_3$=H, $R_2$=F
cis isomers: 136 $R_1$=$R_3$=H, $R_2$=F

S

trans isomers: 13 $R_1$=$R_2$=$R_3$=$OCH_3$;
14 $R_1$=$R_3$=H, $R_2$=F
cis isomers: 15 $R_1$=$R_3$=H, $R_2$=F

Scheme 6

Scheme 7 shows the synthesis of 2-(3,4-dimethoxyphenyl)-5-[3-N'-substituted-N'-hydroxyureidyl propoxy]tetrahydrofuran (**5-8**):

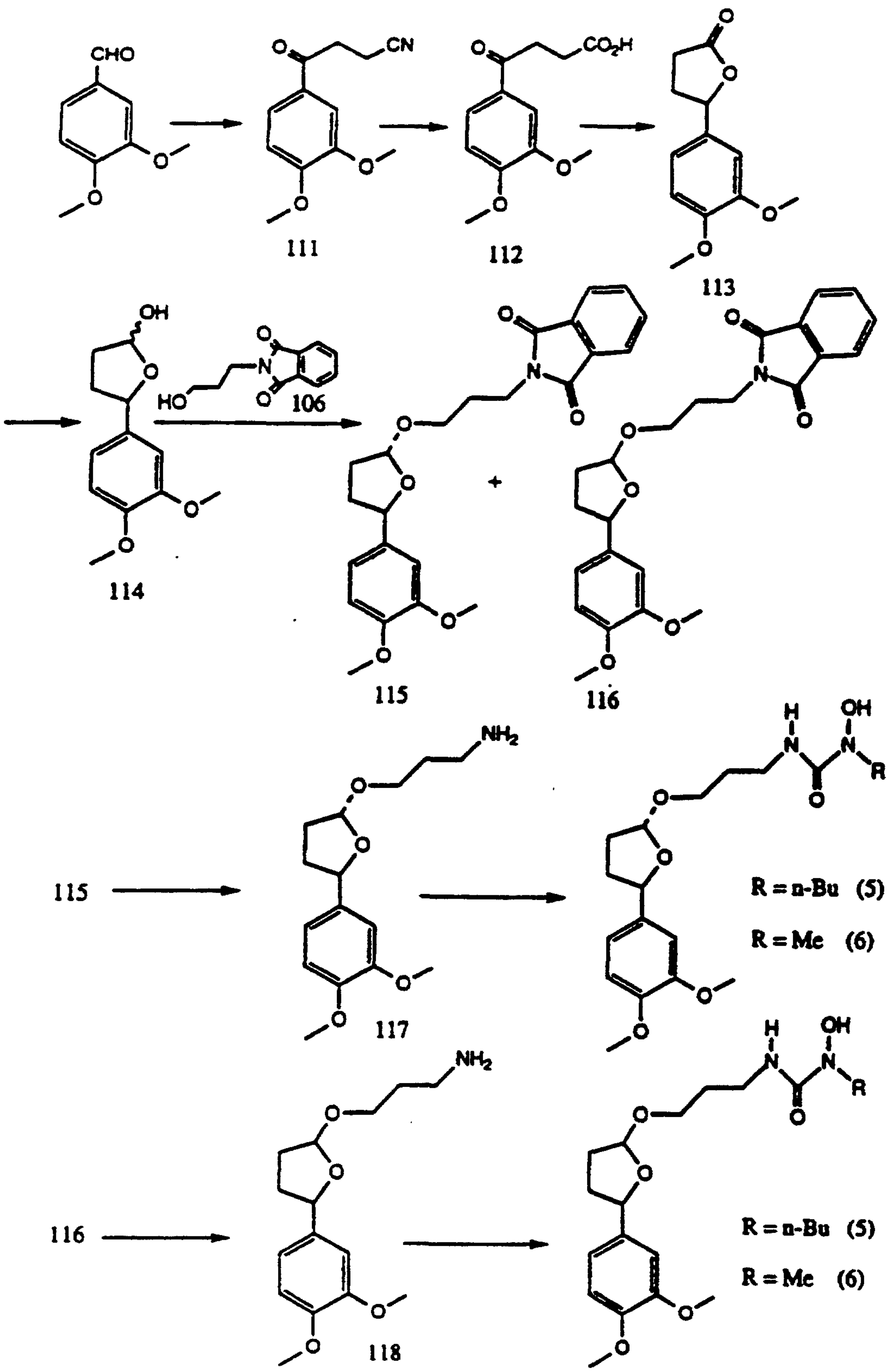

111

112

113

106

114

115 + 116

115

117

R = n-Bu (5)

R = Me (6)

116

118

R = n-Bu (5)

R = Me (6)

Scheme 7

The following examples are merely illustrative, and not intended to limit the scope of the invention.

**Example 1 Preparation of 2-(3,4,5 trimethoxyphenyl)-5-[3-(N'-substituted-N'-hydroxyureidyl)propoxy] tetrahydrofuran (1-4) and 2-(4-fluorophenyl)-5-[3-(N'-substituted-N'-hydroxyureidyl) propoxy] tetrahydrofuran (9-12)**

**(a) Preparation of 4-(3,4,5-trimethoxyphenyl)-4-ketone-butyric acid t-butyl ester (compound 101)**

3,4,5-Trimethoxybenzaldehyde (8.0 g, 40.77 mmol), tert-butyl acrylate (5.29 g, 41.29 mmol) and the catalyst 3-ethyl-5-(2-hydroxyethyl)-4-methylthiazolium bromide (3.52 g, 13.95 mmol) were dissolved in 50 mL dimethyl formamide (DMF). To this solution was added 5.86 mL triethylamine. The reaction mixture was stirred at 60°C for 16 hours, cooled to room temperature and quenched by adding 10% HCl (PH 1-2), and extracted with dichloromethane. The organic layer was washed with water and saturated NaCl solution, dried over $MgSO_4$, filtered and evaporated *in vacuo* to an oil. The product was purified by column chromatography (silica, 3:1 hexane/ethyl acetate) (4.5 g, 34%). $^1H$ NMR ($CDCl_3$): 1.46(2,9H); 2.70(t,2H); 3.24(t,2H); 3.92(s,9H); 7.25(s,2H).

**(b) Preparation of 4-(4-fluorophenyl)-4-ketone-butyric acid t-butyl ester (compound 119)**

This compound was prepared using a process similar to that set forth in Example 1(a), replacing the 3,4,5-trimethoxy-benzaldehyde with 4-fluorobenzaldehyde. $^1H$ NMR ($CDCl_3$): 1.45(s,9H); 2.70(t,2H); 3.23(t,2H); 7.12(m,2H); 8.02(m,2H)

**(c) Preparation of 4-(3,4,5-trimethoxyphenyl)-4-hydroxy-butyric acid t-butyl ester (compound 102)**

The ketone ester **101** (1.09 g, 3.36 mmol) was added to 10 mL THF and 20 mL methanol. An aqueous solution of $NaBH_4$ (127.3 mg, 3.36 mmol in 5 mL water) was added to this mixture in a dropwise manner at 0°C. The reaction mixture was stirred at room temperature for 4 hours, quenched with water and extracted with ethyl acetate. The organic layer was washed with water, saturated NaCl solution, dried over $MgSO_4$, filtered and evaporated *in vacuo* to provide the product (1.13 g, 103%). $^1H$ NMR ($CDCl_3$): 1.46(s,9H); 2.02(m,2H); 2.37(t,2H); 3.84(s,3H); 3.88(s,6H); 4.70(m,1H); 6.58(s,2H).

**(d) Preparation of 4-(4-fluorophenyl)-4-hydroxy-butyric acid t-butyl ester (compound 120)**

This compound was prepared from 119 using a procedure similar to that set forth in Example 1(c), replacing compound **101** with compound 119. $^1H$ NMR ($CDCl_3$): 1.44(s,9H); 2.00(m,2H); 2.32(m,2H); 4.72(m,1H); 7.01(m,2H); 7.30(m,2H).

**(e) Preparation of 4-(3,4,5-trimethoxyphenyl)-δ-lactone (compound 104)**

The hydroxy ester **102** (1.13 g, 3.47 mmol) was added to 4 mL methanol, 1.5 mL water and 5M aqueous sodium hydroxide solution (4.5 mL). The reaction mixture was stirred at room temperature for 30 minutes and then 12 mL of saturated aqueous $NaHCO_3$ solution was added. The aqueous phase was washed with ether, acidified to pH 1-2 by adding conc. HCl, and extracted with benzene (2 x 30 mL). The benzene layer was checked by TLC which showed that some of the lactone has been formed. PPTS (10mg) was added to the benzene extract and the mixture was refluxed for 1 hour to remove water. The reaction mixture was washed with saturated $NaHCO_3$ solution and evaporated *in vacuo* to provide the desired lactone as a solid (700 mg, 80%). $^1H$ NMR ($CDCl_3$): 2.20(m,1H); 2.68(m,3H); 3.85(s,3H); 3.88(s,6H); 5.46(m,1H); 6.55(s,2H).

**(f) Preparation of 4-(4-fluorophenyl)-δ-lactone (compound 122)**

This compound was prepared from **120** using a procedure similar to that set forth in Example 1(e), replacing compound **102** with compound **120.** $^1H$ NMR ($CDCl_3$): 2.20(m,1H); 2.68(m,3H); 5.50(m,1H); 7.10(t,2H); 7.32(m,2H).

**(g) Preparation of 2-(3,4,5-trimethoxyphenyl)-5-hydroxytetrahydrofuran (105)**

Lactone **104** (6.86 g, 27.22 mmol) was dissolved in dry toluene (100 mL) and the solution was cooled to -70°C. A 1.5 M toluene solution of DIBALH (28 mL) was added to the solution in a dropwise manner. The reaction mixture was stirred at -70°C for 1 hour. The reaction was quenched through the addition of methanol (11 mL) while maintaining a temperature of <-60°C. The mixture was warmed to -20°C followed by the addition of saturated aqueous potassium sodium tartrate solution (96 mL) while the reaction temperature was maintained between -10 and 0°C. The reaction

mixture was stirred at 0°C for 3 hours and then the two phases were separated. The aqueous layer was extracted with ethyl acetate. The combined organic layers were washed with water, saturated NaCl solution, and then concentrated *in vacuo* to afford the product (6.51 g, 94%). $^1$H NMR ($CDCl_3$): 1.82-2.48(m,4H); 3.84(s,3H); 3.88(s,6H); 4.97, 5.20(m, 1H); 5.65, 5.79(m,1H); 6.56, 6.70(s,2H).

**(h) Preparation of 2-(4-fluorophenyl)-5-hydroxy-tetrahydrofuran (123)**

This compound was prepared from **122** using a procedure similar to that set forth in Example 1(g), replacing compound **104** with compound 122. $^1$H NMR ($CDCl_3$): 1.79(m,1H); 1.95-2.10(m,1H); 2.20-2.32(m,1H); 2.48(m,1H); 5.00 & 5.22(m,1H); 5.63 & 5.78(m,1H); 7.04(m,2H); 7.30 & 7.41(m,2H).

**(i) Preparation of trans and cis 2-(3,4,5-trimethoxyphenyl)-5-(3-phthalimidyl propoxy) tetrahydrofuran (compounds 107, 108)**

Compound **105** (1.14 g, 4.49 mmol) was dissolved in 4 mL dichloromethane. Triethylamine (681.4 mg, 6.73 mmol) was added to this solution. The reaction mixture was cooled with an ice bath and trifluoroacetic anhydride (1.41 g, 6.73 mmol) was added in a dropwise manner. The reaction mixture was stirred at 0°C for 30 minutes and then 3-phthalimidylpropanol (**106**) (2.4 g, 13.26 mmol) was added. The reaction mixture was warmed to room temperature and stirred at room temperature for 2 hours. The reaction was quenched with saturated aqueous $NaHCO_3$ solution and extracted with ethyl acetate. The organic layer was washed with water and saturated NaCl solution, dried over $MgSO_4$, filtered and evaporated *in vacuo* to an oil which was purified by column chromatography (silica, 2:1 hexane/ ethyl acetate) (**107**: 522 mg (trans); **108**: 271 mg (cis); 1:1 mixture of **107** and **108:** 110 mg; total yield 46%). $^1$H NMR ($CDCl_3$): **107:** 1.70(m,1H); 1.82(m,1H); 2.00(m,2H); 2.02(m,1H); 2.28(m,1H) 3.46(m,1H); 3.83(s,3H); 3.84(m,3H); 3.88 (s,6H); 4.99(t,1H); 5.30(dd,1H); 6.56(s,2H); 7.72(m,2H); 7.85(m,2H). **108**: 1.95(m,3H); 2.00(m,2H); 2.20(m,1H); 3.51 (m,1H); 3.83(s,3H); 3.85(m,2H); 3.88(s,6H); 3.92(m,1H); 4.90(m,1H); 5.16(dd,1H); 6.60(s,2H); 7.72(m,2H); 7.84(m, 2H).

In order to determine the stereochemistry of this molecule, an NOE difference experiment was carried out.

Trans isomer (**107**): In this experiment the triplet at 4.99 ppm was irradiated with a very low rf decoupling pulse and the data work-up was done so as to only measure the presence of an increase in signal. This would represent a positive NOE effect and would indicate the close spacial relationship of these protons. In this experiment an NOE was found for the multiplet at 2.25-2.36 ppm which is a furan ring proton. Another NOE was also seen for the aromatic protons, indicating that this triplet represents the benzylic proton. There was not an NOE observed for the double doublet at 5.30 ppm indicating this was the trans isomer.

Cis isomer (**108**): In order to determine the stereochemistry of this molecule an NOE difference experiment was carried out. In this experiment the multiplet at 4.88-4.93 ppm was irradiated with a very low rf decoupling pulse and the data work-up was done so as to only measure the presence of an increase in signal. This would represent a positive NOE effect and would indicate the close spacial relationship of these protons. In this experiment an NOE was found for the doublet at 5.16 ppm which is the other methine furan proton. Another NOE was also seen for the aromatic protons indicating this triplet represents the benzylic proton. There was also an NOE observed for the multiplet at 1.93-2.20 ppm for the other furan methylene protons.

**(j) Preparation of 2-(4-Fluorophenyl)-5-(3-phthalimidyl propoxy) tetrahydrofuran (compounds 124, 125)**

These compounds were prepared from **123** using a procedure similar to that set forth in Example 1(i), replacing compound **105** with compound **123.** $^1$H NMR ($CDCl_3$): **124** (trans): 1.65(m,1H); 1.80(m,1H) 2.00(m,2H); 2.12(m,1H); 2.31(m,1H); 3.48(m,1H); 3.82(m,3H); 5.02(t,1H); 5.28(dd,1H); 7.00(t,2H); 7.29(m,2H); 7.71(m,2H); 7.85(m,2H). **125** (cis): 1.90(m,2H); 1.99(m,4H); 2.19(m,1H); 3.48(m,1H); 3.82(m,2H); 3.88(m,1H); 4.94(m,1H); 5.15(dd,1H); 7.00(t,2H); 7.30(m,2H); 7.71(m,2H); 7.84(m,2H).

**(k) Preparation of 3-phthalimidylpropanol (compound 106)**

3-Bromopropanol (4.0 g, 28.78 mmol), potassium phthalimide (8.0 g, 43.17 mmol) and potassium carbonate (4.0 g, 28.78 mmol) were added to 20 mL DMF. The reaction mixture was stirred at 70°C for 4 hours, quenched with water and extracted with ethyl acetate. The organic layer was washed with water, saturated NaCl solution and evaporated *in vacuo* to a solid which was crystallized in ethyl acetate (3.5 g, 67%).

**(l) Preparation of trans and cis 2-(3,4,5-trimethoxyphenyl)-5-(3-aminopropoxy)tetrahydrofuran (compounds 109, 110)**

Compound **107** (455 mg, 1.03 mmol) and hydrazine monohydrate (165.3 mg, 5.16 mmol) were added to 2 mL ethanol. The reaction mixture was refluxed for 2 hours, quenched with water and extracted with dichloromethane. The organic layer was washed with water and saturated NaCl solution, dried over $MgSO_4$, filtered and evaporated *in vacuo* to provide the trans product **109** (225 mg, 70%). $^1H$ NMR ($CDCl_3$): 1.75(m,2H); 1.78(m,1H); 1.96(m,1H); 2.20 (m,1H); 2.40(m,1H); 2.82(t,2H); 3.55(m,1H); 3.81(m,1H); 3.83(s,3H); 3.87(s,6H); 5.00(t,1H); 5.34(dd,1H); 6.56(s,2H).

The cis isomer **110** was prepared from **108** using a procedure similar to that described for **109**. $^1H$ NMR ($CDCl_3$): 1.76(m,2H); 2.08(m,3H); 2.27(m,1H); 2.82(t,2H); 3.55(m,1H); 3.84(s,3H); 3.88(s,6H); 3.92(m,1H); 4.95(m, 1H); 5.20(m,1H); 6.64(s,2H).

**(m) Preparation of 2-(4-fluorophenyl)-5-(3-aminopropoxy) tetrahydrofuran (compounds 126, 127)**

These compounds were prepared from **124** and **125** using a procedure similar to that set forth in Example 1 (1), replacing compounds **107** and **108** with compounds **124** and **125.** $^1H$ NMR ($CDCl_3$): **124** (trans): 1.75(m,3H); 1.96 (m,1H) 2.20(m,1H); 2.40(m,1H); 2.82(t,2H); 3.54(m,1H); 3.83(m,1H); 5.05(t,1H); 5.32(dd,1H); 7.01(t,2H); 7.30(m,2H). **125** (cis): 1.74(m,2H); 1.97(m,1H); 2.05(m,2H); 2.25(m,1H); 2.77(t,2H); 3.47(m,1H); 3.85(m,1H); 4.95(m,1H); 5.15(dd, 1H); 7.00(t,2H); 7.34(m,2H).

**(n) Preparation of trans and cis 2-(3,4,5-trimethoxyphenyl)-5-[3-(N'-methyl-N'-hydroxyureidyl) propoxy] tetrahydrofuran (compounds 1, 3)**

Compound **109** (60 mg, 0.19 mmol) and triphosgene (23 mg, 0.078 mmol) were dissolved in 3 mL dichloromethane. Triethylamine (29.3, 0.29 mmol) was added to this solution. The reaction mixture was refluxed for 2 hours and then cooled with ice bath. Triethylamine (34.0 mg, 0.34 mmol) and methylhydroxyamine hydrochloride (32.2 mg, 0.39 mmol) were added to the cold solution. The reaction was stirred at room temperature for 16 hours, quenched with water and extracted with dichloromethane. The organic layer was washed with saturated NaCl solution and evaporated *in vacuo* to an oil which was purified by preparative TLC (silica, ethyl acetate) to provide the trans product **1** (51 mg, 69%). $^1H$ NMR ($CDCl_3$): 1.82(m,3H); 1.95(m,1H); 2.22(m,1H); 2.40(m,1H); 3.15(s,3H); 3.40(m,2H); 3.58(m,1H); 3.84 (s,3H); 3.85(m,1H); 3.88(s,6H); 5.00(t,1H); 5.33(m,1H); 6.32(m,1H); 6.56(s,2H); 7.37(s,1H).

The cis isomer 3 was prepared from **110** using a procedure similar to that described for **1**. $^1H$ NMR ($CDCl_3$): 1.83(m,2H); 2.07(m,3H); 2.28(m,1H); 3.13(s,3H); 3.35(m,2H); 3.55(m,1H); 3.84(s,3H); 3.87(s,6H); 3.88(m,1H); 4.97 (m,1H); 5.20(m,1H); 6.22(m,1H); 6.63(s,2H); 7.37(s,1H).

**(o) Preparation of 2-(4-fluorophenyl)-5-[3-(N'-methyl-N'-hydroxyureidyl)propoxy ] tetrahydrofuran (compounds 9, 11)**

These compounds were prepared from **126** and **127** using a procedure similar to that set forth in Example 1 (n) replacing compounds **109** and **110** with compounds **126** and **127.** $^1H$ NMR ($CDCl_3$): **9** (trans): 1.70(m,1H); 1.78(m, 2H); 1.96(m,1H); 2.19(m,1H); 2.40(m,1H); 3.10(s,3H); 3.31(m,2H); 3.51(m,1H); 3.83(m,1H); 5.05(t,1H); 5.30(dd,1H); 6.38(t,1H); 7.01(t,2H); 7.28(m,2H). **11** (cis): 1.80(m,2H); 2.05(m,3H); 2.24(m,1H); 3.06(s,3H); 3.30(m,2H); 3.48(m,1H); 3.86(m,1H); 4.98(m,1H); 5.16(dd,1H); 6.30(t,1H); 7.02(t,2H); 7.31(m,2H); 8.08(bs,1H)

**(p) Preparation of trans and cis 2-(3,4,5-trimethoxyphenyl)-5[3-(N'-n-butyl-N'-hydroxyureidyl) propoxy] tetrahydrofuran (compounds 2,4)**

Compound **109** (60 mg, 0.19 mmol) and triphosgene (23 mg, 0.078 mmol) were dissolved in 3 mL dichloromethane. Triethylamine (29.3, 0.29 mmol) was added to this solution. The reaction mixture was refluxed for 2 hours and then cooled with ice bath. Butylhydroxyamine (51.4 mg, 0.29 mmol) was added to the cold solution. The reaction mixture was stirred at room temperature for 16 hours, quenched with water and extracted with dichloromethane. The organic layer was washed with saturated NaCl solution and evaporated *in vacuo* to an oil. The trans product **2** was. separated by preparative TLC (silica, ethyl acetate) (46.9 mg, 57%). $^1H$ NMR ($CDCl_3$): 0.93(t,3H); 1.35(m,2H); 1.58 (m,2H); 1.81(m,3H); 1.96(m,1H); 2.21(m,1H); 2.40(m,1H); 3.38(m,2H); 3.50(m,2H); 3.57(m,1H); 3.83(s,3H); 3.85(m, 1H); 3.88(s,6H); 5.00(t,1H); 5.32(m,1H); 6.32(m,1H); 6.56(s,2H).

The cis isomer **4** was prepared from **110** using a procedure similar to that described for **2**. $^1H$ NMR ($CDCl_3$): 0.92(t,3H); 1.32(m,2H); 1.58(m,2H); 1.81(m,2H); 2.08(m,3H); 2.28(m,1H); 3.35(m,2H); 3.47(m,2H); 3.54(m,1H); 3.84 (s,3H); 3.87(s,6H); 3.88(M,1H); 4.97(m,1H); 5.20(m,1H); 6.22(m,1H); 6.63(s,2H).

**(q) Preparation of 2-(4-fluorophenyl)-5[3-(N'-n-butyl-N'-hydroxyureidyl)propoxy] tetrahydrofuran (compounds 10,12)**

These compounds were prepared from **126** and **127** using a procedure similar to that set forth in Example 1 (p) replacing compounds **109** and **110** with compounds **126** and **127.** $^1$H NMR ($CDCl_3$): **10** (trans): 0.90(t,3H); 1.30(m, 2H); 1.55(m,2H); 1.70(m,1H); 1.78(m,2H); 1.96(m,1H); 2.19(m,1H); 2.40(m,1H); 3.31(m,2H); 3.44(m,2H); 3.52(m,1H); 3.82(m,1H); 5.05(t,1H); 5.30(dd,1H); 6.32(t,1H) 7.00(t,2H); 7.28(m,2H); 7.55(bs,1H).
**12** (cis): 0.90(t,3H); 1.30(m,2H); 1.52(m,2H); 1.80(m,2H); 2.04(m,3H); 2.24(m,1H); 3.30(m,2H); 3.40(m,2H); 3.48(m, 1H); 3.85(m,1H) 4.98(t,1H) 5.16(dd,1H); 6.27(t,1H); 7.03(t,2H); 7.32(m,2H); 7.53(bs,1H).

**Example 2 Preparation of 2-(3,4-Dimethoxyphenyl)-5-[3-N'-substituted-N'-hydroxyureidyl propoxy] tetrahydrofuran (5-8)**

**(a) Preparation of 4-(3',4'-dimethoxyphenyl)-4-oxobutyronitrile (111).**

A single portion of neat acrylonitrile (3.2 ml, 0.048 mol) and triethylamine (5 ml, 0.11 mol) was added to a stirred mixture of 3,4-dimethoxybenzaldehyde (7.8 g, 0.047 mol) and 3-benzyl-5-(2-hydroxyethyl)-4-methylthiazolium chloride (5.3 g, 0.02 mol) in dry dimethylformamide (25 ml) under argon. The mixture was left overnight at room temperature. The reaction was diluted with water and extracted with ethyl acetate (3 X 100 ml). The organic extract was washed with water (3 X 100 ml), brine (3 X 100 ml) and the solvent was removed under reduced pressure to give an amber oil. Analysis by TLC (silica gel; ethyl acetate:hexanes, 1:1) revealed a mixture of three spots at Rf 0.80 (starting aldehyde), 0.50 (Compound 1) and 0.30 (unknown by-product). The sample was purified by column (flash) chromatography on silica gel 60 (230-400 mesh) and eluted with ethyl acetate:hexanes (1:1) to give the desired compound (2.26 g, 22%) as a yellow solid. $^1$H NMR ($CDCl_3$) 2.78 (t, 2H, J=8 Hz), 3.33 (t, 2H, J=8 Hz), 3.96 (s, 3H), 3.98 (s, 3H), 6.90(d, 1H, J=8.5 Hz), 7.52 (d, J=2 Hz, 2H), 7.58 (dd, J=2 and 8 Hz, 2H).

**(b) Preparation of 4-(3',4'-dimethoxyphenyl)-4-oxobutyric acid (112).**

A stirred solution of 4-(3',4'-dimethoxyphenyl)-4-oxobutyronitrile (**111**) (2.26 g, 0.01 mol) in acetic acid (15 ml) and hydrochloric acid (12 N, 40 ml) was heated at reflux for 1.5 hours and cooled to room temperature. The solvent was removed under reduced pressure to give a brown solid. Recrystallization from water gave **112** as light tan crystals (1.57 g, 66%). $^1$H NMR ($CDCl_3$) 2.80 (t, J=7.5 Hz, 2H), 3.30 (t, J=7.5 Hz, 2H), 3.94 (s, 3H), 3.96 (s, 3H), 6.89 (d, 1H, J=9 Hz), 7.55 (d, 1H, J=1Hz) and 7.64 (dd, 1H, 1 and 9 Hz).

**(c) Preparation of 4-(3',4'-dimethoxyphenyl)butyrolactone (113).**

A solution of sodium borohydride (0.89 g, 0.023 mol) in water (4 ml) was added dropwise (ca. 5 min) to a stirred solution of **112** (2.8 g, 0.012 mol) in dry, freshly distilled tetrahydrofuran (40 ml) and methanol (20 ml) under argon. The reaction was left overnight at room temperature. Analysis by TLC (silica gel; ethyl acetate:methanol:acetic acid, 9.5:0.5:few drops) indicated the presence of starting material. An additional charge of sodium borohydride (0.5 g, 0.013 mol) in water (2 ml) was added dropwise and the reaction left at room temperature for three hours. Analysis by TLC (same system as above) indicated the absence of starting material. The reaction was quenched with hydrochloric acid (6 N, 25 ml) and left at room temperature for 15 minutes. The mixture was extracted with ethyl acetate (3 X 75 ml). The organic extract was washed with water (3 X 75 ml), brine (3 X 75 ml) and the solvent removed under reduced pressure to give a tan solid (2.0 g, 75%). $^1$H NMR ($CDCl_3$) 2.18 - 2.25 (m, 1H), 2.59-2.70 (m, 3H), 3.89 (s, 3H), 3.90 (s, 3H), 5.44-5.49 (m, 1H) and 6.82-6.87 (m, 3H).

**(d) Preparation of 4-(3',4'-dimethoxyphenyl)butyrolactol (114).**

A solution of diisobutylaluminum hydride (1.5 M, 9 ml, 13.5 mmol) was added in a dropwise manner (ca. 30 min.) to **113** (2.0 g, 9 mmol) in dry toluene (40 ml) under argon which was cooled by a dry ice-acetone bath. The reaction was stirred at -78° C for one hour. Analysis by TLC (silica gel; ethyl acetate:hexanes, 1:1) revealed the absence of starting material and the presence of a new spot at Rf 0.38. The reaction was quenched with methanol (20 ml) and slowly warmed to 0° C. A saturated solution of sodium potassium tartrate (50 ml) was added and stirred at 0° C for 45 minutes. The mixture was extracted with ethyl acetate (3 X 100 ml) and the organic extract washed with water (3 X 75 ml) and brine (3 X 75 ml). Removal of the solvent under reduced pressure gave a dark amber oil (1.7 g, 84 %). $^1$H NMR ($CDCl_3$) (mixture of cis and trans isomers) 1.71- 2.49 (m, 8H), 2.91 (br s, 1H), 3.09 (br s, 1H), 3.89 (s, 6H), 3.90 (s, 6H), 4.97 (m, 1H), 5.19 (t, J=7Hz, 1H), 5.62 (m, 1H), 5.77 (m, 1H) and 6.82-7.28 (m, 6H).

**(e) Preparation of N-(3-hydroxypropyl)phthalimide (106).**

A mixture of 3-bromopropanol (4 g, 0.029 mol), potassium phthalate (8 g, 0.043 mol) and potassium carbonate (4 g, 0.029 mol) in dry DMF (50 ml) was stirred and heated at 70° C for four hours. The mixture was diluted with water (100 ml) and extracted with ethyl acetate (3 X 75 ml). The organic extract was washed with water (3 X 100 ml) and dried ($Na_2SO4$). Removal of the solvent under reduced pressure left a white solid which was extracted with benzene. The benzene extract was evaporated to a white solid and recrystallized from ethyl acetate-hexanes to give white crystals (1.27 g, 24%).

**(f) Preparation of trans and cis 2-(3',4'-dimethoxyphenyl)-5-[3-(N-phthaloyl)]propoxytetrahydrofuran (115 and 116).**

Triflic anhydride (0.68 ml, 4.8 mmol) was added in a single portion to a stirred solution of **114** (0.72 g, 3.2 mmol) in dry dichloromethane (20 ml) and triethylamine (0.68 ml, 4.9 mmol) under argon which was cooled using an ice bath. The reaction was stirred at 0°C for 30 minutes. N-(3-hydroxypropyl)phthalimide (**106**) (1.27 g, 7 mmol) was added to the reaction mixture and the solution was allowed to warm to room temperature and left at this temperature for two hours. The solution was quenched with aqueous sodium bicarbonate solution (saturated, 25 ml) and extracted with ethyl acetate (3 X 50 ml), brine (3 X 50 ml) and dried (sodium sulfate). Removal of the solvent under reduced pressure left an amber oil (2.02 g). Analysis of the oil by TLC (silica gel; ethyl acetate:hexanes, 1:1) revealed the presence of four spots at Rf 0.80, 0.60, 0.50 and 0.35. The spots at Rf 0.60 and 0.50 were in a 2:1 ratio. The sample was purified by column chromatography (flash) on silica gel (230-400 mesh) and eluted with ethyl acetate:hexanes (3:7) to give first the substance at Rf 0.60 as a clear and colorless oil (0.40 g, 30%), identified as trans 2-(3',4'-dimethoxyphenyl)--5-[3-(N-phthaloyl)]-propoxytetrahydrofuran (**115**) (0.40 g, 30%). $^1$H NMR ($CDCl_3$) 1.34-1.94 (m, 2H), 1.96-2.05 (m, 2H), 2.09-2.20 (m, 1H), 2.25-2.36 (m, 1H), 3.46-3.53 (m, 1H), 3.84 (t, 9Hz, 2H), there is also a hidden 1 proton multiplet here, 3.88 (s, 3H), 3.91 (s, 3H), 5.01 (t, 7.3Hz, 1H), 5.30 (dd, J=2 and 5 Hz, 1Hz), 6.82-6.90 (m, 3 H), 7.71-7.74 (m, 2H) and 7.84-7.88 (m, 2H).

In order to determine the stereochemistry of this molecule and NOE difference experiment was carried out. In this experiment the triplet at 5.01 ppm was irradiated with a very low rf decoupling pulse and the data work-up was done so as to only measure the presence of an increase in signal. This would represent a positive NOE effect and would indicate the close spatial relationship of these protons. In this experiment an NOE was found for the multiplet at 2.25-2.36 ppm which is a furan ring proton. Another NOE was also seen for the aromatic protons indicating this triplet presents the benzylic proton. There was not an NOE observed for the double doublet at 5.30 ppm indicating this was the trans isomer.

Continued elution with the same solvent system gave the spot at Rf 0.50 as a colorless oil (0.21 g, 15%), identified as cis 2-(3',4'-dimethoxyphenyl)-5-[3-(N-phthaloyl)]propoxytetrahydrofuran (**116**). $^1$H NMR ($CDCl_3$) 1.92 - 2.12 (m, 6H), 3.44-3.52 (m, 1H), 3.86 (s, 3H), 3.88 (s, 3H), 3.76-3.93 (m, 3H), 4.89-4.94 (m, 1H), 5.35 (d, J=4 Hz), 6.89 (d, J=8 Hz), 6.87 (dd, J=2 and 8 Hz), 6.92 (d, J=2 Hz), 7.69-7.72 (m, 2H) and 7.82-7.85 (m, 2H).

In order to determine the stereochemistry of this molecule an NOE difference experiment was carried out. In this experiment the multiplet at 4.89-4.94 ppm was irradiated with a very low rf decoupling pulse and the data work-up was done so as to only measure the presence of an increase in signal. This would represent a positive NOE effect and would indicate the close spatial relationship of these protons. In this experiment an NOE was found for the doublet at 5.35 ppm which is the other methine furan proton. This indicates that this molecule is the cis isomer. Another NOE was also seen for the aromatic protons indicating this triplet presents the benzylic proton. There was also an NOE present for the multiplet at 1.92-2.12 ppm which contains the other furan methylene protons.

The chromatography also yielded a mixture of **115** and **116** (0.342 g, 26%).

**(g) Preparation of trans 2-(3',4'-dimethoxyphenyl)-5-(3-aminopropoxy)tetrahydrofuran (117).**

Neat hydrazine hydrate (150 µl, 3.2 mmol) was added to a stirred solution of **115** (253 mg, 0.62 mmol) in absolute ethanol (1.5 ml). The solution was heated at reflux for 5 minutes whereupon a white solid precipitated out of solution. The mixture was heated at reflux for two hours. Analysis by TLC (silica gel; ethyl acetate:hexanes, 1:1) revealed the absence of starting material and the presence of a spot at the origin. The reaction was quenched with water (10 ml) and extracted with dichloromethane (5 X 10 ml). The organic phase was washed with water (2 X 10 ml), brine (2 X 10 ml) and dried (sodium sulfate). Removal of the solvent under reduced pressure left a colorless oil (150 mg, 86%). $^1$H NMR ($CDCl_3$) 1.25 (br s, 2H), 1.68-1.78 (m, 3H), 1.81-1.98 (m, 1H), 2.14-2.2 (m, 1H), 2.3-2.36 (m, 1H), 2.80 (t, J=6.5Hz, 2H), 3.47-3.55 (m, 1H), 3.78-3.87 (m, partially hidden, 1H), 3.86 (s, 3H), 3.88 (s, 3H), 4.99 (t, J=7 Hz, 1H), 5.31 (dd, J=2 and 6 Hz, 1H), 6.80-6.88 (m, 3H).

**(h) Preparation of cis 2-(3',4'-dimethoxyphenyl)-5-(3-aminopropoxy)tetrahydrofuran (118).**

Neat hydrazine hydrate (125 µl, 2.57 mmol) was added to a stirred solution of **116** (210 mg, 0.51 mmol) in absolute ethanol (3.0 ml). The solution was heated at reflux for 5 minutes whereupon a white solid precipitated out of solution. The mixture was heated at reflux for two hours. Analysis by TLC (silica gel; ethyl acetate:hexanes, 1:1) revealed the absence of starting material and the presence of a spot at the origin. The reaction was quenched with water (10 ml) and extracted with dichloromethane (5 X 10 ml). The organic phase was washed with water (2 X 10 ml), brine (1 X 10 ml) and dried (sodium sulfate). Removal of the solvent under reduced pressure left a stiff oil (105 mg, 73%). $^1H$ NMR ($CDCl_3$) 1.45 (br s, 2H), 1.73-1.78 (m, 2H), 2.01-2.12 (m, 3H), 2.19-2.29 (m, 1H), 2.81 (t, J=7 Hz, 2H), 3.48-3.53 (m, 1H), 3.85-3.93 (m, partially hidden, 1H), 3.88 (s, 3H), 3.90 (s, 3H), 4.96-5.01 (m, 1H), 5.17 (dd, J=3 and 6 Hz, 1H), 6.83 (d, J=8 Hz, 1H), 6.89 (dd, J=2 and 8 Hz, 1H) and 6.96 (d, J=2 Hz, 1H).

**(i) Preparation of trans 2-(3',4'-dimethoxyphenyl)-5-[3-(N-butyl-N-hydroxyureidyl)propoxy]tetrahydrofuran (5).**

Triethylamine (32 µl, 0.22 mmol) and then triphosgene (19 mg, 0.06 mmol) were added to a stirred solution of **117** (53 mg, 0.19 mmol) in dry dichloromethane (3 ml) under argon. The solution was heated at reflux for 30 minutes and cooled to room temperature. Solid n-butylhydroxylamine (34 mg, 0.38 mmol) was added in one portion to the solution which was left overnight at room temperature. The reaction was quenched with water (10 ml) and extracted with dichloromethane (3 X 10 ml). The combined organic phase was washed with aqueous sodium bicarbonate solution (saturated, 3 X 10 ml) and dried (sodium sulfate). Analysis by TLC (silica gel, ethyl acetate) revealed a complex mixture Rf 0.90, 0.50, 0.25 and 0.00. The sample was purified by column (flash) chromatography on silica gel 60 (230-400 mesh) and eluted with ethyl acetate to give the spot at Rf 0.50 as an opaque oil (8 mg, 11%). $^1H$ NMR ($CDCl_3$) 0.92 (t, J=7 Hz, 3H), 1.27-1.39 (m, 2H), 1.51-1.61 (m, 2H), 1.71-1.86 (m, 3H), 1.88-2,15 (m, 1H), 2.17-2.29 (m, 1H), 2.32-2.42 (m, 1H), 3.28-3.58 (m, 4H), 3.81-3.94 (m, partially hidden, 2H), 3.87 (s, 3H), 3.90 (s, 3H), 5.49-5.05 (m, 1H), 5.31-5.38 (m, 1H), 6.28-6.34 (m, 1H) and 6.81-6.86 (m, 3H). IR (film) 3407, 3193, 2933, 1640, 1516, 1263., 1029 $cm^{-1}$

**(j) Preparation of trans 2-(3',4'-dimethoxyphenyl)-5-[3-(N-methyl-N-hydroxyureidyl)propoxy]tetrahydrofuran (6).**

Triphosgene (12 mg, 0.04 mmol), followed immediately by triethylamine (17 µl, 0.12 mmol) was added to a stirred solution of **117** (32 mg, .011 mmol) in dry dichloromethane (3 ml) under argon. The solution was heated at reflux for 2 hours, cooled to room temperature and placed in an ice bath. Neat triethylamine (32 µl, 0.23 mmol) followed by methylhydroxylamine hydrochloride salt-(19 mg, 0.23 mmol) was added to the reaction mixture. The reaction was left overnight at room temperature. It was then quenched with water (10 ml) and extracted with dichloromethane (3 X 10 ml). The organic extract was washed with water (3 X 10 ml), brine (3 X 10 ml) and the solvent was removed under reduced pressure to give an amber oil. Analysis by TLC (silica gel, ethyl acetate) revealed only one new spot at Rf 0.30. The sample was purified by column (flash) chromatography on silica gel 60 (230-400 mesh) and eluted with ethyl acetate to give the desired compound as an amber oil (12 mg, 30%). $^1H$ NMR ($CDCl_3$) 1.73-1.84 (m, 2H), 1.90-2.01 (m, 1H), 2.03-2.13 (m, 1H), 2.18-2.29 (m, 1H), 2.32-2.43 (m, 1H), 3.13 (s, 3H), 3.30-3.44 (m, 2H), 3.49-3.59 (m, 1H), 3.82-3.92 (m, partially hidden, 3H), 3.88 (s, 3H), 3.91 (m, 3H), 4.96-5.04 (m, 1H), 5.34 (dd, J=2 and 5 Hz, 1H), 6.34 (br t, 5Hz, 1H) and 6.82-6.68 (m, 3H). IR (film) 3407, 3229, 2935, 1636, 1516, 1263 and 1029 $cm^{-1}$.

**(k) Preparation of cis 2-(3',4'-dimethoxyphenyl)-5-[3-(N-butyl-N-hydroxyureidyl)propoxy]tetrahydrofuran (7).**

Triphosgene (18 mg, 0.06 mmol), followed immediately by triethylamine (80 µl, 0.57 mmol) were added to a stirred solution of **118** (50 mg, 0.18 mmol) in dry dichloromethane (3 ml) under argon. The solution was heated at reflux for 2 hours, cooled to room temperature and placed in an ice bath. Neat triethylamine (50 µl, 0.35 mmol) was added, followed by solid n-butylhydroxylamine (32 mg, 0.36 mmol). The reaction was left overnight at room temperature. It was then quenched with water (10 ml) and extracted with dichloromethane (3 X 10 ml). The organic extract was washed with water (3 X 10 ml), brine (3 X 10 ml), and the solvent was removed under reduced pressure to give an amber oil. Analysis by TLC (silica gel, ethyl acetate) revealed two new spots in approximately equal amounts at Rf 0.85 and 0.45. The sample was purified by column (flash) chromatography on silica gel 60 (230-400 mesh) and eluted with ethyl acetate to give first the spot at Rf 0.85 as an amber oil (26 mg). Continued elution with the same solvent system then gave the title compound as an amber oil (25 mg, 35 %). $^1H$ NMR ($CDCl_3$) 1.1 (t, J=7 Hz, 3H), 1.25-1.37 (m, 2H), 1.49-1.59 (m, 2H), 1.76-1.84 (m, 2H), 1.99-2.1 (m, 3H), 2.19-2.26 (m, 1H), 3.26-3.54 (m, 5H), 3.84-3.92 (m, partially hidden, 1H), 3.87 (s, 3H), 3.88 (s, 3H), 4.94-5.02 (m, 1H), 5.17 (d, J=4 Hz, 1H), 6.24 (t, J=4 Hz, 1H), 6.52 ( br s, 1H), 6.83 (d, J=8 Hz, 1H) and 6.89-95 (m, 2H). IR (film) 2913, 1640, 1570, 1463, 1262, 1139 and 1031 $cm^{-1}$.

**(1) Preparation of cis 2-(3',4'-dimethoxyphenyl)-5-[3-(N-methyl-N-hydroxyureidyl)propoxy]tetrahydrofuran (8).**

Triphosgene (20 mg, 0.07 mmol), followed immediately by triethylamine (80 µl, 0.57 mmol)were added to a stirred solution of **118** (56 mg, 0.2 mmol) in dry dichloromethane (3 ml) under argon. The solution was heated at reflux for 2 hours, cooled to room temperature and placed in an ice bath. Neat triethylamine (80 µl, 0.57 mmol) was added followed by solid methyl hydroxylamine hydrochloride salt (32 mg, 0.39 mmol). The reaction was left overnight at room temperature. It was then quenched with water (10 ml) and extracted with dichloromethane (3 X 10 ml). The organic extract was washed with water (3 X 10 ml), brine (3 X 10 ml), and the solvent was removed under reduced pressure to give an amber oil. Analysis by TLC (silica gel, ethyl acetate) revealed one spot at rf 0.30 and some material at the origin. The sample was purified by column (flash) chromatography on silica gel 60 (230-400 mesh) and eluted with ethyl acetate to give the title compound as an amber oil (30 mg, 42%). $^1$H NMR ($CDCl_3$) 1.76 (m, 2H), 1.98-2.10 (m, 3H), 2.18-2.26 (m, 1H), 3.07 (s, 3H), 3.25-3.37 (m, 2H), 3.46-3.54 (m, 1H), 3.85-3.90 (m, partially hidden, 1H), 3.87 (s, 3H), 3.88 (s, 3H), 4.93-5.00 (m, 1H), 5.16 (d, J=4 Hz, 1H), 6.27 (t, J=5 Hz, 1H), 6.83 (d, J=8 Hz, 1H) and 6.88-6.93 (m, 2H). IR (neat) 2933, 1643, 1518, 1261 and 1029 cm$^{-1}$.

**Example 3 Preparation of 2-(2,4,5-trimethoxyphenyl)-5-(3-hydroxyureidyl propoxy)tetrahydrofuran (13) and 2-(4-fluorophenyl)5-(3-hydroxyureidylpropoxy)tetrahydrofuran (14, 15)**

**(a) Preparation of 2-(3,4,5-trimethoxyphenyl)-5-(3-bromopropoxy) tetrahydrofuran (compound 128)**

Compound 105 (1.0 g, 3.94 mmol) was dissolved in 4 mL dichloromethane. Triethylamine (597 mg, 5.90 mmol) was added to this solution. The reaction mixture was cooled with an ice bath and trifluoroacetic anhydride (1.24 g, 5.90 mmol) was added dropwise. The reaction mixture was stirred at 0°C for 30 minutes and then 3-bromopropanol (1.84 g, 13.27 mmol) was added. The reaction mixture was warmed to room temperature and stirred at room temperature for 2 hours. The reaction was quenched with saturated aqueous $NaHCO_3$ solution and extracted with ethyl acetate. The organic layer was washed with water and saturated NaCl solution, dried over $MgSO_4$, filtered and evaporated in vacuo to an oil which was purified by column chromatography (silica, 4:1 hexane/ethyl acetate) (**128**: 430 mg and its cis isomer 250 mg; total yield 46%). $^1$H NMR ($CDCl_3$): **128** (trans): 1.77(m,1H); 1.98(m,1H); 2.15(m,2H); 2.20(m, 1H); 2.40(m,1H); 3.53(t,2H); 3.60(m,1H); 3.83(s,3H); 3.87(m,1H); 3.89(s,6H); 5.01(t,1H); 5.35(dd,1H); 6.57(s,2H).

**(b) Preparation of 2-(4-fluorophenyl)-5-(3-bromopropoxy) tetrahydrofuran (compounds 129, 130)**

These compounds were prepared from **123** using a procedure similar to that set forth in Example 3(a), replacing compound **105** with compound **123**. $^1$H NMR ($CDCl_3$): **129** (trans): 1.72(m,1H) 1.98(m,1H) 2.14(m,2H); 2.20 (m,1H); 2.40(m,1H); 3.53(t,2H); 3.60(m,1H); 3.89(m,1H); 5.06(t,1H); 5.34(m,1H); 7.02(t,2H); 7.30(m,2H). **13**0 (cis): 1.98(m,1H); 2.07(m,2H); 2.14(m,2H); 2.26(m,1H); 3.52(t,2H); 3.58(m,1H); 3.93(m,1H); 5.00(m,1H) 5.20(dd,1H); 7.03 (t,2H); 7.35(m,2H).

**(c) Preparation of 2-(3,4,5-trimethoxyphenyl)-5-(3-O-benzylhydroxylaminopropoxy) tetrahydrofuran (compounds 131)**

Compound **128** (260 mg, 0.69 mmol) was dissolved in 2 mL 1,3-dimethyl-3,4,5,6-tetrahydro-2-(1H)-pyrimidinone (DMPU). Sodium carbonate (220.4 mg, 2.08 mmol) and benzylhydroxylamine hydrochloride (166 mg, 1.04 mmol) were added to this solution. The reaction was stirred at 80°C for 16 hours, quenched with water and extracted with ethyl acetate. The organic layer was washed with water and saturated sodium chloride solution, dried over $MgSO_4$, filtered and evaporated to an oil which was purified by column (flash) chromatography using ethyl acetate as a solvent (114 mg, 40%). $^1$H NMR ($CDCl_3$): 1.72(m,1H); 1.82(m,2H); 1.92(m,1H); 2.18(m,1H); 2.36(m,1H); 3.06(t,2H); 3.52(m, 1H); 3.81(m,1H); 3.83(s,3H); 3.87(s,6H); 4.71(s,2H); 4.98(t,1H); 5.30(dd,1H); 6.55(s,2H); 7.35(m,5H).

**(d) Preparation of 2-(4-fluorophenyl)-5-(3-O-benzylhydroxylaminopropoxy) tetrahydrofuran (compounds 132,133)**

These compounds were prepared from compounds **129** and **130** using a procedure similar to that set forth in Example 3(c), replacing compound **128** with compounds **129** and **130**. $^1$H NMR ($CDCl_3$): **132** (trans): 1.70(m,1H); 1.83(m,2H); 1.94(m,1H); 2.17(m,1H); 2.38(m,1H); 3.07(t,2H); 3.52(m,1H); 3.82(m,2H); 4.71(s,2H); 5.02(t,1H); 5.30 (ss,1H); 7.02(t,2H); 7.30(m,2H); 7.36(m,5H). **133** (cis): 1.85(m,2H); 1.96(m,1H); 2.05(m,2H); 2.26(m,1H); 3.05(t,2H); 3.50(m,1H); 3.88(m,2H); 4.70(s,2H); 4.99(m,1H); 5.17(dd,1H); 5.50(bs,1H); 7.00(t,2H); 7.35(m,7H)

**(e) Preparation of 2-(3,4,5-trimethoxyphenyl)-5-(3-O-benzylhydroxyureidylpropoxy) tetrahydrofuran (compounds 134)**

Compound **131** (114 mg, 0.27 mmol) was dissolved in 3 mL dichloromethane. Trimethylsilyl isocyanate (47.6 mg, 0.41 mmol) was added to this solution. The reaction was stirred at room temperature for 16 hours and then refluxed for 4 hours.. The reaction was quenched with saturated ammonium chloride solution, extracted with ethyl acetate and evaporated to an oil. The product was isolated by preparative TLC using ethyl acetate as solvent. $^1$H NMR ($CDCl_3$): 1.72(m,1H); 1.94(m,3H); 2.16(m,1H); 2.38(m,1H); 3.50(m,1H); 3.62(m,2H); 3.80(m,1H); 3.82(s,3H); 3.84(s,6H); 4.81 (s,2H); 4.99(t,1H) 5.30(m,3H); 6.54(s,2H); 7.37(s,5H).

**(f) Preparation of 2-(4-fluorophenyl)-5-(3-O-benzylhydroxyureidylpropoxy) tetrahydrofuran (compounds 135, 136)**

These compounds were prepared from **132** and **133** using a procedure similar to that set forth in Example 3 (e), replacing compounds **131** with compounds **132** and **133**. $^1$H NMR ($CDCl_3$): **135** (trans): 1.70(m,1H); 1.93(m,3H); 2.16(m,1H); 2.39(m,1H); 3.50(m,1H); 3.62(m,2H); 3.80(m,1H); 4.82(s,2H); 5.04(t,1H); 5.30(dd,1H 5.35(bs,2H); 7.00 (t,2H); 7.29(m,2H); 7.38(s,5H). **136** (cis): 1.98(m,4H); 2.08(m,1H); 2.25(m,1H); 3.48(m,1H); 3.62(m,2H); 3.83(m,1H); 4.81(s,2H); 4.98(m,1H); 5.17(dd,1H); 5.42(bs,1H); 7.00(t,2H); 7.33(m,2H); 7.38(s,5H).

**(g) Preparation of 2-(3,4,5-trimethoxyphenyl)-5-(3-hydroxyureidylpropoxy) tetrahydrofuran (compounds 13)**

Compound **134** (90 mg, 0.19 mmol) was dissolved in 2 mL ethyl acetate and then Pd/C (10%) (18 mg) was added. The reaction mixture was hydrogenated at balloon pressure for 16 hours. The reaction was filtered and the filtrate was concentrated. The product was isolated by preparative TLC using ethyl acetate as solvent (68 mg). $^1$H NMR ($CDCl_3$): 1.75(m,1H); 1.91(m,2H); 1.95(m,1H); 2.20(m,1H); 2.37(m,1H); 3.58(m,1H); 3.66(m,2H); 3.81(s,3H); 3.85(m, 1H); 3.87(s,6H); 5.00(t,1H); 5.35(dd,1H); 5.41(bs,2H); 6.53(s,2H); 8.39(s,1H).

**(h) Preparation of 2-(4-fluorophenyl)-5-(3-hydroxyureidylpropoxy) tetrahydrofuran (compounds 14, 15)**

Compounds **14** and **15** were prepared from **135** and **136** using a procedure similar to that set forth in Example 3(g), replacing compound **134** with compounds **135** and **136.** $^1$H NMR ($CDCl_3$): **14** (trans): 1.72(m,1H); 1.93(m,3H); 2.20(m,1H) 2.38(m,1H); 3.58(m,1H); 3.67(m,2H); 3.85(m,1H); 5.05(t,1H); 5.33(dd,1H); 5.48(bs,2H); 7.00(t,2H); 7.28 (m,2H); 8.48(bs,1H). **15** (cis): 1.92(m,2H); 2.01(m,1H); 2.10(m,2H); 2.26(m,1H); 3.53(m,1H); 3.64(m,2H); 3.87(m,1H); 4.98(m,1H); 5.20(dd,1H); 5.43(bs,2H); 7.01(m,2H); 7.31(m,2H); 8.43(bs,1H).

**Example 4 Preparation of Trans-2-{3-(N-hydroxyureidyl)-but-1-ynyl}-5-(4-fluorophenyl)tetrahydrofuran (207)**

A synthetic scheme for the production of compound 207 is illustrated in Scheme 8.

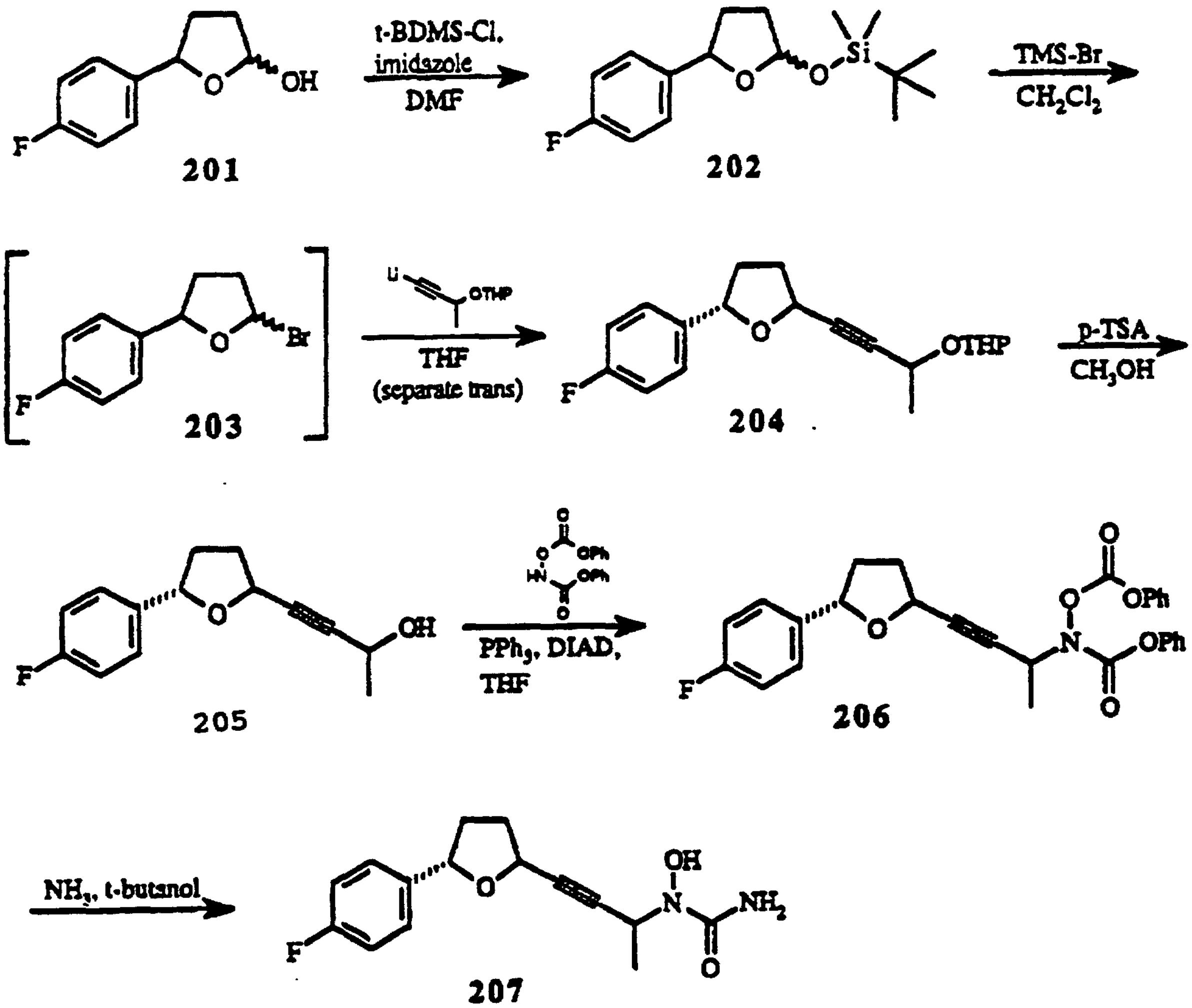


Scheme 8

**(a) Preparation of 2-(t-Butyldimethylsilyloxy)-5-(4-fluorophenyl) tetrahydrofuran (compound 202):**

2-Hydroxy-5-(4-fluorophenyl)-tetrahydrofuran (550 mg, 3.0 mmol), t-butyldimethylsilyl chloride (498 mg, 3.3 mmol) and imidazole (450 mg, 6.6 mmol) were dissolved in 2 mL of dry DMF. This solution was stirred under dry argon overnight, poured into 200 mL of water and extracted with a 2:1 mixture of ethyl acetate and hexane (3 X 100 mL).

The combined organic extracts were washed with water (4 X 200 mL) and brine (100 mL), dried over sodium sulfate and evaporated to give 830 mg (93 %) of 2-(t-butyldimethylsilyloxy)-5-(4-fluorophenyl) tetrahydrofuran (**202**, mixture of cis and trans isomers) as a colorless oil, which did not need any purification. $^1$H-NMR ($CDCl_3$) δ 7.40-7.50(2H, m, minor isomer), 7.25-7.35 (2H, m, major isomer), 7.00-7.10 (2H, m, both major and minor isomers), 5.71-5.75 (1H, m, major isomer), 5.59-5.62 (1H, m, minor isomer), 5.12-5.20 (1H, m, major isomer), 4.90-4.98 (1H, m, minor isomer), 2.40-2.55 (1H, m, both major and minor isomers), 2.05-2.17 (1H, m, both major and minor isomers), 1.87-2.00 (1H, m, both major and minor isomers), 1.67-1.70 (1H, m, both major and minor isomers), 0.92 (s, 9H, both major and minor isomers), 0.16 (s, 6H, both major and minor isomers).

**(b) Preparation of Trans-2-(3-Tetrahydropyranyloxy-but-1-ynyl)-5-(4-fluorophenyl) tetrahydrofuran (compound 204):**

2-(t-Butyldimethylsilyloxy)-5-(4-fluorophenyl) tetrahydrofuran (**202**, 593 mg, 2.0 mmol) was mixed in 10 mL of dry methylene chloride (degassed by bubbling argon prior to use). This solution was cooled to -70°C. While stirring at the same temperature under dry argon, trimethylsilyl bromide (290 μL, 2.2 mmol) was added dropwise. The stirring was continued for an additional 1.5 h to produce 2-bromo-5-(4-fluorophenyl) tetrahydrofuran (**203**) which was not isolated and was used in subsequent chemistry without further purification (see below).

In a separate flask, 3-tetrahydropyranyloxy-but-1-yne (370 mg, 2.4 mmol) was dissolved in dry THF (5 mL). The solution was cooled to -60°C and, while stirring at the same temperature under dry argon, n-butyllithium (1.0 mL, 2.4 mmol) was added dropwise. The stirring was continued for an additional 0.5 hours. The resulting solution was syringed out and added dropwise to the stirred solution of the 2-bromotetrahydrofuran (made above) at -70°C. The stirring was continued at -78°C for an additional 1.5 hours. The reaction flask was stored in the freezer (-78°C) over night (though the TLC did not show any change). The reaction mixture was poured into a 2M solution of ammonium chloride (50 mL) and extracted with methylene chloride (3 X 50 mL). The solution was dried over sodium sulfate and the solvent was removed *in vacuo*. The residue was purified via flash column chromatography (eluent, 10% ethyl acetate in hexane) to obtain two components. From the proton NMR analysis, the less polar component was identified as trans-2-(3-tetrahydropyranyloxy-but-1-ynyl)-5-(4-fluorophenyl) tetrahydrofuran (**204**, 280 mg, 45%) and the more polar component (230 mg) was found to be a mixture of more than one compound. This mixture was discarded. $^1$H-NMR ($CDCl_3$) δ 7.27-7.30 (2H, m,), 7.01 (2H, t, J = 8.7 Hz), 5.09 (1H, t, J =7.1 Hz), 4.91-4.95 (2H, m), 4.57-4.64 (1H, m), 3.78-3.90 (1H, m), 3.50-3.60 (1H, m),2.30-2.50 (2H, m), 2.05-2.17 (1H, m), 1.70-1.90 (3H, m), 1.50-1.65 (4H, m), 1.48 (3H, d, J = 6.6 Hz).

**(c) Preparation of trans-2-(3-Hydroxy-but-1-ynyl)-5-(4-fluorophenyl) tetrahydrofuran (compound 205):**

trans-2-(3-Tetrahydropyranyloxy-but-1-ynyl)-5-(4-fluorophenyl)tetrahydrofuran (**204**, 280 mg, 0.9 mmol) was dissolved in methanol (15 mL). To this solution was added p-toluenesulfonic acid (50 mg) and the resulting solution was stirred for 45 minutes. Saturated sodium bicarbonate solution (10 mL) was added. After 5 minutes of stirring, the solution was added to 10 mL of water, diluted with 15 mL of brine and extracted with methylene chloride (3 X 30 mL). The combined organics were dried over sodium sulfate and the solvent was removed via rotary evaporator to yield 212 mg (100%) of trans-2-(3-hydroxy-but-1-ynyl)-5-(4-fluorophenyl) tetrahydrofuran (**205**). $^1$H-NMR ($CDCl_3$) δ 7.29 (2H, dd, J = 8.7, 5.2 Hz), 7.01 (2H, t, J = 8.7 Hz), 5.09 (1H, t, J = 7.4 Hz), 4.92 (1H, t, J = 7.4 Hz), 4.59 (1H, q, J = 6.6 Hz), 2.30-2.50 (2H, m), 2.05-2.15 (1H, m), 2.00 (1H, br s), 1.75-1.88 (1H, m), 1.47 (3H, d, J = 6.6 Hz).

**(d) Preparation of trans-2-{3-(N-Phenoxycarbonyloxy-N-phenoxycarbonylamino)-but-l-ynyl}-5-(4-fluorophenyl) tetrahydrofuran (compound 206):**

trans-2-(3-Hydroxy-but-1-ynyl)-5-(4-fluorophenyl) tetrahydrofuran (**205**, 210 mg, 0.89 mmol), triphenylphosphine (288 Eg, 1.1 mmol) and N,O-bis(phenoxycarbonyl)hydroxylamine (283 mg, 1.1 mmol) were dissolved in dry THF (5 mL). The solution was cooled to 0°C under dry argon, and diisopropylazodicarboxylate (216 mL, 1.1 μmol) was added dropwise. Stirring was continued for 1 hour at the same temperature. The solvent was evaporated and the residue was purified via flash column chromatography (eluent, 30% ethyl acetate in hexane) to yield 250 mg (57%) of trans-2-{3-(N-phenoxycarbonyloxy-N-phenoxycarbonyl-amino)-but-1-ynyl}-5-(4-fluorophenyl) tetrahydrofuran (**206**). $^1$H-NMR ($CDCl_3$) δ 7.15-7.45 (12H, m), 7.02 (2H, t, *J* = 8.6 Hz), 5.32 (1H, q *J* = 7.0 Hz), 5.07 (1H, t, *J* = 6.8 Hz), 4.96 (1H, t, J = 5.7 Hz), 2.25-2.50 (2H, m), 2.05-2.20 (1H, m), 1.70-1.85 (1H, m), 1.66 (3H, d, J = 7.0 Hz).

**(e) Preparation of trans-2-{3-(N-Hydroxyureidyl)-but-1-ynyl}-5-(4-fluorophenyl)-tetrahydrofuran (compound 207)**

Trans-2-{3-(N-phenoxycabonyloxy-N-phenoxycarbonyl-amino)-but-1-ynyl}-5-(4-fluorophenyl) tetrahydrofuran (**206**, 200 mg, 0.41 mmol) was dissolved in a high pressure tube as a solution in methylene chloride. The solvent was evaporated with a stream of argon and the residue was cooled to -78°C. Ammonia (8 mL) was condensed in this tube and 4 mL of t-butanol was added. The tube was sealed, allowed to slowly warm to the room temperature, and stirred at room temperature for 18 hours. The pressure was released very slowly and the tube was left open for 1 hour. The residue was transferred into a flask and rotavapped twice with added toluene. The residue was purified via flash column chromatography (eluent, 3% methanol in ethyl acetate) and was further purified on a preparative TLC (solvent, 5% methanol in methylene chloride) to give 93 mg (78%) of Trans-2-{3-(N-hydroxyureidyl)-but-1-ynyl}-5-(4-fluorophenyl) tetrahydrofuran (**207**). IR (film) 3481, 3269, 2985, 2877, 2249, 1662, 1670, 1510, 1444, 1224, 1172, 1037 $cm^{-1}$; $^1$H-NMR ($CDCl_3$) δ 8.10 (1H, br s), 7.26 (2H, dd, J = 8.6, 5.4 Hz), 7.00 (2H, t, J = 8.6 Hz), 5.80 (1H, br s), 5.00-5.20 (2H, m), 4.80-5.00 (1H. m), 2.20-2.50 (2H, m), 2.00-2.20 (1H, m), 1.70-1.90 (1H, m), 1.37 (3H, dd, J = 6.9, 1.9 Hz).

**Example 5 Preparation of S,S,S- and S,S,R-isomers of trans-2-{3-(N-Hydroxyureidyl)-but-1-ynyl}-5-(4-fluorophenyl)-tetrahydrofuran (compounds 216 and 217)**

One method for the preparation of the S,S,R- and S,S,S- isomers of trans-2-{3-(N-Hydroxyureidyl)-but-1-ynyl}-5-(4-fluorophenyl)-tetrahydrofuran is illustrated below in Scheme 9.

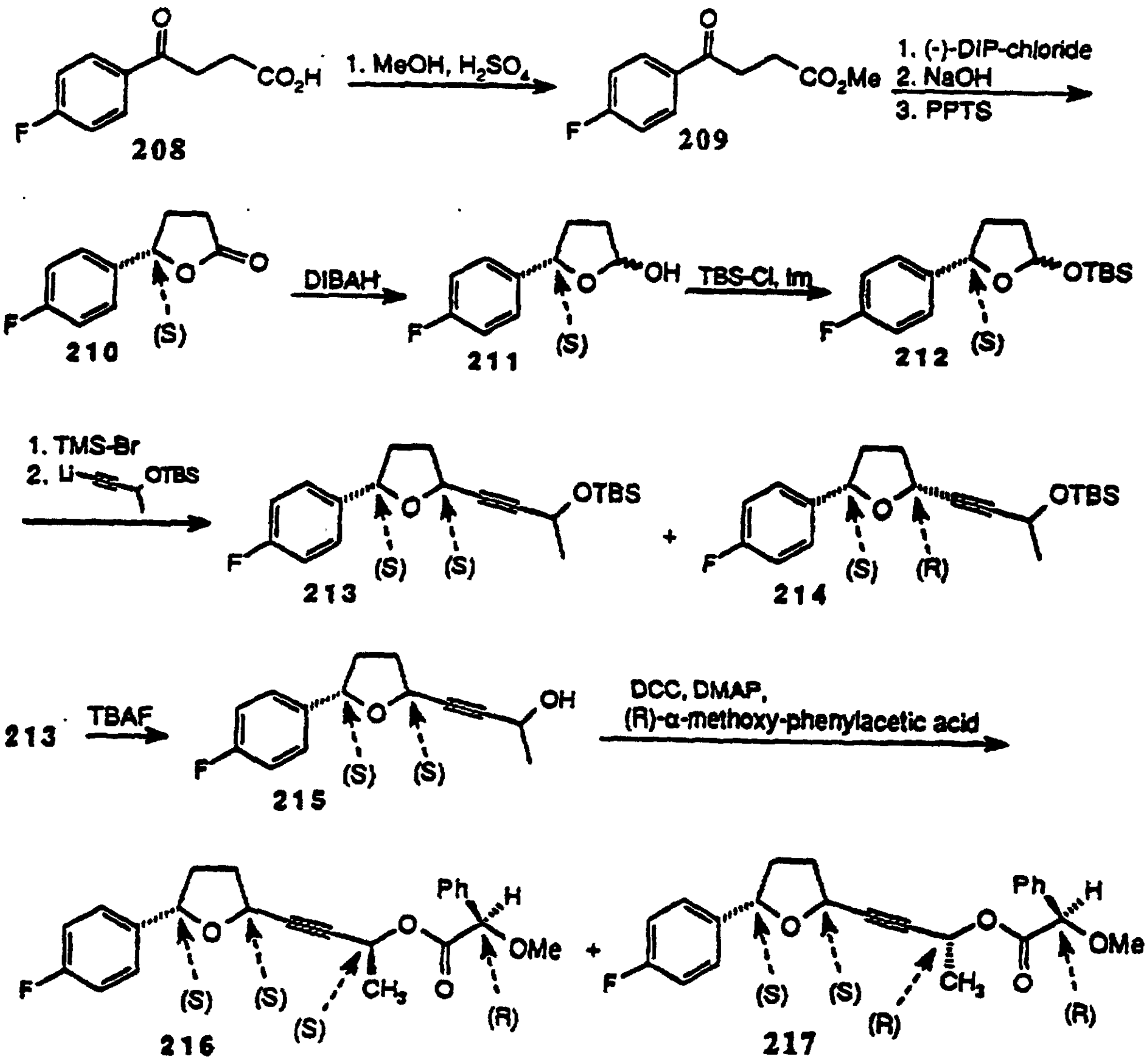


208 209 210 211 212 213 214 215 216 217

Scheme 9

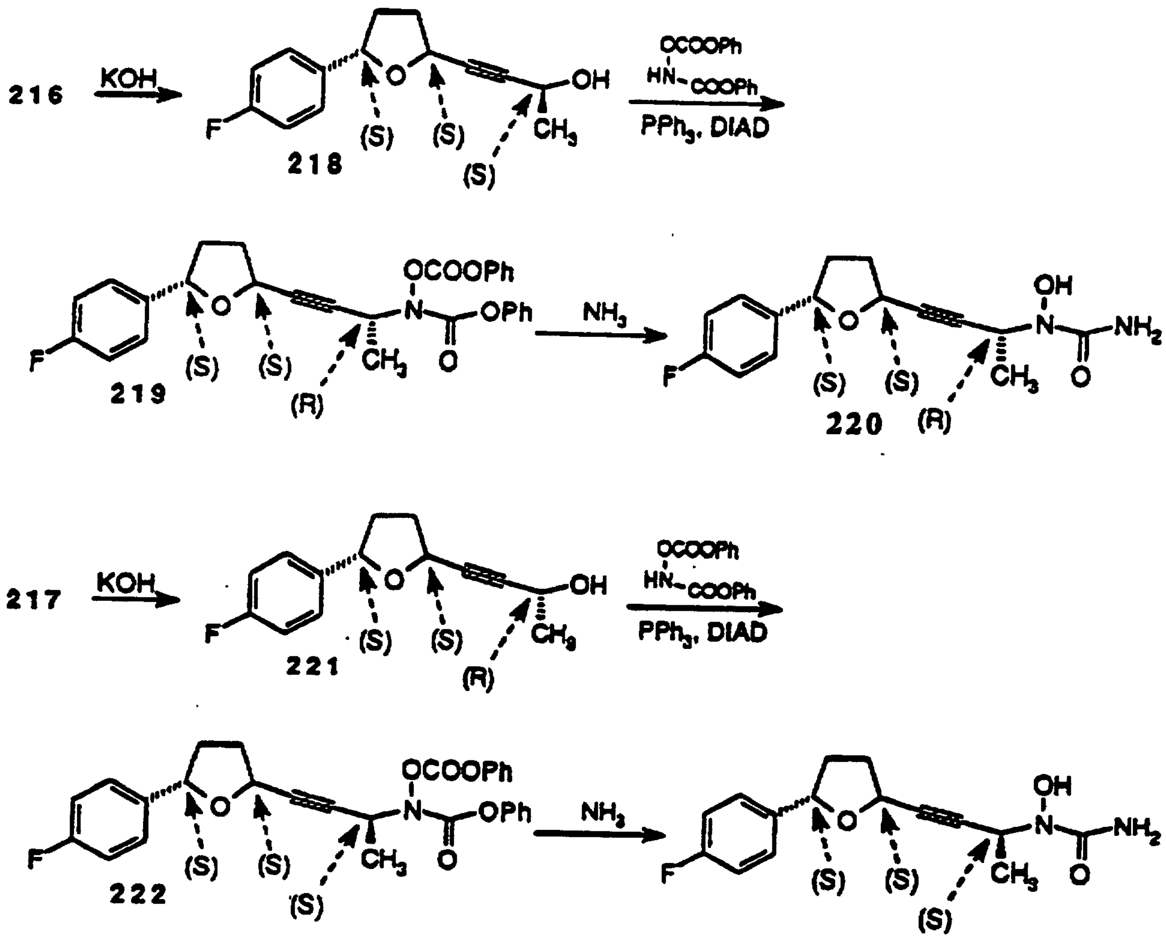


216
218
219
220
217
221
222

Scheme 9 (cont'd)

**(a) Preparation of Methyl 3-(4-fluorobenzoyl)-propionate (compound 209)**

To a solution of 3-(4-fluorobenzoyl)-propionic acid (1.98 g, 10.0 mmol) in methanol (25 mL) was added 0.5 mL of conc. sulfuric acid. The resulting solution was stirred at room temperature under argon for 2 hours. The reaction mixture was neutralized with saturated sodium bicarbonate, the methanol was removed via rotary evaporator and the

residue was dissolved in 50 mL of ethyl acetate. The resulting solution was washed with saturated sodium bicarbonate (3 X 50 mL) and brine (50 mL), dried over sodium sulfate and the solvent was removed in vacuo to give Methyl 3-(4-fluorobenzoyl)-propionate (2 g, 94%). IR (film) 3448, 3111, 3076, 3003, 3958, 1734, 1678, 1601, 1423, 1300, 1240, 1155, 1099 $cm^{-1}$; $^1$H-NMR ($CDCl_3$) δ 7.97 (2H, dd, *J* = 9.0, 5.5 Hz), 7.10 (2H, t, *J* = 8.9 Hz), 3.67 (3H, s), 3.25 (2H, t, *J* = 6.6 Hz), 2.73 (2H, t, *J* = 6.6 Hz); $^{13}$C-NMR ($CDCl_3$) δ 196.50, 173.34, 167.54, 164.17, 132.98, 130.77, 115.91, 115.62, 51.91, 33.31, 28.00.

**(b) Preparation of (S)-5-(4-fluorophenyl)-γ-butyrolactone (compound 210)**

A solution of methyl 3-(4-fluorobenzoyl)-propionate (**209**, 780 mg,, 3.67 mmol) in dry THF (2 mL) was added, dropwise, to a precooled (0°C) solution of (-)-DIP-chloride (2.02 g, 6.25 mmol) in THF (2 mL) with stirring under dry argon. The resulting solution was stirred at the same temperature for 2 hours and allowed to stand at 0-5°C overnight. Maintaining the temperature at 0°C, with stirring, water (2 mL) was added dropwise, followed by methanol (5 mL) and a 5 M NaOH solution (5 mL). The reaction mixture was stirred at room temperature for 1.5 hours, cooled, and 15 mL of saturated bicarbonate solution was added. The resulting mixture was washed with ether (3 X 50 mL) and acidified with 6 N HCl. The acidic mixture was extracted with toluene (3 X 50 mL). The combined toluene extracts were washed with brine (50 mL), dried over sodium sulfate and the solvent was removed in vacuo. The residue was resuspended in 50 mL of toluene and PPTS (10 mg) was added to it. The resulting solution was refluxed under a Dean-Stark trap (first 15 mL of the distillate were drained off) for 2 hours. The solution was cooled, washed with saturated bicarbonate solution (2 X 50 mL), dried over sodium sulfate and the solvent was removed in vacuo to yield 620 mg (94%) of (S)-5-(4-fluorophenyl)-γ-butyrolactone. $^1$H-NMR ($CDCl_3$) δ 7.33 (2H, dd, *J* = 8.8, 5.3 Hz), 7.09 (2H, t, *J* = 8.7 Hz), 5.50 (1H, dd, *J* = 8.4, 5.9 Hz), 2.64-2.71 (3H, m), 2.17-2.22 (1H, m).

**(c) Preparation of (5S)-2-Hydroxy-5-(4-fluorophenyl) tetrahydrofuran (compound 211)**

(S)-5-(4-Fluorophenyl)-γ-butyrolactone (**210**, 620 mg, 3.44 mmol) was dried azeotropically (with hexane) and dissolved in dry methylene chloride (25 mL). The solution was cooled to -78°C and, with stirring under argon, DIBALH (3.5 mL of 1.5 M solution in toluene, 5.16 mmol) was added dropwise. Stirring was continued at -78°C for 2 hours and then a saturated solution of Na-K-tartrate (25 mL) was added. The cooling bath was removed and the stirring was continued for additional 2 hours. The reaction mixture was diluted with methylene chloride (25 mL). The organic layer was separated, washed with water (2 X 50 mL) and brine (50 mL), dried over sodium sulfate and the solvent was removed in vacuo to yield 2-hydroxy-5-(4-fluorophenyl) tetrahydrofuran (620 mg, 100%). $^1$H-NMR ($CDCl_3$) δ 7.30-7.41 (m, 2H), 7.04 (m, 2H), 5.63-5.78 (m, 1H), 5.00-5.22 (m, 1H), 2.48 (m, 1H), 2.20-2.32 (m,1H), 1.95-2.10 (m, 1H), 1.79 (m, 1H).

**(d) Preparation of (5S)-2-(t-Butyldimethylsilyloxy)-5-(4-fluorophenyl) tetrahydrofuran (compound 212):**

(5S)-2-Hydroxy-5-(4-fluorophenyl) tetrahydrofuran (**211**, 620 mg, 3.5 mmol), t-butyldimethylsilyl chloride (700 mg, 5.25 mmol) and imidazole (595 mg, 8.75 mmol) were dissolved in 2 mL of dry DMF. The resulting solution was stirred under dry argon overnight, poured into 200 mL of water, and extracted with a 2:1 mixture of ethyl acetate and hexane (3 X 100 mL). The combined organic extracts were washed with water (4 X 200 mL) and brine (100 mL), dried over sodium sulfate and the solvent was removed in vacuo to yield 1 g (96%) of (5S)-2-(t-Butyldimethylsilyloxy)-5-(4-fluorophenyl) tetrahydrofuran (**212**, mixture of cis and trans isomers) as a colorless oil, which did not need further purification. $^1$H-NMR ($CDCl_3$) 6 7.40-7.50(2H, m, minor isomer), 7.25-7.35 (2H, m, major isomer), 7.00-7.10 (2H, m, both major and minor isomers), 5.71-5.75 (1H, m, major isomer), 5.59-5.62 (1H, m, minor isomer), 5.12-5.20 (1H, m, major isomer), 4.90-4.98 (1H, m, minor isomer), 2.40-2.55 (1H, m, both major and minor isomers), 2.05-2.17 (1H, m, both major and minor isomers), 1.87-2.00 (1H, m, both major and minor isomers), 1.67-1.70 (1H, m, both major and minor isomers), 0.92 (s, 9H, both major and minor isomers), 0.16 (s, 6H, both major and minor isomers).

**(e) Preparation of (2S,5S)-trans-2-(3-t-butyldimethylsilyloxy-but-1-ynyl)-5-(4-fluorophenyl) tetrahydrofuran (compound 213):**

(5S)-2-(t-Butyldimethylsilyloxy)-5-(4-fluorophenyl) tetrahydrofuran (**212**, 1 g, 3.4 mmol) was dissolved in 10 mL of dry methylene chloride (degassed by bubbling argon prior to use). This solution was cooled to -70°C and, while stirring at the same temperature under dry argon, trimethylsilyl bromide (550 μL, 4.1 mmmol) was added dropwise. The stirring was continued for an additional 1.5 hours to yield (5S)-2-bromo-5-(4-fluorophenyl) tetrahydrofuran which was used without isolation (see below). In a separate flask, 3-t-butyldimethylsilyloxy-but-1-yne (840 mg, 4.5 mmol) was dissolved in dry THF (10 mL). The solution was cooled to -60°C and, while stirring at the same temperature under

dry argon, n-butyllithium (1.8 mL of 2.5M solution in hexane, 4.5 mmmol) was added dropwise. The stirring was continued for an additional 0.5 hours. The resulting solution was added dropwise, through a cannula to the stirred solution of the 2-bromotetrahydrofuran (made above) at -70°C. The stirring was continued at -78°C for additional 1.5 hours. The reaction flask was then left in the freezer (-78°C) over night (though the TLC did not show any change). The reaction mixture was poured into 2M solution of ammonium chloride (100 mL) and extracted with methylene chloride (3 X 75 mL). The solution was dried over sodium sulfate and the solvent removed in vacuo. The residue was purified via flash column chromatography (eluent, 10% ethyl acetate in hexane) to obtain two components. From the proton NMR analysis, the less polar one was identified as (2S,5S)-trans-2-(3-t-butyldimethylsilyloxy-but-1-ynyl)-5-(4-fluorophenyl) tetrahydrofuran (213, 765 mg, 65%). $^1$H-NMR ($CDCl_3$) δ 7.29 (2H, m), 7.01 (2H, t, *J* = 8.7 Hz), 5.09 (1H, t, *J* =7.1 Hz), 4.91-4.97 (2H, m), 4.55-4.62 (1H, m), 2.26-2.50 (2H, m), 2.05-2.17 (1H, m), 1.75-1.88 (1H, m), 1.38 (3H, d, *J* = 6.6 Hz), 0.90 (9H,s), 0.12 (6H, s). The more polar component was assigned to be (2R,5S)-cis-2-(3-t-butyldimethylsilyloxy-but-1-ynyl)-5-(4-fluorophenyl) tetrahydrofuran (**214**, 190 mg, 16%).

**(f) Preparation of (2S,5S)-trans-2-(3-hydroxy-but-1-ynyl)-5-(4-fluorophenyl) tetrahydrofuran (compound 215):**

(2S,5S)-trans-2-(3-t-butyldimethylsilyloxy-but-1-ynyl)-5-(4-fluorophenyl) tetrahydrofuran (**213**, 765 mg, 2.2 mmol) was dissolved in 20 mL of THF. The solution was cooled to 0°C and TBAF (6.6 mL of 1M solution in THF) was added to it. The resulting solution was stirred at 0°C for 2h and the solvent was removed in vacuo. The residue was dissolved in ethyl acetate (100 mL), washed with water (3 X 100 mL, added 5 mL of brine each time to separate layers) followed by brine (50 mL), dried over sodium sulfate and the solvent removed in vacuo to yield 500 mg (97%) of (2S, 5S)-trans-2-(3-hydroxy-but-1-ynyl)-5-(4-fluorophenyl) tetrahydrofuran (**215**). $^1$H-NMR ($CDCl_3$) δ 7.29 (2H, dd, *J* = 8.7, 5.2 Hz), 7.01 (2H, t, *J* = 8.7 Hz), 5.09 (1H, t, *J* = 7.4 Hz), 4.92 (1H, t, *J* = 7.4 Hz), 4.59 (1H, q, *J* = 6.6 Hz), 2.30-2.50 (2H, m), 2.05-2.15 (1H, m), 1.75-1.88 (1H, m), 1.72 (1H, br s), 1.47 (3H, d, J = 6.6 Hz).

(2S,5S)-trans-2-(3-Hydroxy-but-1-ynyl)-5-(4-fluorophenyl) tetrahydrofuran (**215**, 500 mg, 2.13 mmol), (R)-α-Methoxy-phenylacetic acid (1.06 g, 6.4 mmol) and DMAP (86 mg, 0.7 mmol) were dissolved in dry methylene chloride (3 mL). DCC (1.5 g, 7.24 mmol) was added and the resulting solution was stirred at room temperature, under dry argon, for 3h (a lot of white solid precipitated within minutes). The solid was filtered off and the filtrate was concentrated in vacuo. The residue was purified via flash column chromatography (eluent, 8% ethyl acetate in hexane) to obtain the two diastereomeric esters. The less polar one was assigned to be from (2S,5S)-trans-2-{3-(S)-hydroxy-but-1-ynyl}-5-(4-fluorophenyl) tetrahydrofuran (**216**, 250 mg, 30%, >95% de from $^1$H-NMR). $^1$H-NMR ($CDCl_3$) δ 7.25-7.50 (7H, m), 7.02 (2H, t, *J* = 8.5 Hz), 5.52-5.60 (1H, m), 5.06 (1H, t, *J* = 6.8 Hz), 4.88-4.94 (1H, m), 4.78 (1H, s), 3.43 (3H, s), 2.25-2.47 (2H, m), 2.00-2.13 (1H, m), 1.75-1.88 (1H, m), 1.37 (3H, d, J = 6.7 Hz). The more polar one was assigned to be from (2S,5S)-trans-2-{3-(R)-hydroxy-but-1-ynyl}-5-(4-fluorophenyl) tetrahydrofuran (**217**, 230 mg, 29%, 72% de from $^1$H-NMR). $^1$H-NMR ($CDCl_3$) δ 7.22-7.50 (7H, m), 7.01 (2H, t, *J* = 8.7 Hz), 5.50-5.60 (1H, m), 4.98 (1H, t, *J* = 7.2 Hz), 4.79-4.85 (1H, m), 4.79 (1H, s), 3.44 (3H, s), 2.20-2.40 (2H, m), 1.88-1.98 (1H, m), 1.72-1.80 (1H, m), 1.51 (3H, d, *J* = 6.7 Hz). Basic hydrolyses (stirring in 10 mL of 1M ethanolic KOH at 50°C for 30 min followed by usual workup) of these two esters gave their respective alcohols; (2S,5S)-trans-2-{3-(S)-hydroxy-but-1-ynyl}-5-(4-fluorophenyl) tetrahydrofuran (**218**, 150 mg, 98%) and its diastereomer (2S,5S)-trans-2-{3-(R)-hydroxy-but-1-ynyl}-5-(4-fluorophenyl) tetrahydrofuran (**221**, 50 mg, 100%). The $^1$H-NMR spectra for both these alcohols were identical to that of **218.**

**(g) Preparation of (2S,5S)-trans-2-{3-(R)-(N-phenoxycabonyloxy-N-phenoxycarbonylamino)-but-1-ynyl}-5-(4-fluorophenyl) tetrahydrofuran (compound 219):**

(2S,5S)-trans-2-{3-(S)-hydroxy-but-1-ynyl}-5-(4-fluorophenyl) tetrahydrofuran (**218**, 150 mg, 0.64 mmol), triphenylphosphine (200 mg, 0.77 mmol) and N,O-bis(phenoxycarbonyl)hydroxylamine (200 mg, 0.77 mmol) were dissolved in dry THF (3 mL). The solution was cooled to 0°C and with stirring under dry argon was added diisopropylazodicarboxylate (142 μL, 0.77 mmol) dropwise. The stirring was continued for 1 h at the same temperature. The solvent was evaporated on a rotavap and the residue was purified via flash column chromatography (eluent, 30% ethyl acetate in hexane) to give 225 mg (72%) of (2S,5S)-trans-2-{3-(R)-(N-phenoxycabonyloxy-N-phenoxycarbonyl-amino)-but-1-ynyl}-5-(4-fluorophenyl) tetrahydrofuran (**219**). $^1$H-NMR ($CDCl_3$) δ 7.15-7.45 (12H, m), 7.02 (2H, t, *J* = 8.6 Hz), 5.32 (1H, q *J* = 7.0 Hz), 5.07 (1H, t, *J* = 6.8 Hz), 4.96 (1H, t, *J* = 5.7 Hz), 2.25-2.50 (2H, m), 2.05-2.20 (1H, m), 1.70-1.85 (1H, m), 1.66 (3H, d, J = 7.0 Hz).

**(h) Preparation of (2S,5S)-trans-2-{3-(S)-(N-phenoxycabonyloxy-N-phenoxycarbonylamino)-but-1-ynyl}-5-(4-fluorophenyl) tetrahydrofuran (compound 222):**

Starting with (2S,5S)-trans-2-{3-(R)-hydroxy-but-1-ynyl}-5-(4-fluorophenyl) tetrahydrofuran (**221**, 150 mg, 0.64 mmol), following the same procedure for **218,** 220 mg (70%) of (2S,5S)-trans-2-{3-(S)-(N-phenoxycabonyloxy-N-phenoxycarbonylamino)-but-1-ynyl}-5-(4-fluorophenyl) tetrahydrofuran (**222**) was obtained. The $^1$H-NMR was identical to that of **219**.

**(i) Preparation of (2S,5S)-trans-2-{3-(R)-(N-hydroxyureidyl)-but-1-ynyl}-5-(4-fluorophenyl) tetrahydrofuran (CMI-947) (compound 220):**

(2S,5S)-trans-2-{3-(R)-(N-phenoxycabonyloxy-N-phenoxycarbonyl-amino)-but-1-ynyl}-5-(4-fluorophenyl) tetrahydrofuran (**219**, 225 mg) was dissolved in a high pressure tube as a solution in methylene chloride. The solvent was evaporated with a stream of argon and the residue was cooled to -78°C. 10 mL of ammonia was condensed in this tube and 2 mL of t-butanol was added. The tube was sealed and was allowed to slowly warm to the room temperature. Then it was left stirring at rt for 18 hours. The pressure was released very slowly and the tube was left open for 1 hour. The residue was transferred into a flask and concentrated under vacuum twice with added toluene. The residue was purified via preparative TLC (eluent, 5% methanol in methylene chloride) to give 120 mg (90%) of (2S,5S)-trans-2-{3-(R)-(N-hydroxyureidyl)-but-1-ynyl}-5-(4-fluorophenyl) tetrahydrofuran (**CMI-947**, **220**)). IR (film) 3209, 2985, 2874, 1653, 1510, 1449, 1336, 1224, 1157, 1037 $cm^{-1}$; $^1$H-NMR ($CD_3OD$) δ 7.34 (2H, dd, *J* = 8.7, 5.4 Hz), 7.04 (2H, t, *J* = 8.8 Hz), 5.00- 5.10 (2H, m), 4.85-4.95 (1H, m), 2.25-2.50 (2H, m), 2.00-2.15 (1H, m), 1.78-1.85 (1H, m), 1.38 (3H, d, *J* = 7.0 Hz).

**(j) Preparation of (2S,5S)-trans-2-{3-(S)-(N-hydroxyureidyl)-but-1-ynyl}-5-(4-fluorophenyl) tetrahydrofuran (CMI-948) (compound 223):**

Starting with (2S,5S)-trans-2-{3-(S)-(N-phenoxycabonyloxy-N-phenoxycarbonyl-amino)-but-1-ynyl}-5-(4-fluorophenyl) tetrahydrofuran (**222**, 225 mg), following the same procedure for **219**, 110 mg (83%) of (2S,5S)-trans-2-{3-(S)-(N-hydroxyureidyl)-but-1-ynyl}-5-(4-fluorophenyl) tetrahydrofuran (**CMI-948, 223**) was obtained. IR (film) 3200, 2985, 2881, 1643, 1510, 1442, 1222, 1035 $cm^{-1}$; $^1$H-NMR ($CD_3OD$) δ 7.34 (2H, dd, *J* = 8.7, 5.5 Hz), 7.04 (2H, t, *J* = 8.9 Hz), 5.00- 5.10 (2H, m), 4.85-4.95 (1H, m), 2.25-2.50 (2H, m), 2.00-2.15 (1H, m), 1.70-1.85 (1H, m), 1.38 (3H, d, *J* = 7.0 Hz).

**Example 6 Preparation of R,R,S- and R,R,R-isomers of trans-2-{3-(N-Hydroxyureidyl)-but-1-ynyl}-5-(4-fluorophenyl)-tetrahydrofuran (compounds 234 and 236)**

One method for the preparation of the S,S,R- and S,S,S- isomers of trans-2-{3-(N-Hydroxyureidyl)-but-1-ynyl}-5-(4-fluorophenyl)-tetrahydrofuran is illustrated below in Scheme 10.

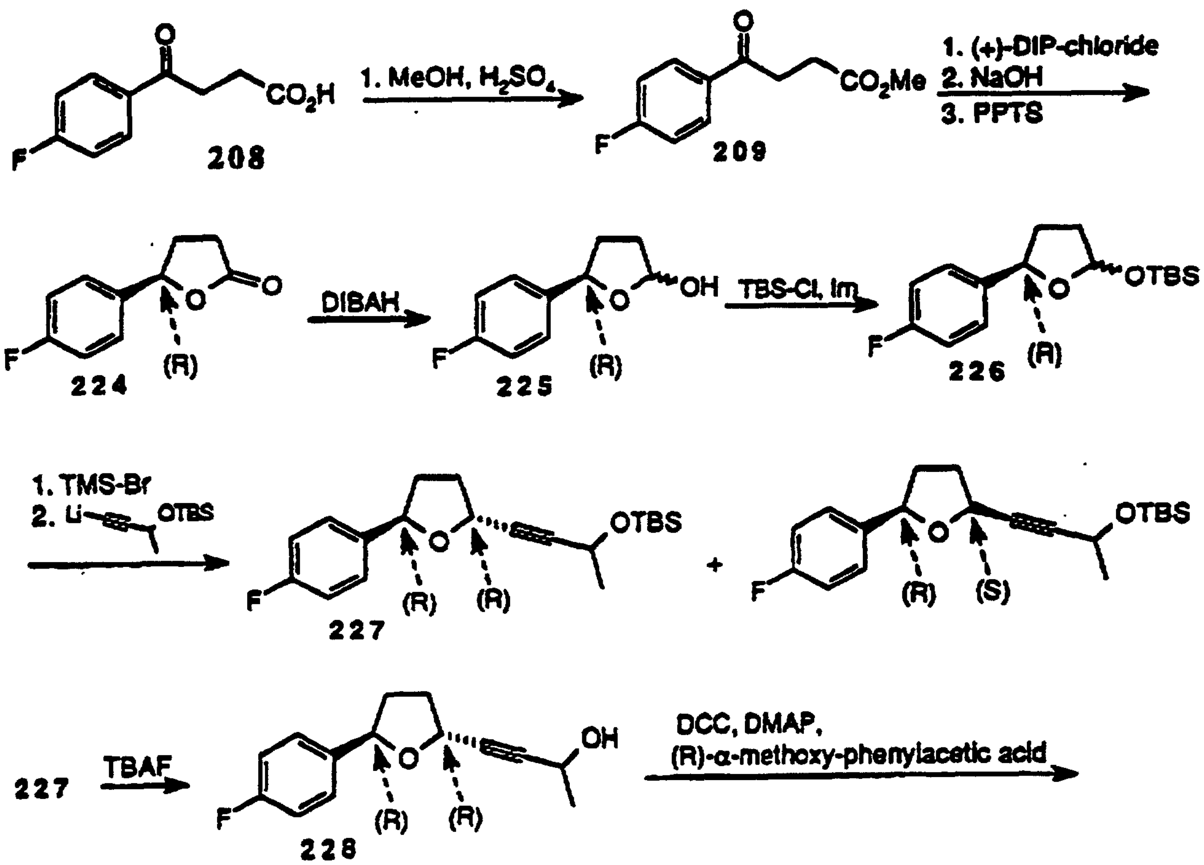


208 209 224 225 226 227 227 228

Scheme 10

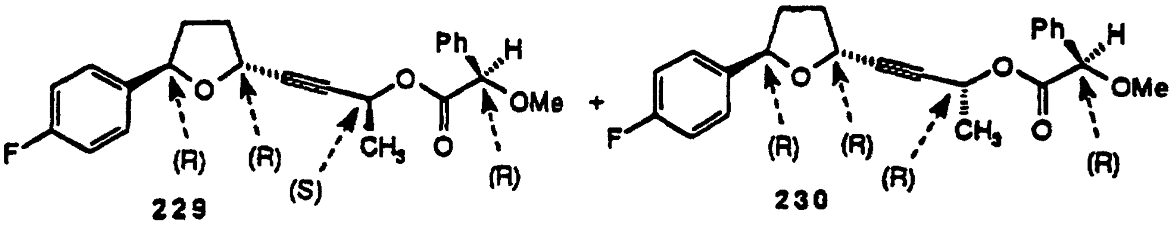

229 + 230

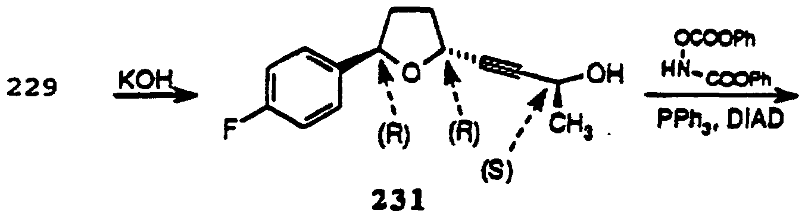


231

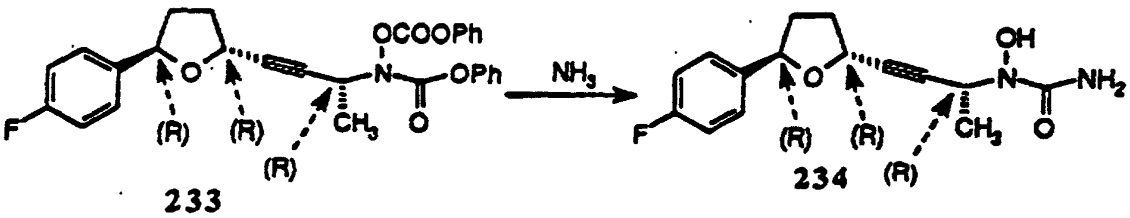


233 → 234

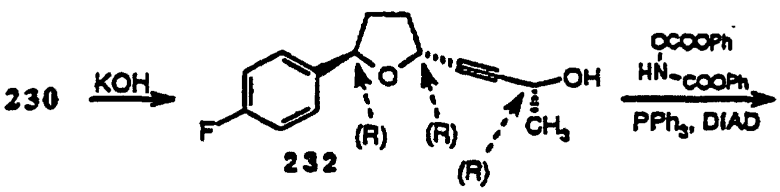


232

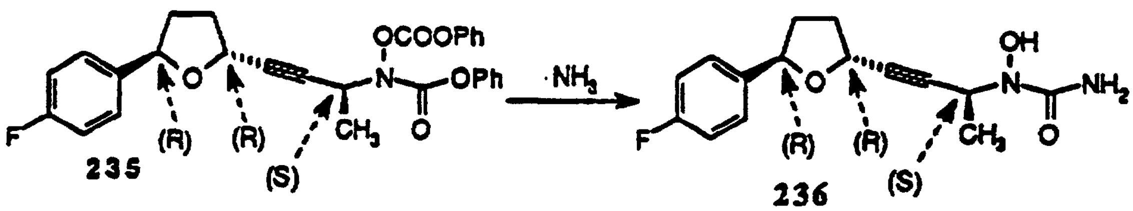


235 → 236

Scheme 10 (cont'd.)

**(a) Preparation of 4-(4-fluorophenyl)-4-oxomethylbutanoate (compound 209)**

To a stirred solution of 3-(4-benzoyl)propionic acid (**208**) (5.0g) in methanol (20mL) was added a few drops of sulfuric acid. After stirring overnight (19 hrs) the reaction was neutralized with saturated aqueous sodium bicarbonate and the methanol was removed under reduced pressure. The residue was dissolved in ethyl acetate (50mL) and washed with saturated aq. sodium bicarbonate (3x15mL), water (2x15mL), and brine (2x15mL), dried ($Na_2SO_4$), filtered and concentrated to give a pale crystalline solid (5.3g, 98%). $^1$H NMR: 2.79(t, 2H), 3.30(T, 2H), 3.71(S, 3H), 7.14(T, 2H), 8.02(m,2H).

**(b) Preparation of R-4-(4-fluorophenyl)-gamma-butyrolactone (compound 224)**

To a cooled (0°C), stirred solution of (+)-DIP chloride (25g, 77.9 mmol) in dry THF (20mL) under argon was slowly added a solution of the keto-ester **209** (10.07g, 48.0mmol) in dry THF (20mL). The reaction was placed in a refrigerator (4°C) for 30 hours, and then was returned to an ice bath and stirred while water (10mL), then methanol (30mL), then 10% $NaOH_{(aq)}$ (60mL) were added. The ice bath was removed. When all of the ester had been hydrolyzed, saturated aq. sodium bicarbonate (80mL) was added. The aqueous was extracted with ether (2x100mL), then acidified to pH 2 and extracted with benzene (2x180mL). Pyridinium-p-toluenesulfonate (60mg) was added to the combined benzene layers which were then heated to reflux using a Dean-Stark trap. When the reaction was complete the benzene solution was washed with saturated aq. sodium bicarbonate (150mL) and brine (2x50mL), dried ($Na_2SO_4$), filtered and concentrated to give a white crystalline solid which was assigned the R configuration based on literature precedent (7.92g, 91%). $^1$H NMR 2.10-2.25(m, 1H), 2.68(m, 3H), 5.50(m, 1H), 7.08(t, 2H), 7.30(m, 2H).

**(c) Preparation of cis and trans-5R-5-(4-fluorophenyl)-2-hydroxy tetrahydrofuran (compound 225):**

To a stirred solution of the lactone **224** (7:25g, 40.3mmol) in dry toluene (50mL), cooled in a dry ice/acetone bath was added diisobutylaluminum hydride (1.5M in toluene)(1.5eq., 40mL). When the reaction was complete, methanol (10mL) was slowly added, then saturated aq. sodium potassium-L-tartrate (60mL) and the ice bath was removed. This solution was stored overnight (16 hours), the layers were separated and the aqueous fraction extracted with ethyl acetate (2x50mL). The combined organic layers were washed with water (3x30mL) and brine (3x30mL), dried ($Na_2SO_4$), filtered and concentrated. The product was a colorless oil which was a mixture of two diasteriomers (ca. 50/50) (6.32g, 86%). 1H NMR: 1.7(m, 1H), 1.9-2.3(m, 2H), 2.42(m, 1H), 3.60(bs, 0.5H), 3.72(bs, 0.5H), 4.98(t, 0.5H), 5.20(t, 0.5H), 5.60(bs, 0.5H), 5.72(m, 0.5H), 7.00(t, 2H), 7.25(m, 1H), 7.40(m, 1H).

**(d) Preparation of cis and trans-5R-5-(4-fluorophenyl)-2-t-butyldimethylsiloxy tetrahydrofuran (compound 226):**

To a stirred solution of the lactol **225** (6.32g, 34.7mmol) in methylene chloride (140 mL) was added imidazole (1.leq, 38.2 mmol, 2.60 grams) and TBDMS chloride (5.77 grams). After stirring overnight the reaction was filtered and concentrated. The crude product was filtered through a plug of silica to give a colorless oil which is a mixture of two diasteriomers (ca. 2:1) (9.61g, 93%). 1H NMR: 0.14(s, 6H), 0.92(s, 9H), 1.7(m, 1H), 1.9-2.2(m, 2H), 2.4-2.5(m, 1H), 4.9(m, 0.33H), 5.16(t, 0.66H), 5.59(m, 0.33H), 5.71(dd, 0.66H), 7.00(m, 2H), 7.25(m, 1.33H), 7.40(m, 0.66H).

For a sample of this compound with a racemic mixture and the 5 position, the presence of each configuration at this center was detectable using a chiral solvating agent [2,2,2-trifluoro-1-(9-anthryl)ethanol, 2.2mg substrate, 40mg CSA]. These condition showed that compound **226** had no detectable amount of the 5S isomer.

For the control, a 2:1 diasteriomeric mixture of compound **226** (2.2mg) in which the 5 position was a racemic mixture was treated with the CSA (40mg). The multiplet at 4.86-4.92ppm (0.33H) became two multiplets at 4.64-4.72 and 4.78-4.84ppm. For the other diasteriomer (same spectrum) the doublet of doublets at 5.66-5.70ppm became two sets of dd's at 5.64-5.68 and at 5.70-5.74ppm. For the chirally reduced compound, the smaller multiplet (w/CSA) appears at 4.62-4.70 and the doublet of doublets appears at 5.68-5.70ppm. No evidence of the other isomers was seen.

**(e) Preparation of 2R,5R-trans-5-(A-fluorophenyl)-2-(3-t-butyldimethylsiloxy-1-butynyl)tetrahydrofuran (compound 227):**

To a solution of **226** (500mg, 1.69mmol) in dry degassed methylene chloride (10mL), cooled to -78°C was added TMS bromide (0.25mL, 1.86mmol). This was stirred for four hours. In a separate flask containing 3-t-butyldimethylsiloxy-1-butyne (0.31g, 1.68mmol) and THF(5mL) was added n-butyllithium (1.6M in hexanes, 1.26mL, 2.02mmol). After 30 minutes, the solution was transferred by cannula to the solution from above. After two hours the reaction was poured into 2M aq. ammonium chloride (25mL) and extracted into methylene chloride (3x25mL), dried

($Na_2SO_4$), filtered and concentrated. Flash chromatography (5% ethyl acetate in hexanes) gave ther trans product as a clear oil (280mg, 48%). $^1$H NMR: 0.17(d, 6H), 0.91(s, 9H), 1.42(d, 3H), 1.8(m, 1H), 2.25-2.50(m, 2H), 4.58(m, 1H), 4.91(m, 1H) 5.09(m, 1H), 7.0(t, 2H), 7.30(m, 2H).

**(f) Preparation of 2R, 5R-trans-5-(4-Fluorophenyl)-2-(3-hydroxy-1-butynyl)tetrahydrofuran (compound 228):**

To a stirred solution of **227** (0.38g, 1.1mmol) in THF (5mL) cooled in an ice bath was added tetrabutyl ammonium fluoride (0.86g, 3.3mmol). The ice bath was removed. After 30 minutes the solvent was removed and the products were separated by flash chromatography (25% ethyl acetate in hexanes). The product was a colorless oil (170mg, 67%). $^1$H NMR: 1.48(d, 3H), 1.8(m, 1H), 2.1(m, 1H), 2.3-2.5(m, 2H), 4.58(m, 1H), 4.91(t, 1H), 5.1(t, 1H), 7.0(t, 2H), 7.29(m, 2H).

The hydroxy function of **228** was esterified with R-alpha-methoxyphenylacetic acid (DCC, DMAP, $CH_2Cl_2$, 55% after chromatography) and the resulting diasteriomers (**229+230**) were separated (flash chromatography), thus isolating the R and S isomers at the carbinol carbon. The ester was removed by base hydrolysis (KOH, 78%)to give the carbinols **231** and **232.** Absolute configurations were assigned based on the Mosher model.

**(g) Preparation of 2R, 5R-trans-5-(4-fluorophenyl)-2-(3R-3-N,O-bisphenoxycarbonyl hydroxylamino-1-butynyl) tetrahydrofuran (compound 233)**

To a cooled (ice bath) solution of 2R,5R-trans-5-((4-fluorophenyl)-2-(3S-3-hydroxy-1-butynyl)tetrahydrofuran (**231**) (29mg, 0.12mmol), triphenylphosphine (39mg, 0.15mmol) and N,O-bisphenoxycarbonyl hydroxylamine (37mg, 0.14mmol) in THF (3mL) was slowly added diisopropylazodicarboxylate (0.029mL, 0.15mmol). The ice bath was removed and when the reaction was complete (a few minutes) the solvent was removed. The product was obtained by flash chromatography (15% ethyl acetate in hexanes) as a colorless oil (32mg, 53%). $^1$H NMR: 1.65(d, 3H), 1.8(m, 1H), 2.1(m, 1H), 2.4(m, 2H), 4.94(m, 1H), 5.08(m, 1H), 5.30(m, 1H), 7.0(t, 2H), 7.15-7.40(m, 2H).

**(h) Preparation of 2R, 5R-trans-5-(fluorophenyl)-2-(3R-3-N-hydroxyureidyl-1-butynyl)tetrahydrofuran (compound 234):**

Compound **233** (32 mg) was combined in a screw cap vessel at -78°C with a stir bar, condensed ammonia (ca. 3mL) and t-butanol (ca. 2mL). The vessel was sealed and the cold bath removed. After stirring overnight at room temperature the pressure was released and the solvent was removed. The product was triturated (25% ethyl acetate in hexanes) to give a white solid (14mg, 74%). $^1$H NMR: 1.41(d, 3H), 1.8(m, 1H), 2.1(m, 1H), 2.3-2.5(m, 2H), 4.93(t, 1H), 5.08(t, 1H), 5.20(m, 1H), 5.38(bs, 1H), 7.0(t, 2H), 7.29(m, 2H). Electrospray MS: M+1=293.

The synthesis of the RRS isomer (CMI-957) proceeds in the same fashion from the ester **230** as did the RRR isomer (CMI-954) from ester 29.

**Example 7: Preparation of 2S, 5S-trans-2-(4-fluorophenoxymethyl)-5-(4-N-hydroxyureidyl-1-butynyl) tetrahydrofuran (compound 1, Figure 4) and 2S,5R-trans-2-(4-fluorophenoxymethyl)-5-(4-N-hydroxyureidylbutyl)tetrahydrofuran (compound 402, Figure 4)**

Preparation of 4S-(4-fluorophenoxymethyl)-gammabutyrolactone (compound 301, Fiaure 4) To a stirred THF (10 mL) solution of (S)-gamma-butyrolactone (1.0 g, 8.61 mmol), 4-fluorophenol (1.16 g, 10.35 mmol), and triphenylphosphine (2.49 g, 9.49 mmol) was added diisopropoxyl azodicarboxylate (1.87 µL, 9.46 mmol) dropwise. After addition, the reaction mixture was stirred at 80°C for 16 hours. The solvent was removed and the product was separated by flash column chromatography (silica, 2:1 hexane/ethyl acetate) (1.38 g, 76.3%). $^1$H NMR ($CDCl_3$); 2.27(m, 1H); 2.42(m, 1H); 2.60(m, 1H); 4.04(m, 1H); 4.15(m, 1H); 4.85(m, 1H); 6.84(m, 2H); 6.98(m, 2H).

Preparation of 2S-(4-fluorophenoxymethyl)-5-hydroxy-tetrahydrofuran (compound 302, Figure 4). To a stirred solution of lactone 301 (1.38 g, 27.22 mmol) in dry toluene (24 mL) at -78°C was added a 1.5 M toluene solution of DIBAL H (6.76 mL, 10.13 mmol) dropwise. The reaction mixture was stirred at -78°C for 2 hours. The reaction was quenched through the addition of methanol (1.7 ml) while maintaining a temperature of <-60°C. The mixture was warmed to -20°C followed by the addition of saturated aqueous potassium sodium tartrate solution (10 mL) while maintaining the reaction temperature between -10 and 0°C. The reaction mixture was stirred at room temperature overnight and then the two phases were separated. The aqueous layer was extracted with ethyl acetate. The combined organic layers were washed with water, saturated NaCl solution, and then concentrated in vacuo to leave an oil which was purified by flash column chromatography (silica, 1:1 hexane/ethyl acetate) (1.41g, 101%). $^1$H NMR ($CDCl_3$); 1.80 (m, 1H); 2.05(m, 2H); 2.26(m, 1H); 3.93(m, 2H); 4.04(m, 2H); 4.47(m, 0.5H); 4.61(m, 0.5H); 5.57(m, 0.5H); 5.66(m, 0.5H); 6.88(m, 2H); 6.98(m. 2H).

Preparation of 2S-(4-fluorophenoxymethyl)-5-(t-butyldimethylsiloxy) tetrahydrofuran (compound 303, Figure 4) To a stirred solution of lactol 302 (1.41 g, 6.65 mmol) in methylene chloride (25 mL) was added imidazole (498.1 mg, 7.32 mmol) and TBDMS chloride (1.10 g, 7.32 mmol). The reaction mixture was stirred at room temperature overnight and then the reaction was filtered and the filtrate was concentrated. The crude product was purified by flash column chromatography using 9:1 hexane/ethyl acetate as a solvent to give a colorless oil which is a mixture of two diasteriomers (ca. 2:1) (1.22 g, 56.2%). $^1$H NMR ($CDCl_3$); 0.11(s, 6H); 0.90(s, 9H); 1.80-2.10(m, 3H); 2.22(m, 1H); 3.91 (m, 2H); 4.38(m, 0.33H); 4.50(m, 0.67H); 5.52(m, 0.33H); 5.59(m, 0.67H); 6.86(m, 2H); 6.96(m. 2H);

Preparation of 2S,5S-trans-2-(4-fluorophenoxymethyl)-5-(4-t-butyldimethylsiloxy-1-butynyl)tetrahydrofuran (compound 304, Figure 4) and 2S, 5R-cis-2-(4-fluorophenoxymethyl)-5-(4-t-butyldimethylsiloxy-1-butynyl) tetrahydrofuran (compound 305, Figure 4) To a stirred solution of 303 (720 mg, 2.21 mmol) in dry methylene chloride (10 mL), cooled to -78°C was added TMS bromide (349.8 µL, 2.65 mmol). The reaction mixture was stirred at -78°C for 4 hours. In a separate flask containing 4-t-butyldimethylsiloxy-1-butyne (812.8 mg, 4.42 mmol) and THF (10 mL) was added n-butyllithium (2.5M in hexane, 2.65 mL, 6.63mmol). After 30 minutes, this was transferred by cannula to the solution from above. After two hours, the reaction was quenched through the addition of saturated aq. ammonium chloride solution and extracted with methylene chloride, dried over MgSO4, filtered and concentrated. Flash column chromatography (silica, 95:5 hexane/ethyl acetate) yielded two products, trans compound 304 (210 mg) and cis compound 305 (160 mg), and the mixture of these two compounds (50 mg). The total yield is 48.5%. $^1$H NMR ($CDCl_3$); 304: 0.10 (s, 6H); 0.91(s, 9H); 1.87(m, 1H); 2.01(m, 1H); 2.22(m, 2H); 2.43(t, 2H); 3.72(t, 2H); 3.92(d, 2H); 4.47(m, 1H); 4.73(m. 1H); 6.86 (m, 2H); 6.95(t, 2H). 305: 0.09 (s, 6H); 0.90(s, 9H); 1.92-2.20(m, 4H); 2.42 (m, 2H); 3.70(t, 2H); 3.92(m, 1H); 4.07(m, 1H); 4.29(m, 1H); 4.62(m, 1H); 6.86(m, 2H); 6.96(t. 2H.

In the preparation of compounds 304 and 305, oxygen protecting groups known to those skilled in the art other than 4-t-butyldimethylsilyl can be used as desired.

In order to determine the stereochemistry of this molecule, a NOE difference experiment was carried out for both 304 and 305. In the NOE difference experiment of 304, the multiplet at 4.73 ppm was irradiated with a very low rf decoupling pulse and the data work-up was done so as to only measure the presence of an increase in signal. This would represent a positive NOE effect and would indicate the close spacial relationship of these protons. In this experiment, a NOE was found for the multiplet at 2.22 ppm which are furan ring protons. When the multiplet at 4.47 ppm was irradiated with a very low rf decoupling pulse and the data work-up was done so as to only measure the presence of an increased in signal. A NOE was found for the multiplet at 2.22 ppm which are furan ring protons. Another NOE was also seen for the protons at 3.92 ppm which are the protons on the methylene next to this multiplet, indicating that this multiplet represents the proton next to the methylene.

In the NOE difference experiment of 305, the triplet at 4.62 ppm was irradiated with a very low rf decoupling pulse and the data work-up was done so as to only measure the presence of an increase in signal. This would represent a positive NOE effect and would indicate the close spacial relationship of these protons. In this experiment, a NOE was found for the multiplet at 4.29 ppm which is the other methine furan proton. Another NOE was also seen for the multiplet at 2.17 ppm which are furan protons. When the multiplet at 4.29 ppm was irradiated with a very low rf decoupling pulse and the data work-up was done so as to only measure the presence of an increase in signal. A NOE was found for the triplet at 4.62 ppm which is the other methine furan proton. Another NOE was seen for the protons at 3.92 and 4.07 ppm which are the protons on the methylene next to this multiplet, indicating that this multiplet represents the proton next to the methylene. Another NOE was also seen for the multiplet at 2.11 ppm which are furan protons.

Preparation of 2S,5S-trans-2-(4-fluorophenoxymethyl)-5-(4-t-hydroxy-1-butyl)tetrahydrofuran (compound 306, Figure 4)

To a stirred solution of 304 (210 mg, 0.54 mmol) in THF (1.4 mL), cooled in an ice bath, was added tetrabutyl ammonium fluoride (420.3 mg, 1.61 mmol). The ice bath was removed and the reaction was stirred at room temperature for 1 hour. The solvent was removed and the product was separated by flash column chromatography (silica, 1:1 hexane/ethyl acetate) (124 mg, 83.2%). $^1$H NMR ($CDCl_3$); 1.88(m, 1H); 2.02(m, 1H); 2.25(m, 2H); 2.50(m, 2H); 3.72 (t, 2H); 3.93(d, 2H); 4.48(m, 1H); 4.76(m, 1H); 6.84(m, 2H); 6.96(t,2H).

Preparation of 2S,5S-trans-2-(4-fluorophenoxymethyl)-5-(4-N,O-bisphenoxycarbonylhydroxylamino-1-butynyl) tetrahydrofuran (compound 307, Figure 4)

To a cooled (ice bath) solution compound 306 (124.0 mg, 0.45 mmol), triphenylphosphine (128.9 mg., 0.49 mmol) and N,O-bisphenoxycarbonyl hydroxylamine (147.3 mg, 0.54 mmol) in THF (5 mL) was added diisopropoxylazodicarboxylate (94.1 µL, 0.48 mmol). The ice bath was removed and the reaction was warmed to room temperature and stirred at room temperature for 30 minutes. The solvent was removed and the product was purified by flash column chromatography (silica, 4:1 hexane/ethyl acetate) (195 mg, 82.0%). $^1$H NMR ($CDCl_3$); 1.85(m, 1H); 2.03(m, 1H); 2.22(m, 2H); 2.75(m, 2H); 3.92(d, 2H); 4.05(m, 2H); 4.47(m, 1H); 4.76(m, 1H); 6.84(m, 2H); 6.95(m,2H); 7.26(m, 5H); 7.41 (m, 5H).

Preparation of 2S,5S-trans-2-(4-fluorophenoxymethyl)-5-(4-N-hydroxyureidyl-1-butynyl)tetrahydrofuran (compound 401, Figure 4)

In a screw top vessel was placed $NH_3$ at -78°C (approximately 1-2 mL). Compound 307 (195.0 mg, 0.37 mmol), predissolved in 20 mL methanol, was added to this cold liquid nitrogen. The vessel was sealed and the dry ice bath was removed. The reaction mixture was stirred at room temperature for 16 hours. The reaction mixture was cooled again by dry ice bath and the pressure was released. The vessel was opened and the solvent was removed. The product was isolated by flash column chromatography using ethyl acetate as a solvent to provide a solid (108 mg. 91.7%). $^1H$ NMR ($CDCl_3$); 1.84(m, 1H); 2.01(m, 1H); 2.22(m, 2H); 2.55(t, 2H); 3.75(t, 2H); 3.94(m, 2H); 4.48(m, 1H); 4.74(t, 1H); 5.25(bs, 2H); 6.86(m,2H); 6.98(m, 2H).

Preparation of 2S,5S-trans-2-(4-fluorophenoxymethyl)-5-(4-N-hydroxyureidyl-1-butynyl)tetrahydrofuran (402)

Compound 1 (75 mg, 0.23 mmol) was dissolved in 2 mL of ethyl acetate and then Pd/C (10%) (15 mg) was added and hydrogenated at balloon pressure for 16 hours. The reaction was filtered and the filtrate was concentrated. The product was isolated by flash column chromatography using ethyl acetate as solvent (70 mg, 92.2%). $^1H$ NMR ($CDCl_3$); 1.50-1.70(m, 8H); 2.10(m, 2H); 3.58(m, 2H); 3.91(m, 2H); 4.08(m, 1H); 4.40(m, 1H); 5.15(bs, 2H); 6.87(m, 2H); 6.97(t, 2H); 7.40(bs,1H).

**II. Pharmaceutical Compositions**

Humans, equines, canines, bovines and other animals, and in particular, mammals, suffering from inflammatory diseases, and in particular, disorders mediated by PAF or products of 5-lipoxygenase can be treated by administering to the patient an effective amount of one or more of the above-identified compounds or a pharmaceutically acceptable derivative or salt thereof in a pharmaceutically acceptable carrier or diluent to reduce formation of oxygen radicals. The active materials can be administered by any appropriate route, for example, orally, parenterally, intravenously, intradermally, subcutaneously, or topically, in liquid, cream, gel, or solid form, or by aerosol form.

As used herein, the term pharmaceutically acceptable salts or complexes refers to salts or complexes that retain the desired biological activity of the above-identified compounds and exhibit minimal undesired toxicological effects. Non-limiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid; (b) base addition salts formed with metal cations such as zinc, calcium, bismuth, barium, magnesium, aluminum, copper, cobalt, nickel, cadmium, sodium, potassium, and the like, or with a cation formed from ammonia, N,N-dibenzylethylenediamine, D-glucosamine, tetraethylammonium, or ethylenediamine; or (c) combinations of (a) and (b); e. g., a zinc tannate salt or the like. The compounds can also be administered as pharmaceutically acceptable quaternary salts known by those skilled in the art, which specifically include the quaternary ammonium salt of the formula $-NR_3^+Z^-$ , wherein R is alkyl or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate.

The active compound is included in the pharmaceutically acceptable carrier or diluent in an amount sufficient to deliver to a patient a therapeutically effective amount without causing serious toxic effects in the patient treated. A preferred dose of the active compound for all of the above-mentioned conditions is in the range from about 10 ng/kg to 300 mg/kg, preferably 0.1 to 100 mg/kg per day, more generally 0.5 to about 25 mg per kilogram body weight of the recipient per day. A preferred dosage for cardiovascular indications is in the range 10 ng/kg to 20 mg/kg. A typical topical dosage will range from 0.01 - 3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

The compound is conveniently administered in any suitable unit dosage form, including but not limited to one containing 1 to 3000 mg, preferably 5 to 500 mg of active ingredient per unit dosage form. A oral dosage of 25-250 mg is usually convenient.

The active ingredient is preferably administered to achieve peak plasma concentrations of the active compound of about 0.00001 - 30 mM, preferably about 0.1 - 30 μM. This may be achieved, for example, by the intravenous

injection of a solution or formulation of the active ingredient, optionally in saline, or an aqueous medium or administered as a bolus of the active ingredient.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or enteric agents.

The active compound or pharmaceutically acceptable salt or derivative thereof can be administered as a component of an elixir, suspension, syrup, wafer, chewing gum or the like. A syrup may contain, in addition to the active compounds, sucrose as a sweetening agent and certain preservatives, dyes and colorings and flavors.

The active compound or pharmaceutically acceptable derivatives or salts thereof can also be mixed with other active materials that do not impair the desired action, or with materials that supplement the desired action, such as antibiotics, antifungals, other antiinflammatories, or antiviral compounds.

Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation (CA) and Scios Nova (Baltimore, MD).

Liposomal suspensions may also be pharmaceutically acceptable carriers. These may be prepared according to methods known to those skilled in the art, for example, as described in U.S. Patent No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives are then introduced into the container. The container is then swirled by hand to free lipid material from the sides of the container and to disperse lipid aggregates, thereby forming the liposomal suspension.

### III. Biological Activity

A wide variety of biological assays have been used to evaluate the ability of a compound to act as a PAF receptor antagonist, including the ability of the compound to bind to PAF receptors, and the effect of the compound on various PAF mediated pathways. Any of these known assays can be used to evaluate the ability of the compounds disclosed herein to act as PAF receptor antagonists.

For example, PAF is known to induce hemoconcentration and increased permeability of microcirculation leading to a decrease in plasma volume. PAF mediated acute circulatory collapse can be used as the basis of an assay to evaluate the ability of a compound to act as a PAF antagonist, by analyzing the effect of the compound on PAF induced decreased plasma volume in an animal model such as mouse.

Endotoxemia causes the release of chemical mediators including eicosanoids, PAF, and tumor necrosis factor (TNF) that stimulate a variety of physiologic responses including fever, hypotension, leukocytosis, and disturbances in glucose and lipid metabolism. Endotoxemia can result in severe shock and death. Endotoxin-induced mouse mortality is a useful animal model to evaluate the pharmacological effect of compounds on endotoxic shock.

Two other common assays used to evaluate the ability of a compound to act as a PAF receptor antagonist are platelet aggregation in vitro and hypotension in rats (Shen, *et al*., "The Chemical and Biological Properties of PAF Agonists, Antagonists, and Biosynthetic Inhibitors", Platelet-Activating Factor and Related Lipid Mediators, F. Snyder, Ed. Plenum Press, New York, NY 153 (1987)).

A wide variety of biological assays have also been used to evaluate the ability of a compound to inhibit the enzyme 5-lipoxygenase. For example, a cytosol 5-lipoxygenase of rat basophilic leukemia cells (RBL) has been widely utilized in studies on leukotriene biosynthesis. Compounds that inhibit 5-lipoxygenase decrease the levels of leukotrienes.

Another biological assay used to evaluate the ability of a compound to inhibit the enzyme 5-lipoxygenase is based on the classic pharmacological model of inflammation induced by inhibition of $LTB_4$ from ionophore stimulated human whole blood.

### Example 5 Ability of Compound to Bind to PAF Receptors

#### (a) Preparation of Human Platelet Membranes

Human platelet membranes are prepared from platelet concentrates obtained from the American Red Cross Blood Services (Dedham, MA). After several washes with platelet wash solution (150 mM NaCl, 10 mM Tris, and 2 mM EDTA, pH 7.5), the platelet pellets are resuspended in 5 mM $MgCl_2$, 10 mM Tris, and 2 mM EDTA at pH 7.0. The cells are then quickly frozen with liquid nitrogen and thawed slowly at room temperature. The freezing and thawing procedure is repeated at least three times. For further fractionation of membrane fragments, the lysed membrane suspension is layered over the top of a discontinuous sucrose density gradient of 0.25, 1.03, and 1.5 M sucrose prepared in 10 mM $MgCl_2$, 10 mM Tris and 2 mM EDTA, pH 7.0, and centrifuged at 63,500 x g for 2 hr. The membrane fractions banding between 0.25 and 1.03 M (membrane A) and between 1.03 and 1.5 M (membrane B) are collected separately. The protein concentration of the membrane preparations is determined by Lowry's method with bovine serum albumin (BSA) as the standard. The membranes are then separated into smaller fractions (4 ml each) and stored at -80° C and thawed before use.

#### (b) [$^3$H]PAF Binding inhibition

The ability of [$^3$H]PAF to bind to specific receptors on human platelet membranes is evaluated at optimal conditions at pH 7.0 and in the presence of 10 mM $MgCl_2$. Membrane protein (100 μg) is added to a final 0.5 ml solution containing 0.15 pmol (0.3 nM concentration) of [$^3$H]PAF and a known amount of unlabeled PAF or PAF receptor antagonist in 10 mM $MgCl_2$, 10 mM Tris and 0.25% BSA at pH 7.0. After incubation for four hours at 0°C, the bound and unbound [$^3$H]PAF are separated through a Whatman GF/C glass fiber filter under vacuum. No degradation of filter bound [$^3$H]PAF should be detected under this assay condition. The nonspecific binding is defined as the total binding in the presence of excess unlabeled PAF (1 mM) where no further displacement is found with higher concentrations of either unlabeled PAF or PAF analogs or PAF receptor antagonists. The specific binding is defined as the difference between total binding and nonspecific binding.

To determine the relative potency of tested compounds, [$^3$H]PAF binding in the presence of inhibitors is normalized in terms of percent inhibition by assigning the total binding in the absence of inhibitors as 0% inhibition and the total binding in the presence of 1 mM unlabeled PAF as 100%. The percent inhibition by the compound can be calculated by the formula expressed below:

% inhibition = [(Total binding - total binding in the presence of compound)/nonspecific binding] x 100%

The $IC_{50}$ is calculated as the concentration of the inhibitor necessary to obtain 50% inhibition of the specific [$^3$H]PAF binding and is calculated by a nonlinear regression computer software program, GraphPad Inplot, version 3.0 (GraphPad software, San Diego, CA).

**Example 6 Effect of Compound on PAF-induced Hemoconcentration**

**(a) Animals**

Female CD-1 mice, weighing 16-20 grams, are obtained from Charles River Laboratory (Wilmington, MA). Tap water and rodent laboratory chow (5001, Purina Mills, St. Louis, MO) are provided ad libitum. The mice are housed for an average of four days prior to use.

**(b) Hematocrit measurement**

PAF (1-O-alkyl-2-acetyl-*sn*-glyceryl-3-phosphorylcholine, Sigma Chemical Co.) is dissolved in 0.25% bovine serum albumin (BSA) in 0.9% NaCl solution. Except for dose-response studies, 10 μg (10 ml/kg) of PAF solution is injected into the tail vein. All test compounds are dissolved in 0.5 DMSO saline solution and intravenously injected at 3 mg/kg body weight 15 minutes prior to PAF challenge. Thirty to fifty μL blood is collected by cutting the tail end into a heparinized micro-hematocrit tube (O.D. 1.50 mm) 15 minutes after PAF administration. All test compounds are given intravenously at 3 mg/kg 15 minutes before PAF (10 ug/kg, intravenously) or AA (0.5 mg/ear) in mice.

**Example 7 Effect of Compounds on Cytosol 5-Lipoxygenase of Rat Basophile Leukemia Cells**

**(a) Enzyme preparation**

Washed rat RBL cells (4x108) were suspended in 20 ml of 50 M potassium phosphate buffer at pH 7,4 containing 10% ethylene glycol/l mM EDTA (Buffer A). The cell suspension was sonicated at 20 KHz for 30 seconds, and the sonicate was centrifuged at 10,000 x g for 10 minutes, followed by further centrifugation at 105,000 x g for 1 hr. The supernatant solution (cytosol fraction) containing 5-lipoxygenase was stored at - 70°C. Protein concentration was determined according to the procedure of Bradford (Bradford Dye Reagent) with bovine serum albumin as a standard.

**(b) Enzyme assay**

For routine assay of 5-lipoxygenase the mixture contained 50 mM potassium phosphate buffer at pH 7.4, 2 mM $CaCl_2$, 2 mM ATP, 25 M arachidonic acid (0.1 Ci) and enzyme (50-100 mg of protein) in a final volume of 200 L. The reaction was carried out at 24°C for 3 minutes. The mixture was extracted with 0.2 ml of an ice-cold mixture of ethyl ether:methanol: 0.2 M citric acid (30:4:1). The extract was subjected to thin-layer chromatography at -10°C in a solvent system of petroleum ether:ethyl ether:acetic acid (15:85:0.1). The silica gel zones corresponding to authentic arachidonic acid and its metabolites were scraped into scintillation vials for counting. The enzyme activity was expressed in terms of the amount of arachidonic acid oxygenated for 3 minutes. Representative compounds 9, 11, 14, and 15, identified above, showed activity in this assay.

**Example 8 Inhibition of soluble 5-lipoxygenase in RBL-1 cell extract**

RBL-1 cells are grown to confluence (2 x $10^6$/ml) in spinner flasks according to specifications from the ATCC. Cells are harvested and washed twice in calcium-free and magnesium-free PBS. Cells are suspended at 2 x $10^7$/ml in 50 mM $K_2HPO_4$pH 7.4, 10% PEG-8000, 1 mM EDTA, 1 mM PMSF, and then sonicated. The lysate is centrifuged at 100,000 x g for 1 hour at 4°C, and the supernatant (cytosol) is removed and stored in aliquots at -70°C.

The 5-LO activity in the RBL-1 cytosol is determined as follows: 0.2 ml reactions consisting of 5 mM $CaCl_2$, 2mM ATP, 50 mM $K_2HPO_4$ph 7.4, varying concentrations of test compound (from 10 ml of compound dissolved in DMSO), and a concentration of RBL-1 cytosol that will convert 50% of the [$^{14}$C] arachidonic acid substrate to oxygenated products (determined experimentally for each cytosol preparation), are incubated for 10 minutes at room temperature. The reaction is initiated by the addition of 5 ml [$^{14}$C] arachidonic acid from an ethanolic stock (final concentration = 9.5 mM), and allowed to proceed for 3 minutes. The reaction is terminated by the addition of 0.2 ml of cold ethyl ether: methanol: 0.2 M citric acid (30:4:1) followed by centrifugation at 10,000 x g for 1 minute. 50 ml of the organic phase is drawn into a glass capillary pipet and spotted onto Silica Gel 60A TLC plates (Whatman #LK6D). The plates are developed in petroleum ether: ethyl ether: acetic acid (15:85:0.1) for 30 minutes at room temperature. Plates are exposed to Kodak XAR-5 film for 24 hours. The film is developed, scanned using a densitometer, and the peak areas of the

arachidonic acid and its products are calculated. The % inhibition is determined from the amount of [$^{14}C$] arachidonic acid converted into oxygenated products in samples containing test compound relative to that of control samples (no test compound).

Table 5 provides data for the inhibition of soluble 5-lipoxygenase in RBL-1 cell extract by racemic compound 202, as well as its enantiomers, compounds 216, 217, 234, and 236.

**Example 9 Inhibition of Leukotriene $B_4$ Production in Ionophore-stimulated human whole blood**

Human blood is drawn into heparinized blood collection tubes, and aliquoted in 1 ml portions into 1.5 ml microfuge tubes. Five milliliters of test compound at varying concentrations, dissolved in DMSO, is added to the blood sample and incubated for 15 minutes at 37°C. Calcium ionophore (5 ml) (A23187) in DMSO is added to a final concentration of 50 mM, and the samples are incubated for 30 minutes at 37°C. Samples are then centrifuged at 1100 x g (2500 rpm, H1000B rotor, in a Sorvall centrifuge) for 10 minutes at 4°C. 100 ml of supernatant is transferred into a 1.5 ml microfuge tube, 400 ml of cold methanol is added, and proteins are precipitated on ice for 30 minutes. The samples are centrifuged at 110 x g for 10 minutes at 4°C, and the supernatant is assayed for $LTB_4$ using a commercially available EIA kit (Cayman Chemical) according to manufacturer's specifications.

Table 5 provides data for the inhibition of leukotriene $B_4$ production in ionophore-stimulated human whole blood by racemic compound 202, as well as its enantiomers, compounds 216, 217, 234, and 236.

**Example 9 Ex-vivo mouse and rat whole blood 5-lipoxygenase evaluation**

CD-1 female mice, weighing 18-25 grams, and CD female rates, weighing 150-230 grams, were obtained from Charles River Labs. Compounds were dissolved in 0.5% DMSO in 0.9% NaCl for administration in mice (0.5 mg/ml) and in an alcohol vehicle (2% benzyl alcohol, 1% ethanol, 40% PEG 300 10% propylene glycol, 47% of 5% dextrose plus 3.5% pluronic F-68 in $DiH_2O$) for use in rates (5 mg/ml). Animals were injected with compound (5 mg/kg) or corresponding vehicle (0.5% DMSO in saline, 10 ml/kg for mice; alcohol vehicle, 1. ml/kg for rats) 15 minutes before they were sacrificed by decapitation. Heparinized whole blood (0.3 ml) was added into 1.5 ml Eppendorf centrifuge tub containing 3 ml of 2 mM calcium ionophore A23187 (the final concentration of A23187 was 20 mM). The sample was incubated for 30 minutes in a water bath of 37°C, and then centrifuged for 2 minutes. The plasma was diluted (x120) and assayed for $LTB_4$ using EIA.

Table 5 provides data for the ex-vivo mouse and rat whole blood 5-lipoxygenase values on administration of racemic compound 202, as well as its enantiomers, compounds 216, 217, 234, and 236.

**Example 10 Rate of Glucuronidation**

The rate of glucuronidation is a measure of the metabolic stability in vivo of the compounds disclosed herein.

*In vitro* glucuronidation reactions were carried out with reaction mixtures containing 2 mg/ml of human microsomal protein, 5 mM magnesium chloride, 100 mM Tris HCl (pH = 7.4), 0.1 - 1.0 mM substrate and 3 mM UDP-glucuronic acid. After incubation at 37° C for 0 (control), 15, 30, 45, 60, 90, 120, 180, 240 minutes, 40 µl aliquots of the reaction mixture were mixed with 80 µl of acetonitrile and centrifuged to remove the precipitated protein. Aliquots of the supernatant -were analyzed by reverse phase HPLC to determine the disappearance of parent compounds and formation of metabolites.

Table 5 provides data for, and Figure 2 illustrates, the rate of glucuronidation of racemic compound 202, as well as its enantiomers, compounds 216, 217, 234, and 236.

Figure 3 illustrates the rate of glucuronidation for illustrated enantiomers.

**Example 11 Effect of Compounds on Cytosol 5-Lipoxygenase of Rat Basophile Leukemia Cells**

RBL-2H3 cells were grown to confluence in tissue culture flasks according to Carter et al. (J Pharm Exp Ther 256(3); 929-937, 1991). The cells were harvested and washed five times in calcium-and magnesium-free D-PBS. The cells were suspended at 2 x $10^7$/ml in 10 mM BES, 10 mM PIPES, pH 6.8, 1 mM EDTA, and then sonicated. The sonicate was centrifuged at 20,000 x g for 20 minutes at 4°C. The supernatant was then removed and stored in aliquots at -70°C.

The 5-LO activity in the RBL-2H3 preparation was determined as follows: 0.1 ml reactions consisting of 0.7 mM $CaCl_2$, 100 mM NaCl, 1 mM EDTA, 10 mM BES, 10 MM PIPES, pH 7.4, varying concentrations of test compound dissolved in DMSO (7.5% DMSO final in assay), and an amount of the RBL-2H3 preparation that will convert 15% of the arachidonic acid substrate mixture to oxygenated products (determined experimentally for each RBL-2H3 preparation), were incubated for 20 minutes at room temperature. The reaction was initiated by the addition of 5 ul of the

arachidonic acid substrate mixture (0.944 nmol [$^{14}C$] arachidonic acid and 6.06 nmol arachidonic acid per assay in 0.028% $NH_4OH$), and allowed to proceed for 5 minutes at 37°C. The reaction was terminated by the addition of 0.12 ml of a mixture of (i) 1.66 mg/ml triphenylphosphine in ethyl ether; (ii) methanol; and (iii) 0.2M citric acid (30:4:1); followed by centrifugation at 1000 x g for 1 minute. 50 ul of the organic phase was drawn into a glass capillary piper and spotted onto silica gel 60A TLC plates (Whatman #6KDF). The plates were developed in ethyl ether acetic acid (100:0.1) for 25 minutes at room temperature. The plates were exposed to Kodak X-OMAT AR film for 40 hours. The film was developed, scanned using a densitometer, and the peak areas of arachidonic acid and its product(s) are calculated. The percent inhibition was determined from the amount of [14C]-arachidonic acid converted into oxygenated products in samples containing test compound relative to that of control samples (no test compound).

The results are provided in Table 4.

**Example 12 Inhibition of Leukotriene $B_4$ Production in Ionophore-stimulated human whole blood**

Human blood was drawn into heparinized blood collection tubes, and aliquoted in 1 ml portions into 1.5 ml microfuge tubes. Test compound (5 ml) of varying concentrations, dissolved in DMSO, was added to the blood sample and incubated for 15 minutes at 37°C. Calcium ionophore (5 ml, A23187) in DMSO was added to a final concentration of 50 mM, and the samples were incubated for 30 minutes at 37°C. Samples are then centrifuged at 1100 x g (2500 rpm, H1000B rotor, in a Sorvall centrifuge) for 10 minutes at 4°C. Supernatant (100 ml) was transferred into a 1.5 ml microfuge tube, 400 ml of cold methanol added, and proteins precipitated on ice for 30 minutes. The samples were centrifuged at 110 x g for 10 minutes at 40°C, and the supernatant assayed for $LTB_4$ using a commercially available EIA kit (Cayman Chemical) according to manufacturer's specifications.

The results are provided in Table 5.

| | RBL IC50 nM | HWB IC5o µm | ex vivo LTB4 dose mg/k | % inh. | time minutes |
|---|---|---|---|---|---|
| 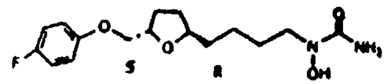 Compound 402 | 1230<br>1400 | 0.153 | MOUSE | | |
| | | | 3,iv | 89 | 15 |
| | | | 3,iv | 22 | 60 |
| | | | 3,iv | 36 | 180 |
| | | | RAT | | |
| | | | 2,po | 34 | 900 |
| | | | 5,po | 34 | 900 |
| 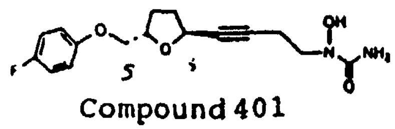 Compound 401 | 1380<br>1490<br>560<br>1600<br>720 | 0.094<br>0.27<br>0.078 | MOUSE | | |
| | | | 3,iv | 94 | 15 |
| | | | 3,iv | 86 | 60 |
| | | | 3,iv | 44 | 180 |
| | | | RAT | | |
| | | | 5,po | 29 | 900 |
| | | | 2,po | 93 | 60 |
| | | | 2,po | 5 | 360 |
| 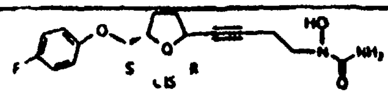 Compound 403 | 2000<br>1260<br>1560<br>880 | 0.43<br>0.52 | RAT | | |
| | | | 2,po | 86 | 60 |
| | | | 2,po | 63 | 360 |

TABLE 4

| | RBL IC50 nM | HWB IC5o µm | ex vivo LTB4 dose mg/k | % inh. | time minutes |
|---|---|---|---|---|---|
| 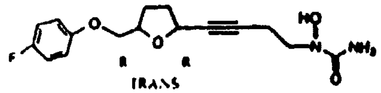 Compound 404 | 1350<br>1380 | 0.15<br>0.27 | | | |
| 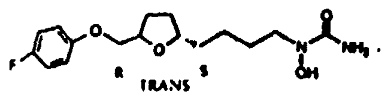 Compound 405 | 950 | 0.42 | | | |
| 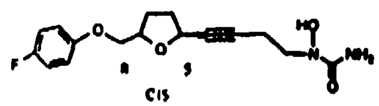 Compound 406 | 910 | 0.18 | | | |

TABLE 4 (continued)

**Example 4 Ex-vivo mouse whole blood 5-lipoxygenase evaluation**

CD-1 female mice, weighing 18-25 grams, and CD female rats, weighing 150-230 grams, were obtained from Charles River Labs. Test compounds were dissolved in 0.5% DMSO in 0.9% NaCl for administration in mice (0.5 mg/ml) and in an alcohol vehicle (2% benzyl alcohol, 1% ethanol, 40% PEG 300, 10% propylene glycol, 47% of 5% dextrose and 3.5% pluronic F-68 in $DiH_2O$) for use in rats (5 mg/ml). Animals were injected with compounds (5 mg/kg) or corresponding vehicle (0.5% DMSO in saline, 10 ml/kg for mice; alcohol vehicle, 1 ml/kg for rats) 15 minutes before they were sacrificed by decapitation. Heparinized whole blood (0.3 ml) was added into 1.5 ml Eppendorf centrifuge tube containing 3 ml of 2 mM calcium ionophore A23187 (the final concentration of A23187 was 20 mM). The sample was incubated for 30 minutes in a water bath at 37°C, and then centrifuged for 2 minutes. The plasma was diluted (x120) and assayed for $LTB_4$ using EIA.

The results are provided in Table 5.

**Example 5 Glucuronidation Studies**

The rate of glucuronidation is a measure of the metabolic stability in vivo of the compounds disclosed herein.

*In vitro* glucuronidation reactions were carried out with reaction mixtures containing 2 mg/ml of human microsomal protein, 5 mM magnesium chloride, 100 mM Tris HCl (pH = 7.4), 0.1 - 1.0 mM substrate and 3 mM UDP-glucuronic acid. After incubation at 37° C for 0 (control), 15, 30, 45, 60, 90, 120, 180, and 240 minutes, 40 µl aliquots of the reaction mixture were mixed with 80 µl of acetonitrile and centrifuged to remove the precipitated protein. Aliquots of the supernatant were analyzed by reverse phase HPLC to determine the disappearance of parent compounds and formation of metabolites. The results are provided in Figure 5.

**Example 6 Eosinophil infiltration Assay**

Accumulation of inflammatory cells in the lung is one of the pathological features of asthma. Elevation of leukocytes, particularly of eosinophils, in blood and lung lavage fluid has been observed after allergen inhalation in the patients. Eosinophils appear to be important effector cells in allergic inflammation, with the cytotoxic properties of its granule proteins and the potential of releasing inflammatory mediators. Prevention of allergen-induced eosinophil influx into the lung is considered a credible target for novel anti-asthmatic drugs.

Leukotrienes are products of the arachidonic acid 5-lipoxygenase (5-LO) pathway. Lipoxygenase metabolites (LTB4, 5-oxo-15-hydroxy-eicosatetraenoic acid) have been identified that possess potent activity to recruit eosinophils. A 5-LO inhibitor which is able to block immediate bronchoconstriction and also to reduce later accumulation of eosinophils into lung tissue consequent to allergen challenge may be beneficial to the prevention and treatment of asthma.

Eosinophil infiltration into the lung can be measured by counting the cell number in the bronchoalveolar lavage fluid (BALF) from allergen-challenged guinea-pigs or mice.

***Guinea pig model:*** Female Hartley guinea-pigs, weighing 400-500g, were actively sensitized to ovalbumin (OVA) by i.p. injection of 20 µg OVA and 100 mg $Al(OH)_3$ in 0.5 ml 0.9%NaCl on Day 1 and Day 2. Animals were challenged with 0.5% OVA (in 0.9% NaCl) aerosol for 30 sec on Day 15 and Day 16. The compounds were prepared in 10% PEG 200 or 0.5% carboxymethylcellulose and administered p.o. 3 times (1 hr. before each challenge and between the two challenges). To prevent histamine release-induced death, pyrilamine (3 mg/kg, i.p.) was given 15 minutes before each challenge. After 24 hours following the first challenge (or 4 hours after the last challenge), animals were bled from the carotid under anesthesia. BAL was performed with 2 x 10 ml of 0.5 mM EDTA in DPBS (w/o $Ca^{2+}$, $Mg^{2+}$) at 37 °C via a trachea cannulation. The total cells in BAL fluid were measured by a Sysmex microcellcounter (F-800) and the differential cells were counted on a cytospin preparation. Percent of Inhibition on total cell or eosinophil accumulation = [(vehicle - sham)-(treated - sham)]/(vehicle-sham)x100

***Mice model:*** Male C57 BL/6 mice, weighing 21-23g, were actively sensitized to OVA by administering 10 µg OVA and 1 mg $Al(OH)_3$ in 0.2 ml 0.9% NaCl on Day 1. Hypersensitivity was developed following a daily inhalation of aerosolized 1% OVA or saline for 30 minutes on Day 14 to Day 21. The compounds were prepared in a 10% PEG 200 or 0.5% carboxymethylcellulose and administered at 20 mg/kg orally, b.i.d. on Day 18 to Day 22. Animals were bled from the carotid under anesthesia four hours after the last inhalation of OVA. BAL was performed with 2 x 1 ml DPBS (w/o 4 $C_8^{2+}$, $M_g^{2+}$) containing 0.5 mM sodium EDTA at 37°C via a tracheal cannulation. The total cells in BAL fluid were counted by a Sysmex microcellcounter (F-800). The differential cells in BAL fluid were counted by a Sysmex microcellcounter (F-800). The differential cells were counted on a cytospin preparation and white giemsa stain. % of Inhibition on total cell or Eosinophil accumulation = [(vehicle - sham)-(treated - sham)]/(vehicle-sham)x100. (See Yeadon M. et al. Agents Actions 38:8-17, 1993; Brusselle G.G. et al. ALA'94, A754; Schwenk U. et al. J. Biol. Biochem. 267:12482-12488, 1992; and Clinic M. et al. Cur. Opin. Immunol. 6:860-864, 1994).

Table 5

| STRUCTURE | RBL dose uM | RBL % inh. | RBL IC50 nM | HWB dose uM | HWB % inh. | HWB IC50 | ex vivo LTB4 dose mg/k | ex vivo LTB4 % Inh. | ex vivo LTB4 IC50 |
|---|---|---|---|---|---|---|---|---|---|
| 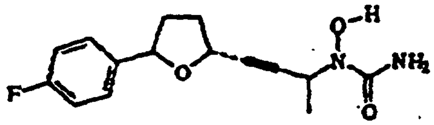<br>207 | 1.6 | 20 | 2800 | | | 0.48 | RAT<br>5,po 96,60'<br>5,po 96,160'<br>2,po 43,60'<br>2,po 38,360' | | |
| 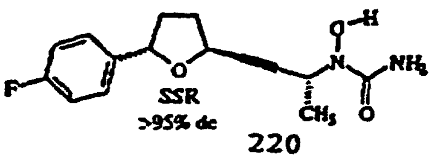<br>SSR<br>>95% dc<br>220 | | | 3800 | | | 0.76 | | | |
| | | | | | | | RAT<br>2,po 88,60'<br>2,po 57,160'<br>2,po 38,360' | | |
| 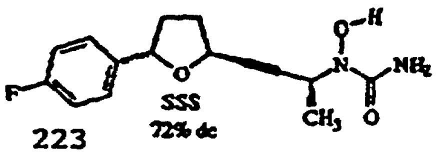<br>223 SSS<br>72% dc | | | 3500 | | | 0.22 | | | |
| | | | | | | | RAT<br>2,po 40,60'<br>2,po 52,160'<br>2,po 46,160'<br>2,po 12,360' | | |

## Table 5

| STRUCTURE | RBL dose uM | RBL % Inh. | RBL IC50 nM | HWB dose uM | HWB % Inh. | HWB IC50 | ex vivo LTB4 dose mg/k | ex vivo LTB4 % Inh. | ex vivo LTB4 IC50 |
|---|---|---|---|---|---|---|---|---|---|
| 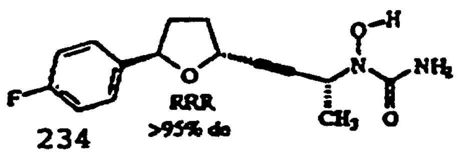 234 RRR >95% de | | | 3100 | | | 3.2 | <br>2,po<br>2,po<br>2,po | RAT<br>73,60'<br>65,180'<br>28,360' | |
| 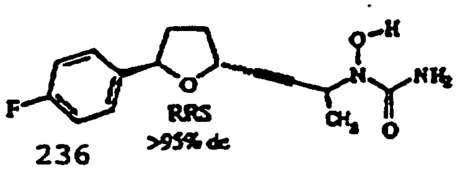 236 RRS >95% de | | | 2000 | | | 0.75 | <br>2,po<br>2,po<br>2,po<br>2,po | RAT<br>42,60'<br>-4,180'<br>-5.6,60'<br>-1,160' | |
| 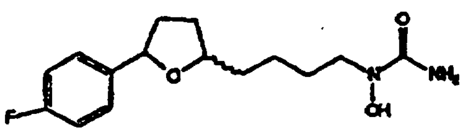 | | | 618 | | | .230 | <br>3,iv<br>3,iv<br>10,po<br>10,po | RAT<br>97,60'<br>22,120'<br>81.8, 60'<br>83.,180' | |
| | | | | | | | | | |

| STRUCTURE | RBL dose uM | RBL % inh. | RBL IC50 nM | HWB dose uM | HWB % inh. | HWB IC50 | ex vivo LTB4 dose mg/k | ex vivo LTB4 % inh. | ex vivo LTB4 IC50 |
|---|---|---|---|---|---|---|---|---|---|
| 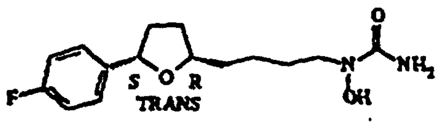 | | | 1100 | | | 0.097 | <br>2,po<br>2,po<br>2,po<br>2,po | RAT<br>61,60'<br>7.2,180'<br>63,60'<br>37,18' | |
| 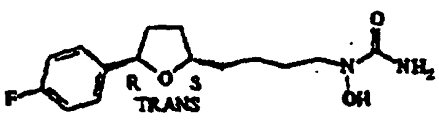 | | | | | | 0.143 | | | |
| 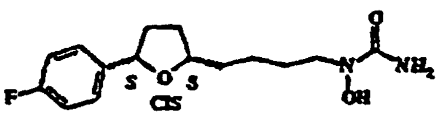 | | | | | | 0.145 | | | |
| 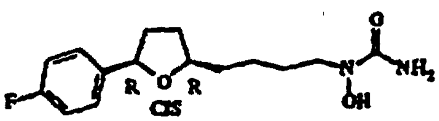 | | | | | | 0.173 | | | |

Modifications and variations of the present invention relating to compounds that reduce the formation of oxygen radicals during an inflammatory or immune response will be obvious to those skilled in the art from the foregoing detailed description of the invention. Such modifications and variations are intended to come within the scope of the appended claims.

## Claims

**1.** A compound of formula:

(I)

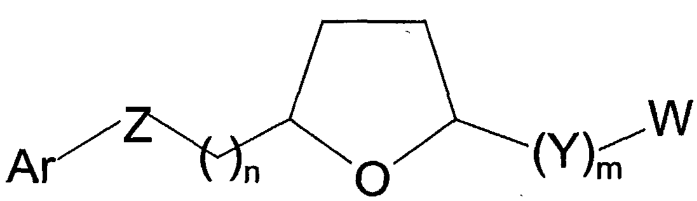

wherein:

Ar is an aryl group where the term aryl refers to phenyl, biphenyl or naphthyl, which group is optionally substituted with halo, $C_{1-6}$ alkoxy, aryloxy, W, cyano or $R^3$; or Ar is a heteroaryl group, where the term heteroaryl refers to an aromatic moiety that contains heteroatoms selected from sulfur, oxygen and nitrogen, which group is optionally substituted with halo, $C_{1-6}$ alkoxy, aryloxy, W, cyano or $R^3$;
mis 0 or 1;
n is 1-6;
W is independently -AN(OM)C(O)N($R^3$)$R^4$, -N(OM)C(O)N($R^3$)$R^4$, -AN($R^3$)C(O)N(OM)$R^4$, -N($R^3$)C(O)N(OM) $R^4$, -AN(OM)C(O)$R^4$, -N(OM)C(O)$R^4$-, AC(O)N(OM)$R^4$, -C(O)N(OM)$R^4$, -C(O)NHA;
A is $C_{1-6}$ alkyl, $C_{2-6}$ alkenyl, $C_{2-6}$ alkynyl, $C_{1-10}$ alkylaryl or aryl $C_{1-10}$ alkyl groups, wherein one or more carbons optionally can be replaced by O, N, or S (with valence completed with hydrogen or oxygen as necessary), however, -Y-A-, -A-, or -AW- should not include two adjacent heteroatoms (i.e., -O-O-, -S-S-, -O-S-, etc.);
M is hydrogen, a pharmaceutically acceptable cation, or a metabolically cleavable leaving group;
Y is O, S, S(O), $S(O)_2$, $NR^3$, or $CHR^5$;
Z is O, S, S(O), $S(O)_2$, $NR^3$;
$R^3$ and $R^4$ are independently hydrogen or $C_{1-10}$ alkyl, $C_{2-10}$ alkenyl, $C_{2-10}$ alkynyl, aryl, aryl $C_{1-10}$ alkyl, $C_{1-10}$ alkylaryl, $C_{1-6}$ alkoxy-$C_{1-10}$ alkyl, $C_{1-6}$ alkylthio-$C_{1-10}$ alkyl, heteroaryl, or heteroaryl $C_{1-10}$ alkyl; and
$R^5$ is hydrogen, $C_{1-6}$ alkyl, $C_{2-6}$ alkenyl, $C_{2-6}$ alkynyl, aryl $C_{1-10}$ alkyl, $C_{1-10}$ alkylaryl, -AN(OM)C(O)N($R^3$)$R^4$, -AN($R^3$)C(O)N(OM)$R^4$, -AN(OM)C(O)$R^4$, -AC(O)N(OM)$R^4$, -AS(O)x$R^3$, -AS(O)$_n$CH$_2$C(O)$R^3$, -AS(O)$_n$CH$_2$CH (OH)$R^3$, or -AC(O)NH$R^3$, wherein x is 0-2;

the term aryl, unless otherwise specified, refers to phenyl, biphenyl or naphthyl, which group is optionally substituted with one or more moieties selected from the group consisting of halo, hydroxyl, amino, $C_{1-10}$ alkylamino, arylamino, $C_{1-10}$ alkoxy, aryloxy, nitro, cyano, sulfonic acid, sulfate, phosphonic acid, phosphate or phosphonate, either unprotected or protected as necessary, W or $R^3$;
the term heteroaryl, unless otherwise specified, refers to an aromatic moiety that contains heteroatoms selected from sulfur, oxygen and nitrogen, which group is optionally substituted with one or more moieties selected from the group consisting of halo, hydroxyl, amino, $C_{1-10}$ alkylamino, arylamino, $C_{1-10}$ alkoxy, aryloxy, nitro, cyano, sulfonic acid, sulfate, phosphonic acid, phosphate or phosphonate, either unprotected or protected as necessary, W or $R^3$.

2. The compound of claim 1, wherein Ar is selected from the group consisting of phenyl, trimethoxyphenyl, dimethoxyphenyl, fluorophenyl, difluorophenyl, pyridyl, dimethoxypyridyl, quinolinyl, furyl, imidazolyl, and thienyl.

3. The compound of claim 1 or claim 2, wherein Ar is 4-fluorophenyl.

4. The compound of any one of claims 1 to 3, wherein Z is O or S.

5. The compound of any one of claims 1 to 4, wherein -$(Y)_m$W is selected from the group consisting of:

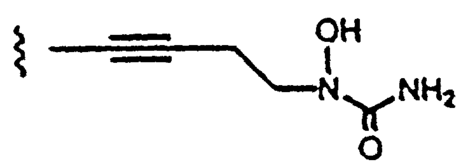

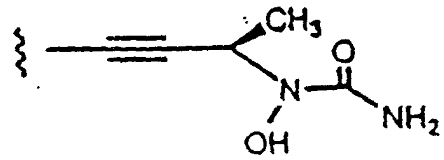

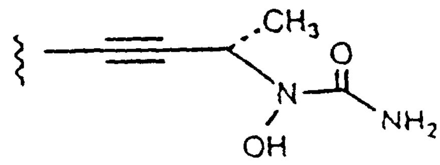

6. The compound of any one of claims 1 to 4, wherein $-(Y)_mW$ is selected from the group consisting of:

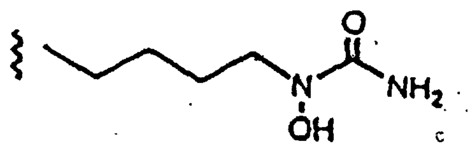

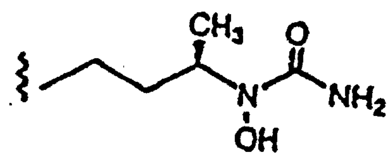

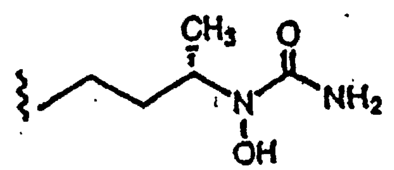

7. The compound of any one of claims 1 to 6 in at least 97% enantiomerically enriched form.

8. A compound as claimed in Claim 1 selected from the group consisting of 2S,5S-trans-2-(4-fluorophenoxymethyl)-5-(4-*tert*-butyldimethylsilyloxy-1-butynyl)tetrahydrofuran, or 2S,5R-cis-2-(4-fluorophenoxymethyl)-5-(4-*tert*-butyldimethylsilyloxy-1-butynyl)tetrahydrofuran.

9. A compound as claimed in Claim 1 selected from the group consisting of 2S,SR-trans-2-(4-fluorophenoxymethyl)-5-(4-N-hydroxyureidyl-1-butyl)tetrahydrofuran, 2S,5S-trans-2-(4-fluorophenoxymethyl)-5-(4-N-hydroxyureidyl-1-butynyl)tetrahydrofuran, 2R,5S-trans-2-(4-fluorophenoxymethyl)-5-(4-N-hydroxyureidyl-1-butyl)tetrahydrofuran, or 2R,5R-trans-2-(4-fluorophenoxymethyl)-5-(4-N-hydroxyureidyl-1-butynyl)tetrahydrofuran.

10. The compound of claim 9, which is 2S,5S-*trans*-2-(4-fluorophenoxymethyl)-5-(4-N-hydroxyureidyl-1-butynyl)tetrahydrofuran.

**11.** A pharmaceutical composition comprising an effective amount of the compound of any one of the preceding claims or a pharmaceutically-acceptable salt thereof, in combination with a pharmaceutically acceptable carrier.

**12.** A compound as defined in any one of Claims 1 to 10, or a pharmaceutically-acceptable salt thereof, for use as a medicament.

**13.** The use of a compound as defined in any one of Claims 1 to 10, or a pharmaceutically-acceptable salt thereof, for the manufacture of a medicament for the treatment of an inflammatory disorder.

**14.** The use of a compound as defined in any one of Claims 1 to 10, or a pharmaceutically-acceptable salt thereof, for the manufacture of a medicament for the treatment of a disorder mediated by 5-lipoxygenase.

**15.** The use of a compound as defined in any one of Claims 1 to 10, or a pharmaceutically-acceptable salt thereof, for the manufacture of a medicament for the treatment of a cardiovascular disorder.

**16.** The use of a compound as defined in any one of Claims 1 to 10, or a pharmaceutically-acceptable salt thereof, for the manufacture of a medicament for the treatment of hypertension.

**17.** The use of a compound as defined in any one of Claims 1 to 10, or a pharmaceutically-acceptable salt thereof, for the manufacture of a medicament for the treatment of asthma.

**18.** The use of a compound as defined in any one of Claims 1 to 10, or a pharmaceutically-acceptable salt thereof, for the manufacture of a medicament for the treatment of a disease or disorder selected from the group consisting of osteoarthritis, gout, lung edema, adult respiratory distress syndrome, pain, aggregation of platelets, shock, rheumatoid arthritis, juvenile rheumatoid arthritis, psoriatic arthritis, psoriasis, autoimmune uveitis, allergic encephalomyelytis, systemic lupus erythematosis, acute necrotizing hemorrhagic encephalopathy, idiopathic thrombocytopenia, polychondritis, chronic active hepatitis, idiopathic sprue, Crohn's disease, Graves ophthalomopathy, primary biliary cirrhosis, uveitis posterior, interstitial lung fibrosis, allergic asthma, atopic dermatitis and contact dermatitis.

**19.** The use of a compound as defined in any one of Claims 1 to 10, or a pharmaceutically-acceptable salt thereof, for the manufacture of a medicament for the treatment of inflammations of the bowel.

**20.** The use of a compound as defined in any one of Claims 1 to 10, or a pharmaceutically-acceptable salt thereof, for the manufacture of a medicament for the treatment of Crohn's disease.

## Patentansprüche

**1.** Verbindung mit der Strukturformel:

(I)

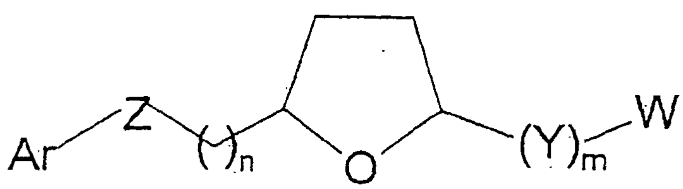

wobei folgendes gilt:

Ar ist eine Arylgruppe, wobei der Ausdruck "Aryl" Phenyl, Biphenyl oder Naphthyl bezeichnet, wobei diese Gruppe wahlweise durch Halogen, $C_{1-6}$-Alkoxy, Aryloxy, W, eine Cyan-Gruppe oder $R^3$ substituiert ist; alternativ ist Ar eine Heteroaryl-Gruppe, wobei der Ausdruck "Heteroaryl" eine aromatische Komponente bezeichnet, die Heteroatome enthält, die aus der Gruppe Schwefel, Sauerstoff und Stickstoff ausgewählt sind, wobei diese Gruppe optional durch Halogen, $C_{1-6}$-Alkoxy, Aryloxy, W, eine Cyan-Gruppe oder $R^3$ substituiert ist;
m ist 0 oder 1 ;

n ist eine Zahl von 1 bis 6;
W ist unabhängig -AN(OM)C(O)N($R^3$)$R^4$, -N(OM)C(O)N($R^3$)$R^4$, -AN($R^3$)C(O)N(OM)$R^4$, -N($R^3$)C(O)N(OM)$R^4$, -AN(OM)C(O)$R^4$, -N(OM)C(O)$R^4$, -AC(O)N(OM)$R^4$, -C(O)N(OM)$R^4$, -C(O)NHA;
A ist $C_{1-6}$-Alkyl, $C_{2-6}$-Alkenyl, $C_{2-6}$-Alkynyl, $C_{1-10}$-Alkylaryl oder eine Aryl-$C_{1-10}$-Alkyl-Gruppe, wobei ein oder mehrere Kohlenstoffatome wahlweise durch O, N oder S substituiert werden können (wobei die Valenzen mit Wasserstoff oder Sauerstoff in der erforderlichen Weise vervollständigt werden), doch sollten -Y-A-, -A-, oder -AW- nicht zwei benachbarte Heteroatome (d.h. -O-O-, -S-S-, -O-S-, usw.) umfassen;
M ist Wasserstoff, ein pharmazeutisch akzeptables Kation oder eine methabolisch abspaltbare austretende Gruppe;
Y ist O, S, S(O), $S(O)_2$, $NR^3$ oder $CHR^5$;
Z ist O, S, S(O)$S(O)_2$, $NR^3$;
$R^3$ und $R^4$ sind unabhängig Wasserstoff oder $C_{1-10}$-Alkyl, $C_{2-10}$-Alkenyl, $C_{2-10}$-Alkynyl, Aryl, Aryl-$C_{1-10}$-Alkyl, $C_{1-10}$-Alkylaryl, $C_{1-6}$-Alkoxy-$C_{1-10}$-Alkyl, $C_{1-6}$-Alkylthio-$C_{1-10}$-Alkyl, Heteroaryl, oder Heteroaryl-$C_{1-10}$-Alkyl; und
$R^5$ ist Wasserstoff, $C_{1-6}$-Alkyl, $C_{2-6}$-Alkenyl, $C_{2-6}$-Alkynyl, Alkyl-$C_{1-10}$-Alkyl, $C_{1-10}$-Alkylaryl, -AN(OM)C(O)N ($R^3$)$R^4$, -AN($R^3$)C(O)N(OM)$R^4$, -AN(OM)C(O)$R^4$, -AC(O)N(OM)$R^4$, -AS(O)x$R^3$, -AS$(O)_n$$CH_2$C(O)$R^3$, -AS $(O)_n$$CH_2$CH(OH)$R^3$, oder -AC(O)NH$R^3$, wobei x eine Zahl von 0 bis 2 ist;

der Ausdruck "Aryl" bezeichnet, wenn nicht anders spezifiziert, Phenyl, Biphenyl oder Naphthyl, wobei diese Gruppe wahlweise mit einer oder mehreren Komponenten substituiert ist, die aus der Gruppe ausgewählt sind, die Halogen, Hydroxyl, Amino, $C_{1-10}$-Alkylamino, Arylamino, $C_{1-10}$-Alkoxy, Aryloxy, Nitro, Ciano, Sulfonsäure, Sulfat, Phosphonsäure, Phosphate oder Phosphonate je nach Notwendigkeit entweder ungeschützt oder geschützt, W oder $R^3$ umfaßt;
der Ausdruck "Heteroaryl" betrifft, wenn nicht anders spezifiziert, eine aromatische Komponente, die Heteroatome umfaßt, die aus der Gruppe Schwefel, Sauerstoff und Stickstoff ausgewählt sind, wobei diese Gruppe optional mit einer oder mehreren Komponenten substituiert ist, die aus der Gruppe ausgewählt sind, die Halogen, Hydroxyl, Amino, $C_{1-10}$-Alkylamino, Arylamino, $C_{1-10}$-Alkoxy, Aryloxy, Nitro, Cyano, Sulfonsäure, Sulfat, Phosphonsäure, Phosphat oder Phosphonat, je nach Erfordernis entweder geschützt oder ungeschützt, W oder $R^3$ umfaßt.

**2.** Verbindung nach Anspruch 1, bei der Ar aus der Gruppe ausgewählt ist, die aus Phenyl, Trimethoxyphenyl, Dimethoxyphenyl, Fluorophenyl, Difluprophenyl, Pyridyl, Dimethoxypyridyl, Quinolinyl, Furyl, Imidazolyl und Thienyl besteht.

**3.** Verbindung nach Anspruch 1 oder 2, bei der Ar 4-Fluorophenyl ist.

**4.** Verbindung nach einem der Ansprüche 1 bis 3, bei der Z gleich O oder S ist.

**5.** Verbindung nach einem der Ansprüche 1 bis 4, bei der $-(Y)_m W$ aus der Gruppe ausgewählt ist, die aus folgenden Verbindungen besteht:

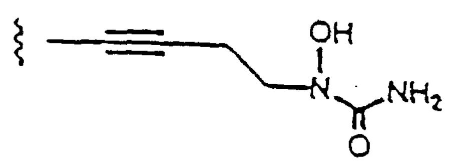

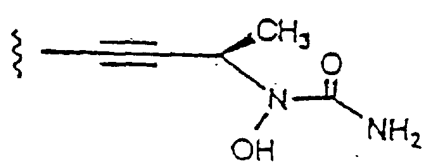

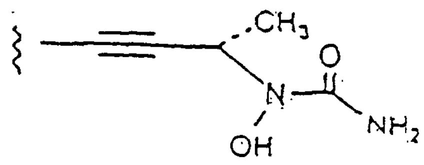

**6.** Verbindung nach einem der Ansprüche 1 bis 4; bei der $-(Y)_mW$ aus der Gruppe ausgewählt ist, die aus folgenden Verbindungen besteht:

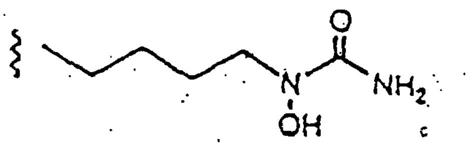

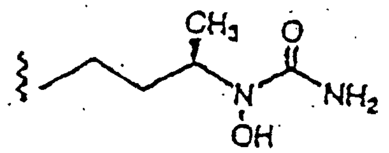

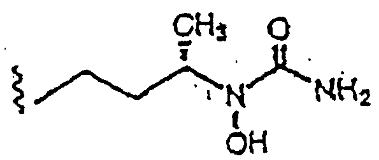

**7.** Verbindung nach einem der Ansprüche 1 bis 6, in einer wenigstens zu 97 % enantiomer angereicherten Form.

**8.** Verbindung nach Anspruch 1, die aus der Gruppe ausgewählt ist, die aus folgenden Verbindungen besteht:

2S,5S-trans-2-(4-fluorophenoxymethyl)-5-(4-*tert*-butyldimethylsilyloxy-1-butynyl)tetrahydrofuran oder
2S,5R-ds-2-(4-fluorophenoxymethyl)-5-(4-*tert*-butyldimethylsilyloxy-1-butynyl)tetrahydrofuran.

**9.** Verbindung nach Anspruch 1, die aus der Gruppe ausgewählt ist, die folgende Verbindungen umfaßt:

2S,5R-trans-2-(4-fluorophenoxymethyl)-5-(4-N-hydroxyureidyl-1-butyl)tetrahydrofuran,
2S,5S-trans-2-(4-fluorophenoxymethyl)-5-(4-N-hydroxyureidyl-1-butynyl)tetrahydrofuran,
2R,5S-trans-2-(4-fluorophenoxymehtyl)-5-(4-N-hydroxyureidyl-1-butyl)tetrahydrofuran oder
2R,5R-trans-2-(4-fluorophenoxymethyl)-5-(4-N-hydroxyureidyl-1-butynyl)tetrahydrofuran.

**10.** Verbindung nach Anspruch 9, die 2S,5S-trans-2-(4-fluorophenoxymethyl)-5-(4-N-hydroxyureidyl-1-butynyl)tetrahydrofuran ist.

**11.** Pharmazeutische Zusammensetzung, die eine wirksame Menge der Verbindung nach einem der vorhergehenden Ansprüche oder ein pharmazeutisch akzeptables Salz hiervon in Verbindung mit einem pharmazeutisch akzeptablen Träger umfaßt.

**12.** Verbindung nach einem der Ansprüche 1 bis 10 oder ein pharmazeutisch akzeptables Salz hiervon zur Verwendung als Medikament.

**13.** Verwendung einer Verbindung nach einem der Ansprüche 1 bis 10 oder eines pharmazeutisch akzeptablen Salzes hiervon für die Herstellung eines Medikamentes für die Behandlung einer entzündlichen Störung.

**14.** Verwendung einer Verbindung nach einem der Ansprüche 1 bis 10 oder eines pharmazeutisch akzeptablen Salzes hiervon für die Herstellung eines Medikamentes für die Behandlung einer Störung die durch 5-Lipoxygenase vermittelt wird.

**15.** Verwendung einer Verbindung nach einem der Ansprüche 1 bis 10 oder eines pharmazeutisch akzeptablen Salzes hiervon für die Herstellung eines Medikamentes zur Behandlung einer kardiovaskulären Störung.

**16.** Verwendung einer Verbindung nach einem der Ansprüche 1 bis 10 oder eines pharmazeutisch akzeptablen Salzes hiervon für die Herstellung eines Medikamentes zur Behandlung von Bluthochdruck.

**17.** Verwendung einer Verbindung nach einem der Ansprüche 1 bis 10 oder eines pharmazeutisch akzeptablen Salzes hiervon für die Herstellung eines Medikamentes für die Behandlung von Asthma.

**18.** Verwendung einer Verbindung nach einem der Ansprüche 1 bis 10 oder eines pharmazeutisch akzeptablen Salzes hiervon für die Herstellung eines Medikamentes für die Behandlung einer Erkrankung oder einer Störung, die aus folgender Gruppe ausgewählt ist: Osteoarthritis, Gicht, Lungenödem, Atemnot-Syndrom bei Erwachsenen, Schmerz, Plättchenaggregation, Schock, rheumatische Arthritis, rheumatische Arthritis bei Jugendlichen, entzündliche Arthritis, Schuppenflechte, Autoimmun-Uveitis, allergische Encephalomyelytis, systemische Lupus-Erythematose, akute nekrotisierende hämoragische Encephalopatie, idiopatische Thrombocytopenie, Polychondritis, chronische aktive Hepatitis, idiopatisches Sprue, Morbus Crohn, Ophthalomopatie nach Graves, primäre Leberzirrhose, Uveitis posterior, interstitielle Lungenfibrose, allergisches Asthma, atopische Dermatitis und Kontaktdermatitis.

**19.** Verwendung einer Verbindung nach einem der Ansprüche 1 bis 10 oder eines pharmazeutisch akzeptablen Salzes hiervon für die Herstellung eines Medikamentes für die Behandlung von Darmentzündungen.

**20.** Verwendung einer Verbindung nach einem der Ansprüche 1 bis 10 oder eines pharmazeutisch akzeptablen Salzes hiervon für die Herstellung eines Medikamentes für die Behandlung von Morbus Crohn.

**Revendications**

**1.** Composé de formule :

(I)

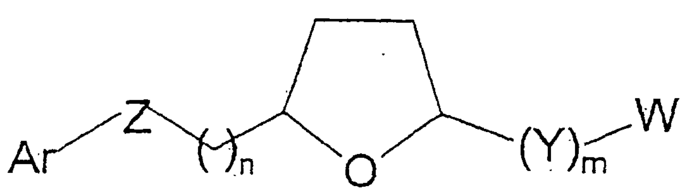

dans laquelle :

Ar est un groupe aryle où le terme aryle désigne un groupe phényle, biphényle ou naphtyle, lequel groupe est éventuellement substitué par un halogéno, un alcoxy en $C_{1-6}$, un aryloxy, W, un cyano ou $R^3$ ; ou bien Ar est un groupe hétéroaryle, où le terme hétéroaryle désigne un groupe aromatique qui contient des hétéroatomes choisis parmi le soufre, l'oxygène et l'azote, lequel groupe est éventuellement substitué par un halogéno, un alcoxy en $C_{1-6}$, un aryloxy, W, un cyano ou $R^3$ ;
m vaut 0 ou 1 ;
n vaut de 1 à 6 ;
W est, indépendamment, $-AN(OM)C(O)N(R^3)R^4$, $-N(OM)C(O)N(R^3)R^4$, $-AN(R^3)C(O)N(OM)R^4$, $-N(R^3)C(O)N(OM)R^4$, $-AN(OM)C(O)R^4$, $-N(OM)C(O)R^4$, $-AC(O)N(OM)R^4$, $-C(O)N(OM)R^4$, $-C(O)NHA$ ;
A est un groupe alkyle en $C_{1-6}$, alcényle en $C_{2-6}$, alcynyle en $C_{2-6}$, (alkyle en $C_{1-10}$)-aryle ou aryl-(alkyle en

$C_{1-10}$), dans lequel un ou plusieurs atomes de carbone peuvent éventuellement être remplacés par O, N ou S (la valence étant complétée par de l'hydrogène ou de l'oxygène si nécessaire), toutefois, -Y-A-, -A- ou -AW- ne devraient pas inclure deux hétéroatomes adjacents (à savoir, -O-O-, -S-S-, -O-S-, etc.) ;

M est un hydrogène, un cation pharmaceutiquement acceptable ou un groupe partant pouvant être métaboliquement clivé;

Y est O, S, S(O), $S(O)_2$, $NR^3$ ou $CHR^5$ ;

Z est O, S, S(O), $S(O)_2$, $NR^3$ ;

$R^3$ et $R^4$ sont, indépendamment l'un de l'autre, un hydrogène ou un alkyle en $C_{1-10}$, un alcényle en $C_{2-10}$, un alcynyle en $C_{2-10}$, un aryle, un aryl-(alkyle en $C_{1-10}$), un (alkyle en $C_{1-10}$)-aryle, un (alcoxy en $C_{1-6}$)-(alkyle en $C_{1-10}$), un (alkyle en $C_{1-6}$)-thio-(alkyle en $C_{1-10}$), un hétéroaryle ou hétéroaryl-(alkyle en $C_{1-10}$) ; et

$R^5$ est un hydrogène, un alkyle en $C_{1-6}$, un alcényle en $C_{2-6}$, un alcynyle en $C_{2-6}$, un aryl-(alkyle en $C_{1-10}$), un (alkyle en $C_{1-10}$)-aryle, $-AN(OM)C(O)N(R^3)R^4$, $-AN(R^3)C(O)N(OM)R^4$, $-AN(OM)C(O)R^4$, $-AC(O)N(OM)R^4$, $-AS(O)xR^3$, $-AS(O)_nCH_2C(O)R^3$, $-AS(O)_nCH_2CH(OH)R^3$ ou $-AC(O)NHR^3$, dans lequel x vaut de 0 à 2 ;

le terme aryle, sauf indication contraire, désigne un phényle, un biphényle ou un naphtyle, lequel groupe est éventuellement substitué par un ou plusieurs groupes choisis dans le groupe formé par les halogéno, hydroxyle, amino, (alkyle en $C_{1-10}$)-amino, arylamino, alcoxy en $C_{1-10}$, aryloxy, nitro, cyano, acides sulfoniques, sulfates, acides phosphoniques, phosphates ou phosphonates, soit non protégés, soit protégés, si nécessaire, W ou $R^3$ ;

le terme hétéroaryle, sauf indication contraire, désigne un groupe aromatique qui contient des hétéroatomes choisis parmi le soufre, l'oxygène et l'azote, lequel groupe est éventuellement substitué par un ou plusieurs groupes choisis dans le groupe formé par les halogéno, hydroxyle, amino, (alkyle en $C_{1-10}$)-amino, arylamino, alcoxy en $C_{1-10}$, aryloxy, nitro, cyano, acides sulfoniques, sulfates, acides phosphoniques, phosphates ou phosphonates, soit non protégés, soit protégés, si nécessaire, W ou $R^3$.

**2.** Composé selon la revendication 1, dans lequel Ar est choisi dans le groupe formé par les phényle, triméthoxyphényle, diméthoxyphényle, fluorophényle, difluorophényle, pyridyle, diméthoxypyridyle, quinolinyle, furyle, imidazolyle et thiényle.

**3.** Composé selon la revendication 1 ou 2, dans lequel Ar est le 4-fluorophényle.

**4.** Composé selon l'une quelconque des revendications 1 à 3, dans lequel Z est O ou S.

**5.** Composé selon l'une quelconque des revendications 1 à 4, dans lequel $-(Y)_mW$ est choisi dans le groupe formé par :

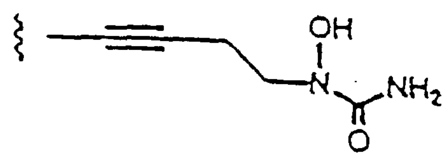

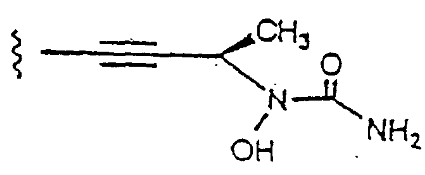

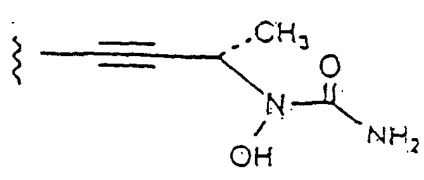

6. Composé selon l'une quelconque des revendications 1 à 4, dans lequel $-(Y)_mW$ est choisi dans le groupe formé par :

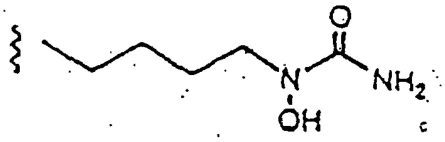

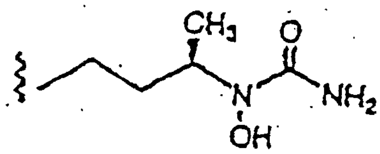

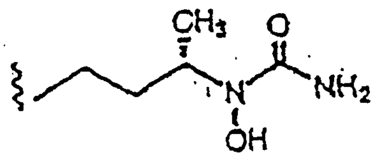

7. Composé selon l'une quelconque des revendications 1 à 6, dont au moins 97% sont sous une forme énantiomériquement enrichie.

8. Composé selon la revendication 1, choisi dans le groupe formé par :

le 2S,5S-trans-2-(4-fluorophénoxyméthyl)-5-(4-*tert*-butyldiméthylsilyloxy-1-butynyl)tétrahydrofuranne; ou
le 2S,5R-cis-2-(4-fluorophénoxyméthyl)-5-(4-*tert*-butyldiméthylsilyloxy-1-butynyl)tétrahydrofuranne.

9. Composé selon la revendication 1, choisi dans le groupe formé par :

le 2S,5R-trans-2-(4-fluorophénoxyméthyl)-5-(4-N-hydroxyuréidyl-1-butyl)tétrahydrofuranne ;
le 2S,5S-trans-2-(4-fluorophénoxyméthyl)-5-(4-N-hydroxyuréidyl-1-butynyl)tétrahydrofuranne ;
le 2R,5S-trans-2-(4-fluorophénoxyméthyl)-5-(4-N-hydroxyuréidyl-1-butyl)tétrahydrofuranne; ou
le 2R,5R-trans-2-(4-fluorophénoxyméthyl)-5-(4-N-hydroxyuréidyl-1-butynyl)tétrahydrofuranne.

10. Composé selon la revendication 9, qui est le 2S,5S-trans-2-(4-fluorophénoxyméthyl)-5-(4-N-hydroxyuréidyl-1-butynyl)tétrahydrofuranne.

11. Composition pharmaceutique comprenant une quantité efficace du composé selon l'une quelconque des revendications précédentes ou de l'un de ses sels pharmaceutiquement acceptables, en combinaison avec un véhicule pharmaceutiquement acceptable.

12. Composé selon l'une quelconque des revendications 1 à 10, ou un de ses sels pharmaceutiquement acceptables, pour une utilisation en tant que médicament.

13. Utilisation d'un composé selon l'une quelconque des revendications 1 à 10, ou de l'un de ses sels pharmaceutiquement acceptables, pour la fabrication d'un médicament destiné au traitement d'un trouble inflammatoire.

14. Utilisation d'un composé selon l'une quelconque des revendications 1 à 10, ou de l'un de ses sels pharmaceutiquement acceptables, pour la fabrication d'un médicament destiné au traitement d'un trouble médié par la 5-lipoxygénase.

15. Utilisation d'un composé selon l'une quelconque des revendications 1 à 10, ou de l'un de ses sels pharmaceutiquement acceptables, pour la fabrication d'un médicament destiné au traitement d'un trouble cardiovasculaire.

**16.** Utilisation d'un composé selon l'une quelconque des revendications 1 à 10, ou de l'un de ses sels pharmaceutiquement acceptables, pour la fabrication d'un médicament destiné au traitement de l'hypertension.

**17.** Utilisation d'un composé selon l'une quelconque des revendications 1 à 10, ou de l'un de ses sels pharmaceutiquement acceptables, pour la fabrication d'un médicament destiné au traitement de l'asthme.

**18.** Utilisation d'un composé selon l'une quelconque des revendications 1 à 10, ou de l'un de ses sels pharmaceutiquement acceptables, pour la fabrication d'un médicament destiné au traitement d'une maladie ou d'un trouble choisi dans le groupe formé par l'arthrose, la goutte, l'oedème pulmonaire, le syndrome de détresse respiratoire de l'adulte, la douleur, l'agrégation plaquettaire, l'état de choc, la polyarthrite rhumatoïde, la polyarthrite juvénile, le rhumatisme psoriasique, le psoriasis, l'uvéite auto-immune, l'encéphalomyélite allergique, le lupus érythémateux disséminé, l'encéphalopathie hémorragique nécrosante aiguë, la thrombocytopénie idiopathique, la polychondrite, l'hépatite chronique active, la sprue idiopathique, la maladie de Crohn, l'ophtalmopathie de Graves, la cirrhose biliaire primitive, l'uvéite postérieure, la fibrose pulmonaire interstitielle, l'asthme allergique, la dermatite atopique et la dermatite de contact.

**19.** Utilisation d'un composé selon l'une quelconque des revendications 1 à 10, ou de l'un de ses sels pharmaceutiquement acceptables, pour la fabrication d'un médicament destiné au traitement des inflammations de l'intestin.

**20.** Utilisation d'un composé selon l'une quelconque des revendications 1 à 10, ou de l'un de ses sels pharmaceutiquement acceptables, pour la fabrication d'un médicament destiné au traitement de la maladie de Crohn.

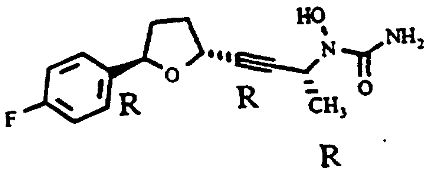

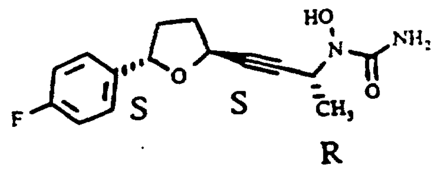

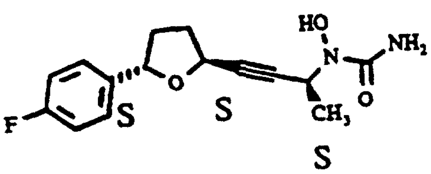

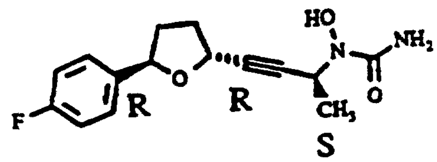

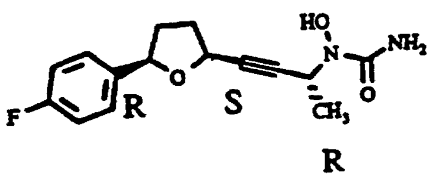

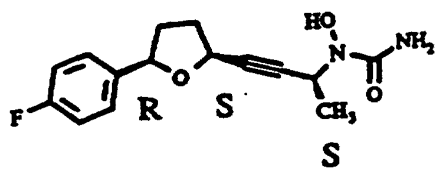

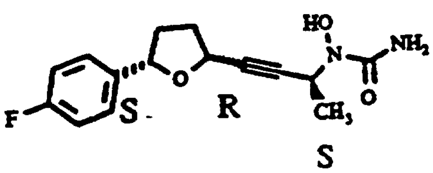

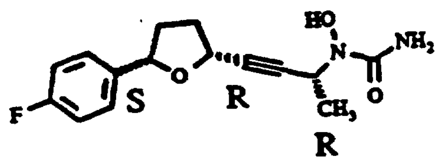

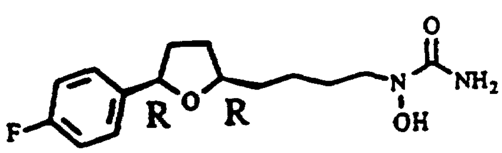

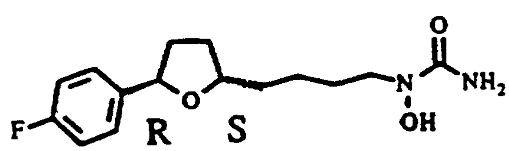

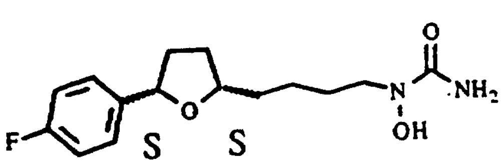

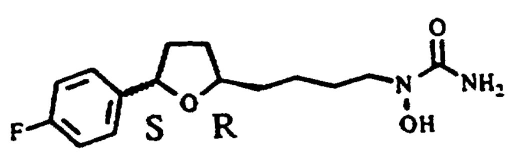

Figure 1a

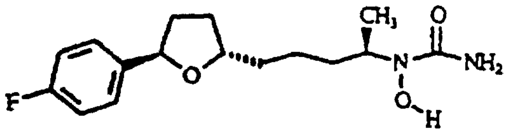

R S R

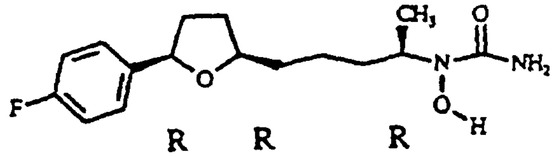

R R R

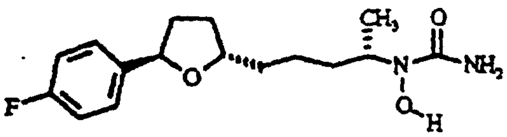

R S S

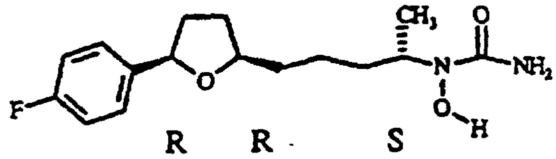

R R S

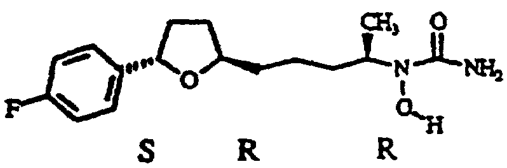

S R R

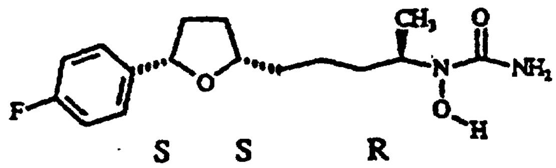

S S R

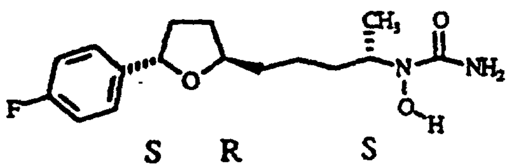

S R S

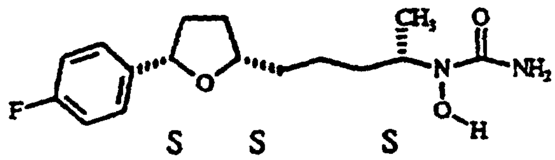

S S S

Figure 1b

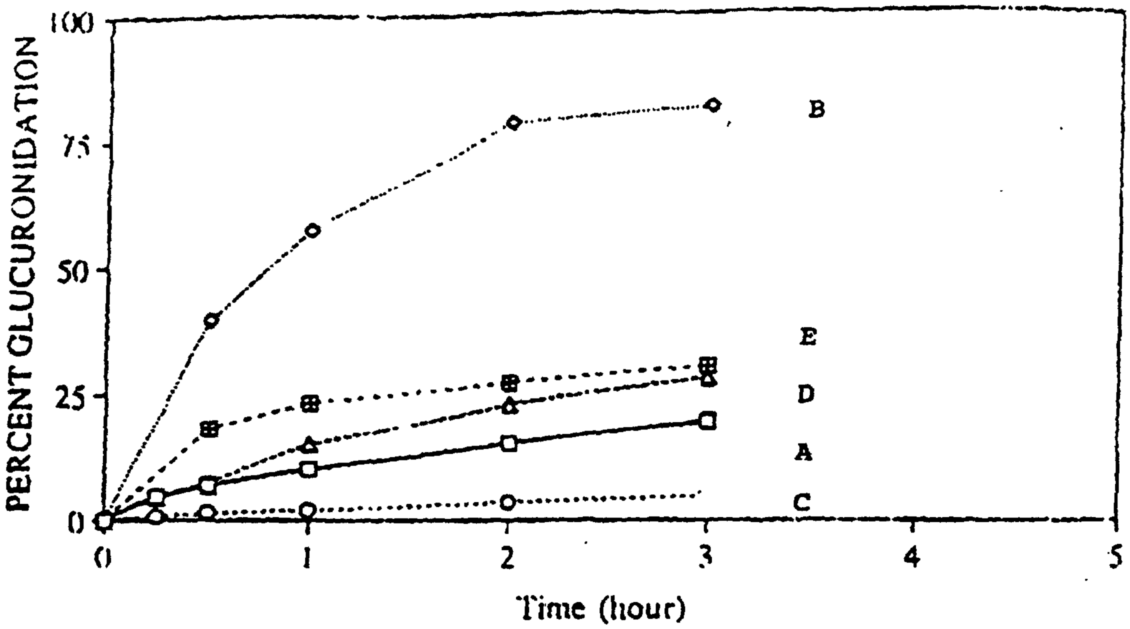


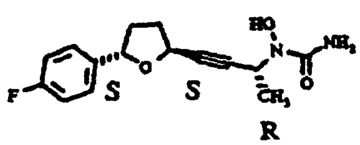

A

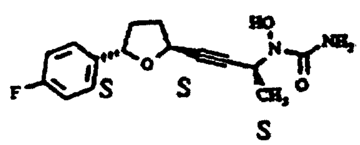

B

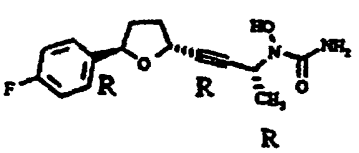

C

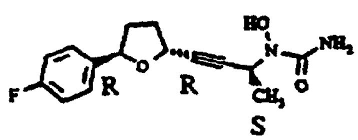

D

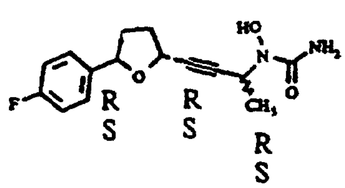

E

Figure 2

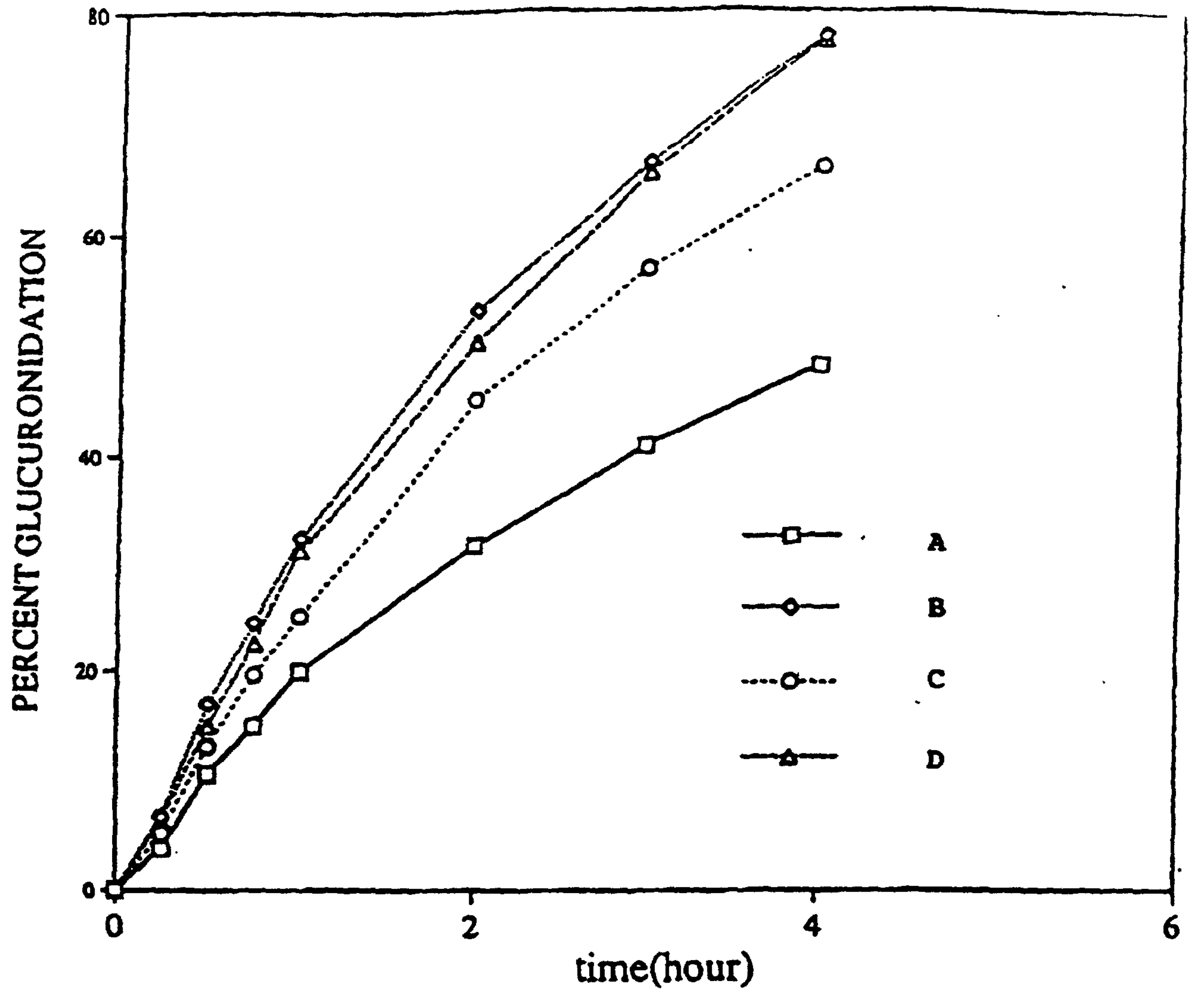


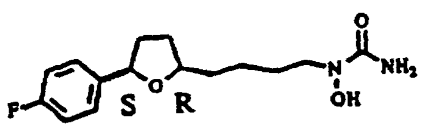

A

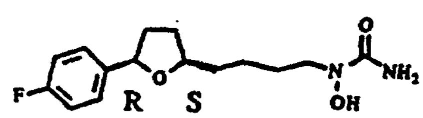

B

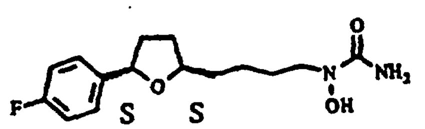

C

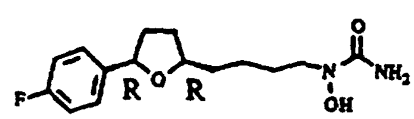

D

Figure 3

FIGURE 4

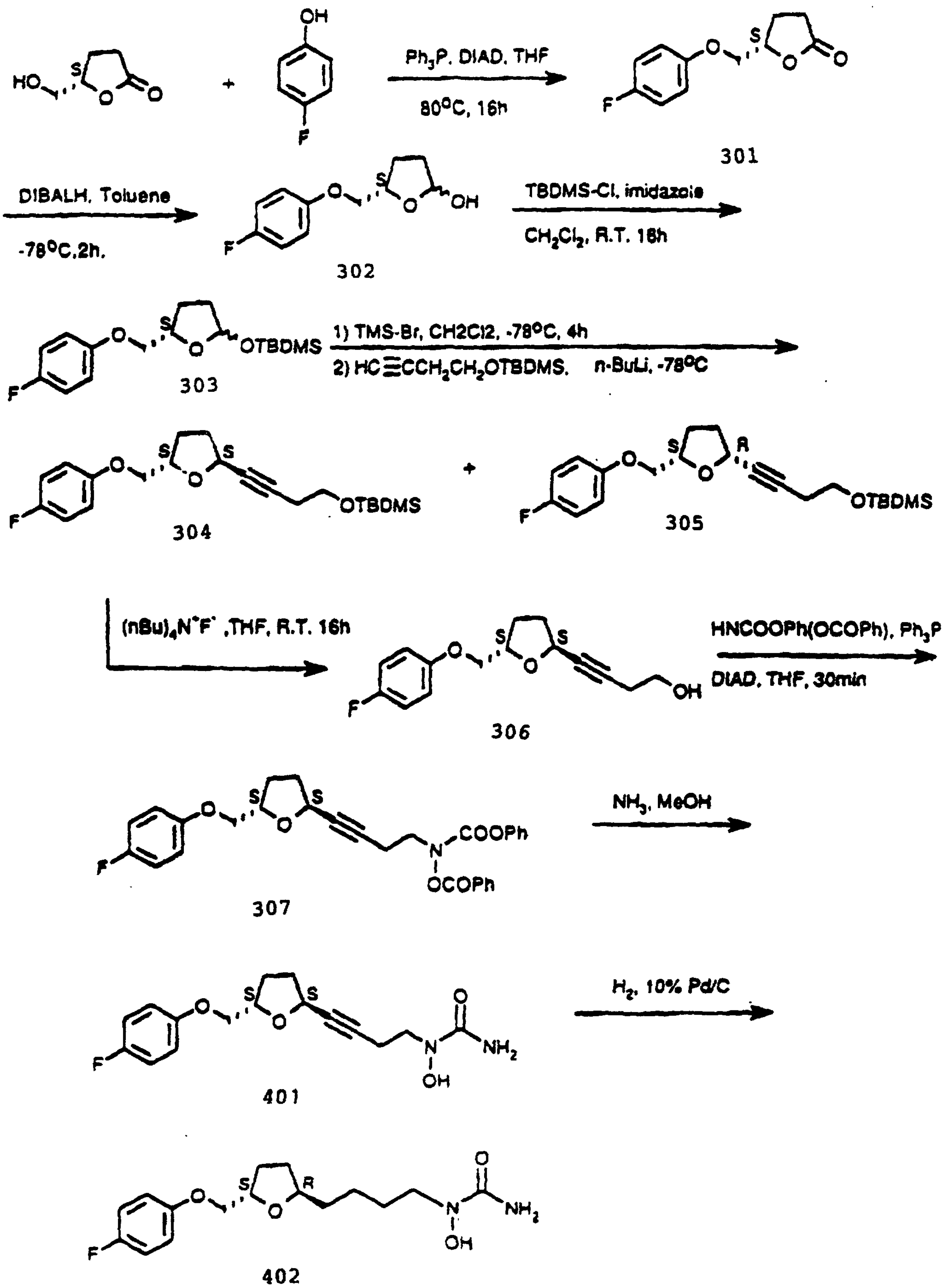

Ph$_3$P, DIAD, THF
80°C, 16h
301
DIBALH, Toluene
-78°C, 2h.
302
TBDMS-Cl, imidazole
CH$_2$Cl$_2$, R.T. 16h
303
1) TMS-Br, CH2Cl2, -78°C, 4h
2) HC≡CCH$_2$CH$_2$OTBDMS, n-BuLi, -78°C
304
305
(nBu)$_4$N$^+$F$^-$, THF, R.T. 16h
306
HNCOOPh(OCOPh), Ph$_3$P
DIAD, THF, 30min
307
NH$_3$, MeOH
401
H$_2$, 10% Pd/C
402

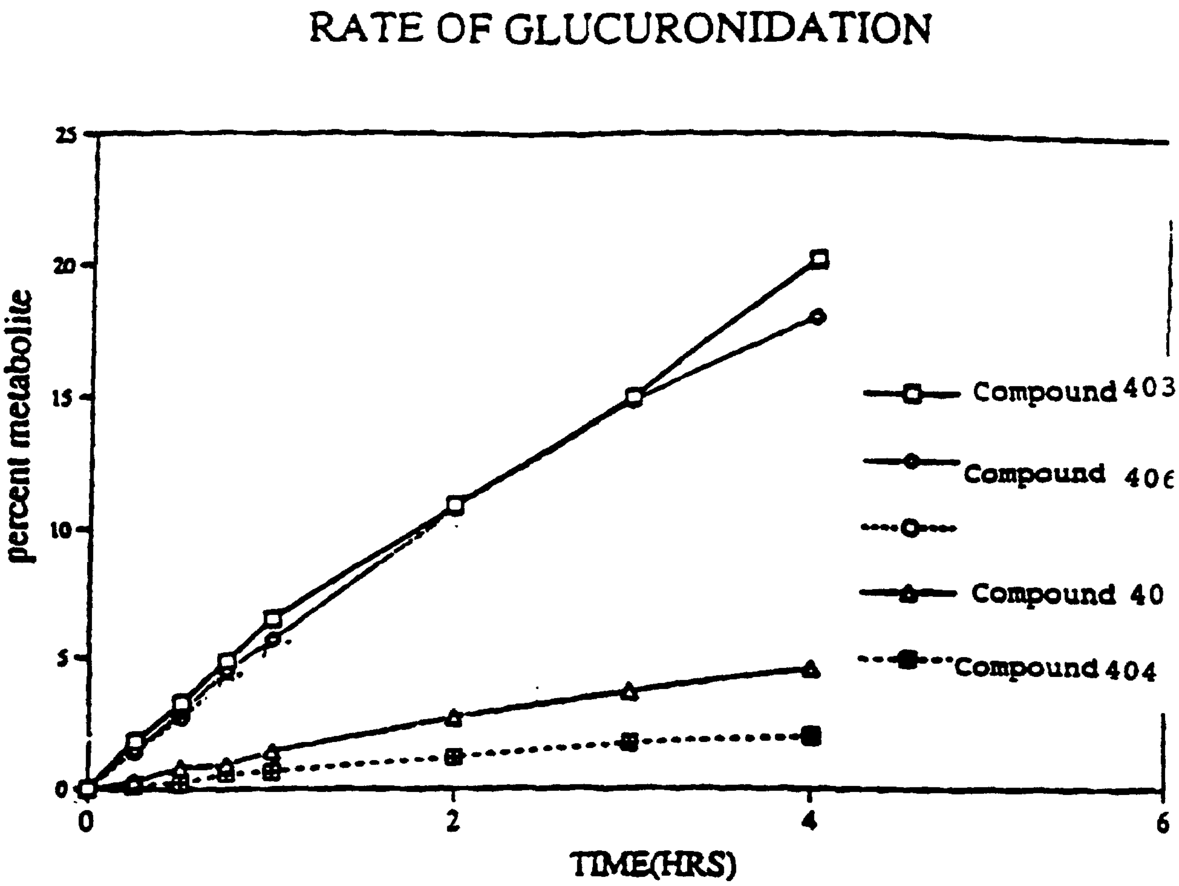
RATE OF GLUCURONIDATION
percent metabolite
25
20
15
10
5
0
0
2
4
6
TIME(HRS)
Compound 403
Compound 406
Compound 40
Compound 404

Figure 5